# EUROPEAN PATENT APPLICATION

(11) **EP 4 410 310 A1**
(43) Date of publication of application: **07.08.2024**
(21) Application number: 22876442.9
(22) Date of filing: 29.09.2022
(51) Int. Cl.: A61K 45/00, A61K 31/5513, A61P 11/00, A61P 29/00

(54) **PROPHYLACTIC OR THERAPEUTIC AGENT FOR RESPIRATORY DISEASES**

(30) Priority: 30.09.2021 JP 2021162383
(71) Applicant: Nippon Chemiphar Co., Ltd., Tokyo 101-0032 (JP)
(72) Inventor: IMAI Toshiyasu, Misato-shi, Saitama 341-0005 (JP)
(74) Representative: V.O.
(86) International application number: PCT/JP2022/036415
(87) International publication number: WO 2023/054578

(57) **Abstract**

The present invention relates to a pharmaceutical composition for preventing or treating respiratory disease, particularly, inflammatory respiratory disease, the pharmaceutical composition including a compound having a P2X4 receptor antagonistic action or a pharmaceutically acceptable salt of the compound as an active ingredient.

## Description

### Technical Field

The present invention relates to an agent for preventing or treating respiratory disease, particularly, inflammatory respiratory disease.

The present application claims priority based on Japanese Patent Application No. 2021-162383 filed in Japan on September 30, 2021, the content of which is incorporated herein.

### Background Art

In general, there are a wide variety of diseases that belong to respiratory disease, and the classification by The Japanese Respiratory Society alone includes infectious respiratory disease, bronchial obstruction, allergic pulmonary disease, interstitial lung disease, neoplastic lung disease, pulmonary vascular lesion, pleural disease, and respiratory failure. Thus, the causes and symptoms are diverse, and bronchial obstruction, allergic pulmonary disease, and interstitial lung disease from the above classification belong to inflammatory respiratory disease. Examples of typical diseases that belong to these diseases include chronic obstructive pulmonary disease (COPD), interstitial pneumonia, bronchial asthma, and pneumonia.

Among the diseases, COPD is estimated to affect 8.6% of the population aged 40 and over, or approximately 5.3 million patients (NICE study), and it is considered that the majority of them are undiagnosed or untreated. COPD is the ninth leading cause of death overall, and seventh leading cause of death among men. Therefore, the early realization of a therapeutic agent is desired.

COPD is a general term for diseases that have been conventionally referred to as chronic bronchitis or emphysema. The greatest cause of COPD is smoking, and 15 to 20% of smokers develop COPD. Inhaling smoke from cigarettes causes inflammation in the bronchi in the lung, resulting in cough and phlegm production, or narrowing of the bronchi, thus reducing airflow. Alveoli, which are small grape-like aciniform sacs located at the back of the branching bronchi, are destroyed, resulting in a state referred to as emphysema, which causes the ability to take in oxygen and expel carbon dioxide to be decreased. These changes are considered to coexist in COPD, and cannot be reversed even with treatment (Non Patent Literature 1). Currently, there is no treatment for COPD that can radically cure COPD and restore the lung to its original healthy state, and the only way to treat COPD is to recognize the disease as early as possible and begin appropriate treatment. Treatment methods include cessation of smoking, drug therapy, and respiratory rehabilitation. The drug therapy mainly includes, for example, bronchodilators (anticholinergics/β2 agonists/theophylline drugs) and inhaled steroids.

However, both bronchodilators and inhaled steroids have side effects. For example, since a bronchodilator β2 agonist may have side effects such as tachycardia, tremor, and hypokalemia, a β2 agonist needs to be administered with caution to patients with hypertension or heart disease. For this reason, a bronchodilator must be administered by inhalation. Furthermore, inhaled steroids may have a side effect of dysphonia such as hoarseness.

As described above, COPD cannot be radically cured at the present moment, and, as for the therapeutic agent, a novel pharmaceutical product that contributes to improving patients' QOL is desired, from the viewpoints of side effects.

Patent Literature 1 discloses a P2X4 receptor antagonist.

However, a compound described in an example in Patent Literature 1 is a selective serotonin reuptake inhibitor, for example, Paroxetine, Fluoxetine, or the like, and has a structure that is completely different from that of a compound of the present application, which is a benzodiazepine derivative compound. In addition, Patent Literature 1 only shows experimental results obtained using a neuropathic pain pathological model in which a nerve injury (L5 spinal nerve injury model) has been performed, and whether the P2X4 receptor antagonist has a therapeutic effect against inflammatory respiratory disease cannot be determined.

Patent Literature 2 also describes a compound exhibiting a P2X4 receptor antagonistic action. However, as in Patent Literature 1, Patent Literature 2 only shows effects shown by the neuropathic pain model, and whether the compound has a therapeutic effect against inflammatory respiratory disease is unclear.

In addition, the applicant has filed other patent applications related to P2X4 receptor antagonists as shown in Patent Literature 3 to 9, 11, and 12, but, in all of these applications, it is unclear whether the P2X4 receptor antagonists have therapeutic effects against inflammatory respiratory disease.

Moreover, Patent Literature 10 describes that a compound exhibiting a P2X4 receptor antagonistic action is effective in preventing or treating cough. However, it is unclear whether the compound has any effect other than preventing or treating cough, simply put, suppressing coughs, and there is no teaching that the compound is useful for preventing or treating respiratory disease, especially inflammatory respiratory disease.

### Citation List

### Patent Literature

Patent Literature 1: WO 2008/020651 A
Patent Literature 2: WO 2010/093061 A
Patent Literature 3: WO 2008/023847 A
Patent Literature 4: WO 2012/008478 A
Patent Literature 5: WO 2012/014910 A
Patent Literature 6: WO 2012/017876 A
Patent Literature 7: WO 2013/105608 A
Patent Literature 8: WO 2015/005468 A
Patent Literature 9: WO 2015/005467 A
Patent Literature 10: WO 2019/177117 A
Patent Literature 11: WO 2020/050253 A
Patent Literature 12: WO 2021/049628 A

### Non Patent Literature

Non Patent Literature 1: The Japanese Respiratory Society, Respiratory Disease B-01 Bronchial Obstruction Chronic Obstructive Pulmonary Disease (COPD): https://www.jrs.or.jp/uploads/uploads/files/disease_qa/dise ase_b01.pdf
Non Patent Literature 2: Jun-ichi Fuchikami, Maki Takahashi; Cigarette smoke-induced animal models to evaluate drug efficacy on chronic obstructive pulmonary disease (COPD); Folia Pharmacol. Jpn. 127, 183-189 (2006) Non Patent Literature 3: Nicole M. R., et al. An update and systematic review on drug therapies for the treatment of refractory chronic cough. Expert Opinion Pharmacotherapy. 2018 May; 19 (7), 687-711

### Summary of Invention

### Technical Problem

An object of the present invention is to provide a pharmaceutical composition for preventing or treating respiratory disease, particularly, inflammatory respiratory disease.

### Solution to Problem

As a result of conducting intensive research in order to achieve the above object, the present inventor found that a compound represented by General Formulas (A) to (BII) having a P2X4 receptor antagonistic action is useful in preventing or treating respiratory disease, particularly, inflammatory respiratory disease, thus completing the present invention.

That is, the present invention provides a pharmaceutical composition for preventing and/or treating respiratory disease, particularly, inflammatory respiratory disease, the pharmaceutical composition including, as an active ingredient, a compound having a P2X4 receptor antagonistic action or a pharmaceutically acceptable salt thereof. More preferably, as the compound having a P2X4 receptor antagonistic action, the compound represented by the following general formula (BI) or (BII) can be used.

As the compound having a P2X4 receptor antagonistic action, for example, compounds represented by the following general formulas (A) to (BII) can be used.

The pharmaceutical composition provided by the present invention can be used for preventing or treating, for example, respiratory disease, particularly can be used for preventing or treating inflammatory respiratory disease, can be further used for preventing or treating, for example, bronchial obstruction, allergic pulmonary disease, or interstitial lung disease, and can be further particularly used for preventing or treating, for example, COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia.

In one embodiment, the pharmaceutical composition provided by the present invention can be used, for example, for preventing or treating the respiratory disease accompanied by depression of respiratory function.

In one embodiment, the pharmaceutical composition provided by the present invention can be used, for example, for preventing or treating the respiratory disease accompanied by airflow obstruction.

In one embodiment, the pharmaceutical composition provided by the present invention can be used, for example, for preventing or treating the respiratory disease accompanied by respiratory inflammation.

In one embodiment, the pharmaceutical composition provided by the present invention can be used, for example, for preventing or treating the respiratory disease accompanied by cough reflex.

From another viewpoint, the present invention provides use of a compound having a P2X4 receptor antagonistic action or a pharmaceutically acceptable salt thereof for production of the pharmaceutical composition; and a method for prevention or treatment of respiratory disease, particularly, a method for prevention or treatment of inflammatory respiratory disease, furthermore, a method for prevention or treatment in which the inflammatory respiratory disease is, for example, bronchial obstruction, allergic pulmonary disease, or interstitial lung disease, further particularly, a method for prevention or treatment in which the bronchial obstruction is, for example, COPD, bullous lung disease, diffuse panbronchiolitis, bronchiolitis obliterans, bronchiectasis, ciliary dyskinesia, or sinobronchial syndrome, even further particularly, a method for prevention or treatment in which the allergic pulmonary disease is, for example, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), or hypersensitivity pneumonitis, and even further particularly, a method for prevention or treatment in which the interstitial lung disease is, for example, idiopathic interstitial pneumonia or acute interstitial pneumonia, the method including a step of administering a prophylactically or therapeutically effective dose of the compound having the P2X4 receptor antagonistic action or a pharmaceutically acceptable salt thereof to a mammal, including a human.

Moreover, in one aspect, the present invention provides use of a compound having a P2X4 receptor antagonistic action or a pharmaceutically acceptable salt thereof for production of the pharmaceutical composition; and a method for prevention or treatment of the respiratory disease accompanied by depression of respiratory function, a method for prevention or treatment of the respiratory disease accompanied by airflow obstruction, a method for prevention or treatment of the respiratory disease accompanied by respiratory inflammation, or a method for prevention or treatment of the respiratory disease accompanied by cough reflex, the method including a step of administering a prophylactically or therapeutically effective dose of the compound having the P2X4 receptor antagonistic action or a pharmaceutically acceptable salt thereof to a mammal, including a human.

### Advantageous Effects of Invention

The pharmaceutical composition provided by the present invention is useful as a pharmaceutical composition for preventing or treating respiratory disease, particularly useful as a pharmaceutical composition for preventing or treating inflammatory respiratory disease, further useful as a pharmaceutical composition for preventing or treating, for example, bronchial obstruction, allergic pulmonary disease, or interstitial lung disease, and further useful as a pharmaceutical composition for preventing or treating, for example, COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia, and each is expected to be highly effective.

Furthermore, in one aspect, the pharmaceutical composition provided by the present invention is useful as a pharmaceutical composition for preventing or treating the respiratory disease accompanied by depression of respiratory function.

Moreover, in one aspect, the pharmaceutical composition provided by the present invention is useful as a pharmaceutical composition for preventing or treating the respiratory disease accompanied by airflow obstruction.

Moreover, in one aspect, the pharmaceutical composition provided by the present invention is useful as a pharmaceutical composition for preventing or treating the respiratory disease accompanied by respiratory inflammation.

Moreover, in one aspect, the pharmaceutical composition provided by the present invention is useful as a pharmaceutical composition for preventing or treating the respiratory disease accompanied by cough reflex.

### Brief Description of Drawings

Figs. 1(a) to 1(e) are diagrams respectively showing the effects of the administration of Compound A and the like on the following physiological indicators (in the present specification, may be referred to as measurement parameters) in a cigarette smoke-exposed model obtained using guinea pigs.
(a) Specific Airway Resistance (sRaw)
(b) Tidal Volume (TV)
(c) Respiratory Rate (RR)
(d) Minute Volume (MV)
(e) Peak Expiratory Flow (PEF)

Fig. 2 is a diagram showing cough reflex in the cigarette smoke-exposed model obtained using guinea pigs and the effect of the administration of Compound A and the like on the cough reflex.

Figs. 3(a) to 3(e) are diagrams respectively showing the effects of the administration of Compound A and the like on the following blood components (cells) in the cigarette smoke-exposed model obtained using guinea pigs.
(a) White Blood Cells (WBC)
(b) Macrophages
(c) Neutrophils
(d) Eosinophils
(e) Lymphocytes

Fig. 4 is a diagram showing weight loss in the cigarette smoke-exposed model obtained using guinea pigs and the effect of the administration of Compound A on the weight loss.

### Description of Embodiments

In one embodiment, a pharmaceutical composition provided by the present invention can be used as a pharmaceutical composition for preventing or treating, for example, respiratory disease.

In one embodiment, the pharmaceutical composition provided by the present invention can be used as a pharmaceutical composition for preventing or treating, for example, inflammatory respiratory disease.

In one embodiment, the pharmaceutical composition provided by the present invention can be used as a pharmaceutical composition for preventing or treating, for example, bronchial obstruction, allergic pulmonary disease, or interstitial lung disease.

In one embodiment, the pharmaceutical composition provided by the present invention can be used as a pharmaceutical composition for preventing or treating, for example, COPD, bullous lung disease, diffuse panbronchiolitis, bronchiolitis obliterans, bronchiectasis, ciliary dyskinesia, or sinobronchial syndrome.

In one embodiment, the pharmaceutical composition provided by the present invention can be used as a pharmaceutical composition for preventing or treating, for example, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), or hypersensitivity pneumonitis.

In one embodiment, the pharmaceutical composition provided by the present invention can be used as a pharmaceutical composition for preventing or treating, for example, idiopathic interstitial pneumonia, or acute interstitial pneumonia.

In the above embodiments, the pharmaceutical composition provided by the present invention can be used as a pharmaceutical composition for the following purposes.

In one embodiment, the pharmaceutical composition provided by the present invention can be used as a pharmaceutical composition for preventing or treating the respiratory disease accompanied by depression of respiratory function.

In one embodiment, the pharmaceutical composition provided by the present invention can be used as a pharmaceutical composition for preventing or treating the respiratory disease accompanied by airflow obstruction.

In one embodiment, the pharmaceutical composition provided by the present invention can be used as a pharmaceutical composition for preventing or treating the respiratory disease accompanied by respiratory inflammation.

In one embodiment, the pharmaceutical composition provided by the present invention can be used as a pharmaceutical composition for preventing or treating the respiratory disease accompanied by cough reflex.

In the present specification, "airflow obstruction" refers to narrowing of the airway and bronchi, which are passages for the air, that causes inability to exhale quickly.

In the present specification, the term "prevention" is a concept including preventing onset of a "diseased" or "abnormal" symptom, state, or disease before an outbreak thereof and an action or a method therefor.

In the present specification, examples of the "diseased" or "abnormal" symptom, state, or disease include, in particular, "shortness of breath", in other words, rapid breathing (tachypnea), inability to sufficiently inhale (condition in which tidal volume is limited), inability to sufficiently exhale (expiration impairment), being in need of respiratory effort (condition in which effort is required for contracting muscle of respiration), breathless, a condition in which more breathing is desired (condition in which increase in tidal volume is limited), heavy breathing, or a condition of panting or wheezing (symptoms after maximum exercise intensity).

In the present specification, "treatment" in one embodiment is eliminating, completely curing, healing, or remitting the "diseased" or "abnormal" symptom, state, or disease and an action or a method therefor. In another embodiment, "treatment" is eliminating, completely curing, healing, or remitting the "diseased" or "abnormal" symptom, state, or disease. In still another embodiment, "treatment" is a concept including eliminating, completely curing, healing, or remitting the "diseased" or "abnormal" symptom, state, or disease and an action or a method therefor, suppressing exacerbation of the "diseased" or "abnormal" symptom, state, or disease and an action or a method therefor, and improvement.

Here, the term "improvement" is a concept including approach of a "diseased" or "abnormal" symptom, state, or disease to a "healthy" or "normal" state or an action or a method therefor, and causing a "diseased" or "abnormal" symptom, state, or disease to be in a "healthy" or "normal" state or an action or a method therefor. Therefore, the term "improvement" in one embodiment includes a concept in which a numerical value which serves as an indicator of a "diseased" or "abnormal" symptom or state becomes small or large so as to approach a normal value or be the normal value in accordance with the "improvement". Furthermore, the term "suppression" is a concept including stopping or slowing down exacerbation or progression of a symptom, state, or disease and an action or a method therefor, and improving the symptom, state, or disease or an action or a method therefor. Here, the term "improvement" has the meaning described above. The expression "exacerbation or progression of symptom, state, or disease" includes exacerbation or progression of a "diseased" or "abnormal" symptom, state, or disease and exacerbation or progression from a "healthy" or "normal" state to a "diseased" or "abnormal" symptom, state, or disease. The term "suppression" in one embodiment is stopping or slowing down exacerbation or progression of a symptom, state, or disease or an action or a method therefor. The term "suppression" in another embodiment is stopping or slowing down exacerbation or progression of a symptom, state, or disease.

In the present specification, "X, Y, and/or Z" is defined as "at least one selected from the group consisting of X, Y, and Z".

As an active ingredient of the pharmaceutical composition of the present invention, compounds represented by the following general formulas (A) to (BII) or a pharmaceutically acceptable salt thereof can be used.

Abbreviations used in the tables shown below and the like are as follows. Me: methyl group, Et: ethyl group, Pr: n-propyl group, iPr: isopropyl group, tBu: tert-butyl group, Ac: acetyl group, Ph: phenyl group

In the tables shown below and the like, a substituent may be marked together with a position number indicating a substitution position of the substituent. In addition, in order to distinguish position numbers of positions that appear to be identical to each other in a chemical formula, one position number may be indicated with a prime symbol "'" for convenience, however, as long as a definitive structure for a compound name can be specified, position numbers may be indicated without using the prime symbol.

(A-1) A compound represented by the following general formula (A) or a pharmaceutically acceptable salt thereof: 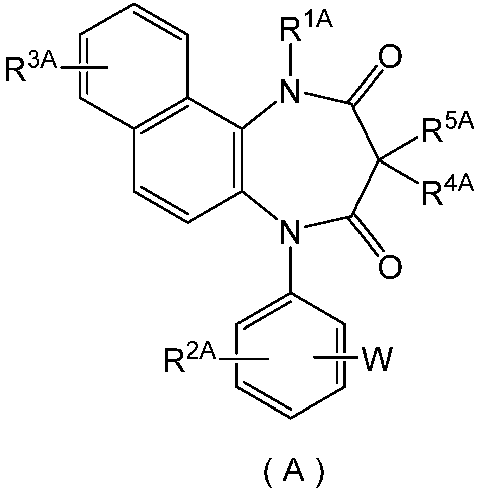
(in the formula, R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group;
R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group;
R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms and substituted with a phenyl group; and
W^{A} represents a five or six membered heterocyclic ring comprising 1 to 4 nitrogen atoms as the members of the ring, which may have a substituent)

In the general formula (A), examples of the alkyl group having 1 to 8 carbon atoms represented by R^{1A}, R^{2A}, R^{3A}, R^{4A}, and R^{5A} can comprise a methyl group, an ethyl group, a propyl group, an isopropyl group, a butyl group, an i-butyl group, a t-butyl group, a pentyl group, a hexyl group, and the like.

Examples of the alkenyl group having 2 to 8 carbon atoms represented by R^{1A} can comprise an allyl group and the like.

Examples of the alkyl group having 1 to 8 carbon atoms and substituted with 1 to 3 halogen atoms represented by R^{1A}, R^{2A}, R^{3A}, R^{4A}, and R^{5A} or R^{11A}, R^{12A}, R^{13A}, R^{14A}, and R^{15A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkyl group having 1 to 3 carbon atoms and substituted with a phenyl group represented by R^{1A}, R^{4A}, and R^{5A} can comprise a benzyl group and the like.

Examples of the alkoxy group having 1 to 8 carbon atoms represented by R^{2A} and R^{3A} can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkoxy group having 1 to 8 carbon atoms and substituted with 1 to 3 halogen atoms represented by R^{2A} and R^{3A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethoxy group, a 2-chloroethoxy group, a 2-bromoethoxy group, a 2-fluoroethoxy group, and the like.

Examples of the halogen atom represented by R^{2A} and R^{3A} can comprise a fluorine atom, a chlorine atom, a bromine atom, and the like.

Examples of the alkylamino group having 1 to 8 carbon atoms represented by R^{2A} and R^{3A} can comprise a methylamino group, an ethylamino group, and the like.

Examples of the dialkylamino group having 1 to 8 carbon atoms represented by R^{2A} and R^{3A} can comprise a dimethylamino group, a diethylamino group, and the like.

Examples of the acylamino group having 2 to 8 carbon atoms represented by R^{2A} and R^{3A} can comprise an acetylamino group.

Examples of the acylamino group having 2 to 8 carbon atoms and substituted with 1 to 3 halogen atoms represented by R^{2A} and R^{3A} can comprise a trifluoromethylcarbonylamino group.

Examples of the alkylsulfonylamino group having 1 to 8 carbon atoms represented by R^{2A} and R^{3A} can comprise a methylsulfonylamino group.

Examples of the acyl group having 2 to 8 carbon atoms represented by R^{2A} and R^{3A} can comprise an acetyl group.

Examples of the alkoxycarbonyl group (the alkoxy moiety has 1 to 8 carbon atoms) represented by R^{2A} and R^{3A} can comprise a methoxycarbonyl group, an ethoxycarbonyl group, and the like.

Examples of the alkylthio group having 1 to 8 carbon atoms represented by R^{2A} and R^{3A} can comprise a methylthio group.

Examples of the alkylsulfinyl group having 1 to 8 carbon atoms represented by R^{2A} and R^{3A} can comprise a methylsulfinyl group.

Examples of the alkylsulfonyl group having 1 to 8 carbon atoms represented by R^{2A} and R^{3A} can comprise a methylsulfonyl group.

Examples of the five or six membered heterocyclic ring comprising 1 to 4 nitrogen atoms as the members of the ring, which may have a substituent, represented by W^{A} can comprise tetrazole, 1,2,4-triazole, 1,2,3-triazole, 1,2,4-oxadiazole, pyrazole, imidazole, oxazole, isoxazole, pyrrole, thiazole, pyridine, and pyrrolidine.

Examples of the substituent that may be comprised in the five or six membered heterocyclic ring comprising 1 to 4 nitrogen atoms as the members of the ring, which may have a substituent, represented by W^{A} can comprise an alkyl group having 1 to 8 carbon atoms such as a methyl group and an ethyl group, an alkyl group having 1 to 8 carbon atoms and substituted with 1 to 3 halogen atoms such as a trifluoromethyl group, a halogen atom such as a fluorine atom, a cyano group, an oxo group, a thioxo group, and the like.

R^{2A} and R^{3A} in the general formula (A) may have 1 to 3 same or different ones in the benzene ring substituted by R^{2A} and R^{3A}.

As the compound represented by the general formula (A), the following compounds are preferable.

(A-2) The compound according to (A-1), in which W^{A} represents tetrazole, 1,2,4-triazole, 1,2,3-triazole, 1,2,4-oxadiazole, pyrazole, or imidazole that may have a substituent selected from an alkyl group having 1 to 8 carbon atoms, an alkyl group having 1 to 8 carbon atoms and substituted with 1 to 3 halogen atoms, a halogen atom, a cyano group, an oxo group, and a thioxo group.

(A-3) The compound according to (A-1) or (A-2), in which W^{A} represents tetrazole, 1,2,4-triazole, or 1,2,3-triazole that may have a substituent selected from an alkyl group having 1 to 8 carbon atoms, an alkyl group having 1 to 8 carbon atoms and substituted with 1 to 3 halogen atoms, a halogen atom, and a cyano group.

(A-4) The compound according to any one of (A-1) to (A-3), in which W^{A} represents 5-oxo-1,2,4-oxadiazole or 5-thioxo-1,2,4-oxadiazole.

(A-5) The compound according to any one of (A-1) to (A-4), in which W^{A} represents tetrazole.

(A-6) The compound according to any one of (A-1) to (A-5), in which R^{1A} represents a hydrogen atom or an alkyl group having 1 to 8 carbon atoms.

(A-7) The compound according to any one of (A-1) to (A-6), in which R^{1A} represents a hydrogen atom.

(A-8) The compound according to any one of (A-1) to (A-7), in which R^{4A} represents a hydrogen atom, and R^{5A} represents a hydrogen atom or an alkyl group having 1 to 8 carbon atoms.

(A-9) The compound according to any one of (A-1) to (A-8), in which R^{4A} and R^{5A} both represent hydrogen atoms.

(A-10) The compound according to any one of (A-1) to (A-9), in which R^{2A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).

(A-11) The compound according to any one of (A-1) to (A-10), in which R^{2A} represents a hydrogen atom.

(A-12) The compound according to any one of (A-1)(A-11), in which R^{3A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).

(A-13) The compound according to any one of (A-1) to (A-12), in which R^{3A} represents a hydrogen atom.

Examples of the pharmaceutically acceptable salt of the compound represented by the general formula (A) can comprise hydrochloride and the like, in a case where R^{2A} and R^{3A} in the general formula (A) represent amino groups or the like. In addition, in a case where R^{2A} and R^{3A} in the general formula (A) represent carboxyl groups, examples of the pharmaceutically acceptable salt of the compound represented by the general formula (A) can comprise a salt of an alkali metal such as sodium, potassium, and lithium.

Typical compounds comprised in the compounds represented by the general formula (A) are as follows.

### <Typical Compound A-100>

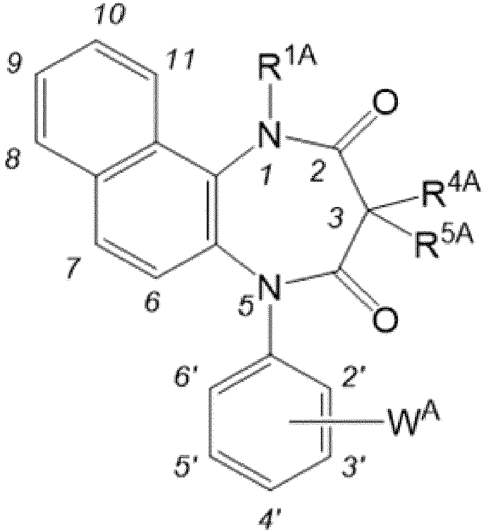

(R^{1A,} R^{4A}, R^{5A}, and W^{A} in the formula and a substitution position of W^{A} are indicated in Tables 1 to 3)

In Tables 1 to 3, the substitution position of W^{A} indicates a substitution position on a benzene ring. That is, positions 2, 3, and 4 in the tables correspond to positions 2', 3', and 4' in a formula of Typical Compound A-100, respectively.

**[Table 1]**

| R¹ | Position of W | W | R⁴/R⁵ |
|---|---|---|---|
| H | 2- | 1H-tetrazol-5-yl | H/H |
| H | 3- | 1H-tetrazol-5-yl | H/H |
| H | 3- | (1-methyl-1H-tetrazol)-5-yl | H/H |
| H | 4- | 1H-tetrazol-5-yl | H/H |
| CH₃ | 3- | 1H-tetrazol-5-yl | H/H |
| CH₃ | 3- | 1H-tetrazol-5-yl | CH₃/H |
| benzyl | 3- | 1H-tetrazol-5-yl | H/H |
| H | 3- | 1H-tetrazol-1-yl | H/H |
| H | 3- | 1H-tetrazol-1-yl | CH₃/CH₃ |
| H | 3- | (1,2,3-triazol)-5-yl | H/H |
| H | 3- | (1,2,4-triazol)-3-yl | H/H |
| H | 4- | (1,2,4-triazol)-3-yl | H/H |

**[Table 2]**

| R¹ | Position of W | W | R⁴/R⁵ |
|---|---|---|---|
| H | 2- | (1,2,4-triazol)-1-yl | H/H |
| H | 3- | (1,2,4-triazol)-1-yl | H/H |
| H | 3- | [5-(trifluoromethyl)-1,2,4-triazol]-3-yl | H/H |
| H | 3- | [5-(trifluoromethyl)-1,2,4-triazol]-3-yl | ethyl/H |
| H | 3- | [5-fluoro-1,2,3-triazol]-4-yl | H/H |
| H | 3- | [5-fluoro-1,2,3-triazol]-4-yl | CH₃/CH₃ |
| H | 3- | [5-cyano-1,2,3-triazol]-4-yl | H/H |
| H | 4- | 1H-imidazol-1-yl | H/H |
| H | 4- | 1H-imidazol-1-yl | Pr/H |
| H | 3- | 1H-imidazol-2-yl | H/H |
| H | 3- | 1H-imidazol-4-yl | H/H |
| H | 3- | imidazolin-2-yl | H/H |

**[Table 3]**

| R¹ | Position of W | W | R⁴/R⁵ |
|---|---|---|---|
| H | 2- | pyrazol-3-yl | H/H |
| H | 3- | pyrazol-4-yl | H/H |
| H | 3- | pyrazol-5-yl | CH₃/H |
| H | 3- | (1,2,4-oxadiazol)-3-yl | H/H |
| H | 3- | (1,3,4-oxadiazol)-2-yl | H/H |
| H | 3- | (5-oxo-1,2,4-oxadiazol)-3-yl | H/H |
| H | 3- | pyrrol-1-yl | H/H |
| H | 4- | pyrrolidin-2-yl | H/H |
| CH₃ | 4- | pyrrolidin-2-yl | CH₃/H |
| H | 4- | (1,3-oxazol)-5-yl | H/H |
| H | 3- | (1,3-oxazol)-5-yl | H/H |
| H | 2- | (1,3-thiazol)-5-yl | H/H |

### <Typical Compound A-200>

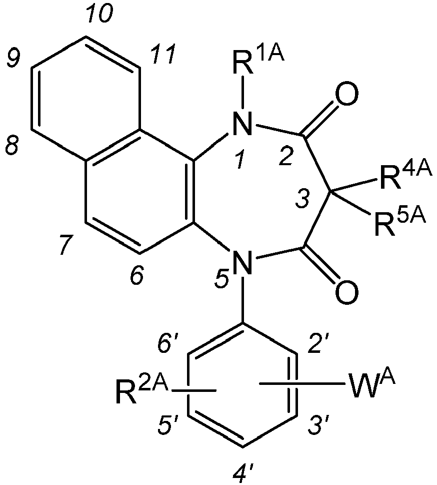

(R^{1A}, R^{2A}, R^{4A}, R^{5A}, and W^{A} in the formula and a substitution position of W^{A} are indicated in Tables 4 and 5)

In Tables 4 and 5, the substitution position of W^{A} indicates a substitution position on a benzene ring. That is, positions 2, 3, and 4 in the tables correspond to positions 2', 3', and 4' in a formula of Typical Compound A-200, respectively.

**[Table 4]**

| R¹ | R² | Position of W | W | R⁴/R⁵ |
|---|---|---|---|---|
| H | 4-OH | 3- | 1H-tetrazol-5-yl | H/H |
| H | 4-OCH₃ | 3- | 1H-tetrazol-5-yl | H/H |
| CH₃ | 2-Cl | 3- | 1H-tetrazol-5-yl | H/H |
| H | 2,6-Cl | 3- | 1H-tetrazol-5-yl | H/H |
| H | 4-F | 3- | 1H-tetrazol-5-yl | H/H |
| H | 4-Br | 3- | 1H-tetrazol-5-yl | ethyl/H |
| H | 3-OCH₃ | 4- | (1-methyl-1H-tetrazol) -5-yl | H/H |
| H | 4-CH₃ | 3- | 1H-tetrazol-5-yl | H/H |

**[Table 5]**

| R¹ | R² | Position of W | W | R⁴/R⁵ |
|---|---|---|---|---|
| H | 4-Cl | 3- | (1,2,3-triazol)-5-yl | CH₃/H |
| H | 4-CF₃ | 3- | (1,2,3-triazol)-5-yl | H/H |
| H | 3-SCH₃ | 4- | (1,2,4-triazol)-1-yl | H/H |
| H | 3-SO₂CH₃ | 4- | 1H-imidazol-1-yl | H/H |
| H | 3-NHSO₂CH₃ | 4- | 1H-imidazol-1-yl | H/H |
| H | 4-OCH₃ | 3- | 1H-imidazol-4-yl | H/H |
| H | 4-F | 2- | pyrazol-3-yl | H/H |

### <Typical Compound A-300>

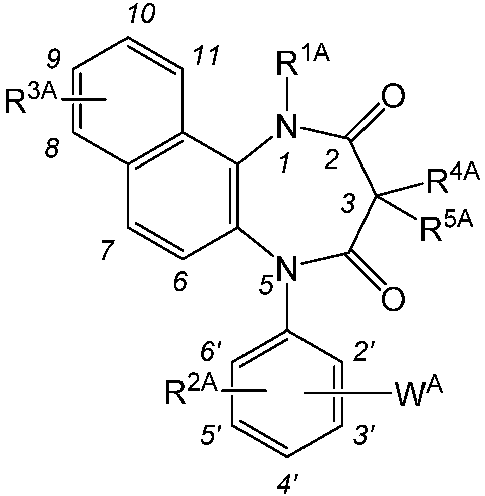

(R^{1A}, R^{2A}, R^{3A}, R^{4A}, R^{5A} and W^{A} in the formula and a substitution position of W^{A} are indicated in Tables 6 and 7)

In Tables 6 and 7, the substitution position of W^{A} indicates a substitution position on a benzene ring. That is, positions 3 and 4 in the tables correspond to positions 3' and 4' in a formula of Typical Compound A-300, respectively.

**[Table 6]**

| R¹ | R² | Position of W | W | R³ | R⁴/R⁵ |
|---|---|---|---|---|---|
| H | H | 3- | 1H-tetrazol-5-yl | 9-Br | H/H |
| H | 4-OCH₃ | 3- | 1H-tetrazol-5-yl | 9-Cl | H/H |
| H | 4-OH | 3- | 1H-tetrazol-5-yl | 10-OCH₃ | H/H |
| H | 2-Cl | 3- | 1H-tetrazol-5-yl | 9-Br | H/H |
| H | 2, 6-Cl | 3- | 1H-tetrazol-5-yl | 9-CH₃ | H/H |
| H | H | 3- | 1H-tetrazol-5-yl | 10-Cl | CH₃/H |
| H | 3-OCH₃ | 4- | (1-methyl-1H-tetrazol) -5-yl | 9-CF₃ | H/H |

**[Table 7]**

| R¹ | R² | Position of W | W | R³ | R⁴/R⁵ |
|---|---|---|---|---|---|
| H | 4-CH₃ | 3- | 1H-tetrazol-1-yl | 9-CN | Pr/H |
| CH₃ | H | 3- | (1,2,3-triazol)-5-yl | 9-OH | H/H |
| ethyl | H | 3- | (1,2,3-triazol)-5-yl | 10-F | H/H |
| H | 3-Br | 4- | (1,2,4-triazol)-1-yl | 9-SCH₃ | H/H |
| allyl | H | 4- | 1H-imidazol-1-yl | 8-OCH₃ | H/H |
| H | H | 3- | 1H-imidazol-1-yl | 10-OCH₃ | CH₃/CH₃ |

(B-1) A compound represented by the following general formula (BI) or a pharmaceutically acceptable salt thereof: 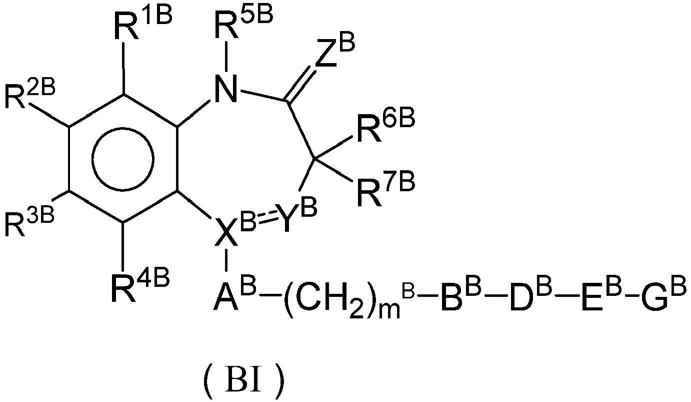
(in the formula, R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with the benzene ring to which they bind, and the ring constituted by R^{1B} and R^{2B} bound to each other, together with the carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
X^{B} represents C, CH, or N,
Y^{B} represents N, NH, or C(=O),
provided that when X^{B} is N, Y^{B} is not N or NH, and when X^{B} is C or CH, Y^{B} is not C(=O),
the double line consisting of the solid line and the broken line represents a single bond or a double bond,
Z^{B} represents an oxygen atom or a sulfur atom,
A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
B^{B} represents N(R^{8B})C(=O), NHCONH, CON(R^{9B}), NHC(=S)NH, N(R^{10B})SO₂, SO₂N(R^{11B}), or OSO₂, wherein
R^{8B}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
E^{B} represents O, S, NR^{12B}, or an atomic bond, wherein
R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
m^{B} represents an integer of 0 to 5,
provided that when R^{1B} and R^{2B} do not bind together to form a ring, those compounds are excluded wherein, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).

(B-2) A compound represented by the following general formula (BII) or a pharmaceutically acceptable salt thereof:

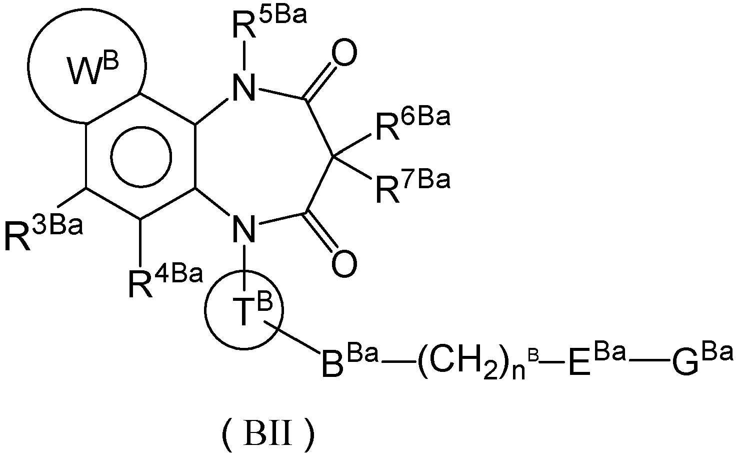

(In the formula, 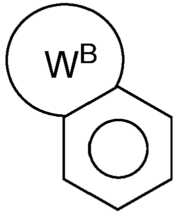
represents a naphthalene ring, a quinoline ring, an isoquinoline ring, a tetrahydronaphthalene ring, an indane ring, a tetrahydroquinoline ring, or a tetrahydroisoquinoline ring, and
these rings may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6Ba} and R^{7Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group, 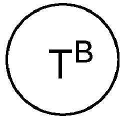
represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent,
B^{Ba} represents N(R^{8Ba})C(=O), NHCONH, CON(R^{9Ba}), NHC(=S)NH, N(R^{10Ba})SO₂, SO₂N(R^{11Ba}), or OSO₂, wherein
R^{8Ba}, R^{9Ba}, R^{10Ba}, and R^{11Ba} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
E^{Ba} represents O, S, NR^{12Ba}, or an atomic bond, wherein
R^{12Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
n^{B} represents an integer of 0 to 5).

Next, the substituents in the general formulas (BI) and (BII) of the present specification is described.

Examples of the alkyl group having 1 to 8 carbon atoms can comprise a methyl group, an ethyl group, a propyl group, an isopropyl group, a butyl group, an i-butyl group, a t-butyl group, a pentyl group, a hexyl group, and the like.

Examples of the cycloalkyl group having carbon atoms 3 to 8 can comprise a cyclopropyl group, a cyclohexyl group, and the like.

Examples of the alkenyl group having 2 to 8 carbon atoms can comprise an allyl group, and the like.

Examples of the alkoxy group having 1 to 8 carbon atoms can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkyl group having 1 to 8 carbon atoms and substituted with 1 to 3 halogen atoms can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkoxy group having 1 to 8 carbon atoms substituted with 1 to 3 halogen atoms can comprise a methoxy group, an ethoxy group, a propoxy group, an isopropoxy group, a butoxy group, a t-butoxy group, and the like substituted with 1 to 3 halogen atoms such as a fluorine atom, a chlorine atom, and a bromine atom, and preferable examples thereof can comprise a trifluoromethoxy group, a chloromethoxy group, a 2-chloroethoxy group, a 2-bromoethoxy group, a 2-fluoroethoxy group, and the like.

Examples of the halogen atom can comprise a fluorine atom, a chlorine atom, a bromine atom, and the like.

Examples of the alkylamino group having 1 to 8 carbon atoms can comprise a methylamino group, an ethylamino group, and the like.

Examples of the dialkylamino group having 2 to 8 carbon atoms can comprise a dimethylamino group, a diethylamino group, and the like.

Examples of the acylamino group having 2 to 8 carbon atoms can comprise an acetylamino group and the like.

Examples of the acyl group having 2 to 8 carbon atoms can comprise an acetyl group and the like.

Examples of the alkoxycarbonyl group (the alkoxy moiety has 1 to 8 carbon atoms) can comprise a methoxycarbonyl group and the like.

Examples of the aralkyl group (the aryl moiety has 6 to 10 carbon atoms, and the alkylene moiety has 1 to 8 carbon atoms) can comprise a benzyl group and the like.

Examples of the alkyl group having 1 to 8 carbon atoms and substituted with a hydroxyl group can comprise a 2-hydroxyethyl group and the like.

Examples of the alkylsulfinyl group having 1 to 6 carbon atoms can comprise a methanesulfinyl group and the like.

Examples of the alkylthio group having 1 to 6 carbon atoms can comprise a methylthio group and the like.

Examples of the alkylsulfonyl group having 1 to 6 carbon atoms can comprise a methanesulfonyl group and the like.

Examples of the substituent that may be comprised in the phenyl group which may be substituted, the pyridyl group which may be substituted, the imidazolyl group which may be substituted, the oxazolyl group which may be substituted, and the thiazolyl group which may be substituted can comprise a halogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, and the like.

As the compound represented by the general formula (BI), the following compounds are preferable.

### (B-1-1)

The compound according to (B-1), in which R^{1B} and R^{2B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a phenyl group which may be substituted, a pyridyl group which may be substituted, or an aralkyl group (the aryl moiety has 6 to 10 carbon atoms, and the alkylene moiety has 1 to 8 carbon atoms).

### (B-1-2)

The compound according to (B-1) or (B-1-1), in which R^{1B} and R^{2B} bind together to form a naphthalene ring or a tetrahydronaphthalene ring together with the benzene ring to which they bind, and the benzene ring or the cyclohexene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),

### (B-1-3)

The compound according to (B-1) or (B-1-1), in which R^{1B} and R^{2B} bind together to form a naphthalene ring together with the benzene ring to which they bind, and the benzene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group.

### (B-1-4)

The compound according to (B-1) or (B-1-1), in which R^{1B} and R^{2B} bind together to form a naphthalene ring or a tetrahydronaphthalene ring together with the benzene ring to which they bind.

### (B-1-5)

The compound according to any one of (B-1) and (B-1-1) to (B-1-4), in which R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-1-6)

The compound according to any one of (B-1) and (B-1-1) to (B-1-5), in which R^{5B} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-1-7)

The compound according to any one of (B-1) and (B-1-1) to (B-1-6), in which R^{5B} represents a hydrogen atom.

### (B-1-8)

The compound according to any one of (B-1) and (B-1-1) to (B-1-7), in which R^{6B} and R^{7B} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms, or a pharmaceutically acceptable salt thereof.

### (B-1-9)

The compound according to any one of (B-1) and (B-1-1) to (B-1-8), in which R^{6B} and R^{7B} both represent hydrogen atoms.

### (B-1-10)

The compound according to any one of (B-1) and (B-1-1) to (B-1-9), in which R^{3B}, R^{4B}, R^{5B}, R^{6B}, and R^{7B} are hydrogen atoms.

### (B-1-11)

The compound according to any one of (B-1) and (B-1-1) to (B-1-10), in which X^{B} represents N, Y^{B} represents C(=O), and the double line consisting of the solid line and the broken line represents a single bond.

### (B-1-12)

The compound according to any one of (B-1) and (B-1-1) to (B-1-11), in which X^{B} represents C, Y^{B} represents N, and the double line consisting of the solid line and the broken line represents a double bond.

### (B-1-13)

The compound according to any one of (B-1) and (B-1-1) to (B-1-12), in which Z^{B} represents an oxygen atom.

### (B-1-14)

The compound according to any one of (B-1) and (B-1-1) to (B-1-13), in which A^{B} represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-1-15)

The compound according to any one of (B-1) and (B-1-1) to (B-1-14), in which A^{B} represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-1-16)

The compound according to any one of (B-1) and (B-1-1) to (B-1-15), in which A^{B} represents a phenyl group or a pyridyl group.

### (B-1-17)

The compound according to any one of (B-1) and (B-1-1) to (B-1-16), in which A^{B} represents an atomic bond.

### (B-1-18)

The compound according to any one of (B-1) and (B-1-1) to (B-1-17), in which B^{B} represents NHC(=O), NHCONH, CONH, NHC(=S)NH, NHSO₂, SO₂NH, or OSO₂.

### (B-1-19)

The compound according to any one of (B-1) and (B-1-1) to (B-1-18), in which B^{B} represents NHC(=O), NHCONH, or NHSO₂.

### (B-1-20)

The compound according to any one of (B-1) and (B-1-1) to (B-1-19), in which B^{B} represents NHC(=O) or NHSO₂.

### (B-1-21)

The compound according to any one of (B-1) and (B-1-1) to (B-1-20), in which B^{B} represents NHC(=O).

### (B-1-22)

The compound according to any one of (B-1) and (B-1-1) to (B-1-21), in which D^{B} represents an alkylene chain having 1 to 6 carbon atoms that may have 1 to 4 of the same or different substituents selected from an alkyl group having 1 to 8 carbon atoms and an alkyl group having 1 to 8 carbon atoms and substituted with 1 to 3 halogen atoms as a substituent, and may further have a double bond.

### (B-1-23)

The compound according to any one of (B-1) and (B-1-1) to (B-1-22), in which D^{B} represents an atomic bond.

### (B-1-24)

The compound according to any one of (B-1) and (B-1-1) to (B-1-23), in which D^{B} has 1 to 4 of the same or different substituents selected from an alkyl group having 1 to 8 carbon atoms and an alkenyl group having 2 to 8 carbon atoms as a substituent.

### (B-1-25)

The compound according to any one of (B-1) and (B-1-1) to (B-1-24), in which D^{B} has 1 to 4 of the same or different substituents selected from an alkyl group having 1 to 3 carbon atoms and an alkenyl group having 2 or 3 carbon atoms as a substituent.

### (B-1-26)

The compound according to any one of (B-1) and (B-1-1) to (B-1-25), in which E^{B} represents an atomic bond.

### (B-1-27)

The compound according to any one of (B-1) and (B-1-1) to (B-1-26), in which G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-28)

The compound according to any one of (B-1) and (B-1-1) to (B-1-27), in which G^{B} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-29)

The compound according to any one of (B-1) and (B-1-1) to (B-1-28), in which G^{B} represents benzene or pyridine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-30)

The compound according to any one of (B-1) and (B-1-1) to (B-1-29), in which G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-31)

The compound according to any one of (B-1) and (B-1-1) to (B-1-30), in which m^{B} represents 0.

### (B-1-32)

The compound according to any one of (B-1) and (B-1-1) to (B-1-31), in which A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-33)

The compound according to any one of (B-1) and (B-1-1) to (B-1-32), in which A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-34)

The compound according to any one of (B-1) and (B-1-1) to (B-1-33), in which R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-35)

The compound according to any one of (B-1) and (B-1-1) to (B-1-34), in which, in the general formula (BI), R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

As the compound represented the general formula (BII), the following compounds are preferable.

### (B-2-1)

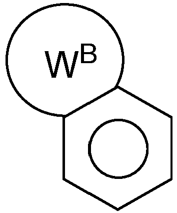

The compound according to (B-2) in which the above moiety represents a naphthalene ring or a tetrahydronaphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms) as a substituent.

### (B-2-2)

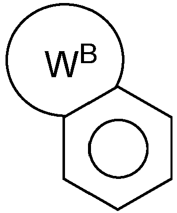

The compound according to (B-2) or (B-2-1) in which the above moiety represents a naphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group as a substituent.

### (B-2-3)

The compound according to any one of (B-2), (B-2-1), and (B-2-2) in which R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-2-4)

The compound according to any one of (B-2) and (B-2-1) to (B-2-3), in which R^{5Ba} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-2-5)

The compound according to any one of (B-2) and (B-2-1) to (B-2-4), in which R^{5Ba} represents a hydrogen atom.

### (B-2-6)

The compound according to any one of (B-2) and (B-2-1) to (B-2-5), in which R^{6Ba} and R^{7Ba} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms.

### (B-2-7)

The compound according to any one of (B-2) and (B-2-1) to (B-2-6), in which R^{6Ba} and R^{7Ba} both represent hydrogen atoms.

### (B-2-8)

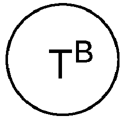

The compound according to any one of (B-2) and (B-2-1) to (B-2-7), in which the above moiety represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-2-9)

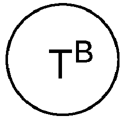

The compound according to any one of (B-2) and (B-2-1) to (B-2-8), in which the above moiety represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-2-10)

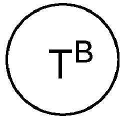

The compound according to any one of (B-2) and (B-2-1) to (B-2-9), in which the above moiety represents an atomic bond.

### (B-2-11)

The compound according to any one of (B-2) and (B-2-1) to (B-2-10), in which B^{Ba} represents NHC(=O), NHCONH, CONH, NHC(=S)NH, NHSO₂, SO₂NH, or OSO₂.

### (B-2-12)

The compound according to any one of (B-2) and (B-2-1) to (B-2-11), in which B^{Ba} represents NHC(=O), NHCONH, or NHSO₂.

### (B-2-13)

The compound according to any one of (B-2) and (B-2-1) to (B-2-12), in which E^{Ba} represents an atomic bond.

### (B-2-14)

The compound according to any one of (B-2) and (B-2-1) to (B-2-13), in which G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-15)

The compound according to any one of (B-2) and (B-2-1) to (B-2-14), in which G^{Ba} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-16)

The compound according to any one of (B-2) and (B-2-1) to (B-2-15), in which n^{B} represents 0.

In the general formula (BI), it is preferable that R^{B1} and R^{B2} bind together to form a condensed ring selected from a naphthalene ring or a tetrahydronaphthalene ring together with the benzene ring to which they bind, and it is particularly preferable that R^{B1} and R^{B2} bind together to form a naphthalene ring together with the benzene ring to which they bind.

In the general formula (BI), it is preferable that R^{B3}, R^{B4}, R^{B5}, R^{B6}, and R^{B7} represent hydrogen atoms.

In the general formula (BI), it is preferable that X^{B} represents N, Y^{B} represents C(=O), and the double line consisting of the solid line and the broken line represents a single bond.

In the general formula (BI), it is preferable that Z^{B} represents an oxygen atom.

In the general formula (BI), it is preferable that A^{B} represents a benzene ring or a pyridine ring, and it is particularly preferable that A^{B} represents a benzene ring.

In the general formula (BI), it is preferable that m^{B} represents 0 to 4, and it is particularly preferable that m^{B} represents 0.

In the general formula (BI), it is preferable that B^{B} represents N(R^{8B})C(=O) or N(R^{10B})SO₂, and in this case, it is more preferable that R^{8B} and R^{10B} represent hydrogen atoms. Furthermore, in the general formula (BI), it is particularly preferable that B^{B} represents NHC(=O).

In the general formula (BI), it is preferable that D^{B} represents an alkylene chain having 1 to 6 carbon atoms which has 1 to 4 of the same or different substituents selected from an alkyl group having 1 to 8 carbon atoms and an alkenyl group having 2 to 8 carbon atoms as a substituent, or represents an atomic bond, it is more preferable that D^{B} represents an alkylene chain having 1 to 8 carbon atoms which has 1 to 4 of the same or different substituents selected from an alkyl group having 1 to 3 carbon atoms and an alkenyl group having 2 or 3 carbon atoms as a substituent, or represents an atomic bond, and it is particularly preferable that D^{B} represents an atomic bond.

In the general formula (BI), it is preferable that E^{B} represents O or an atomic bond, and it is particularly preferable that E^{B} represents an atomic bond.

In the general formula (BI), it is preferable that G^{B} represents benzene or pyridine which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent, and it is particularly preferable that G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is more preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

Typical compounds comprised in the compounds represented by the general formulas (BI) and/or (BII) are as follows.

### <Typical Compound Example B-100>

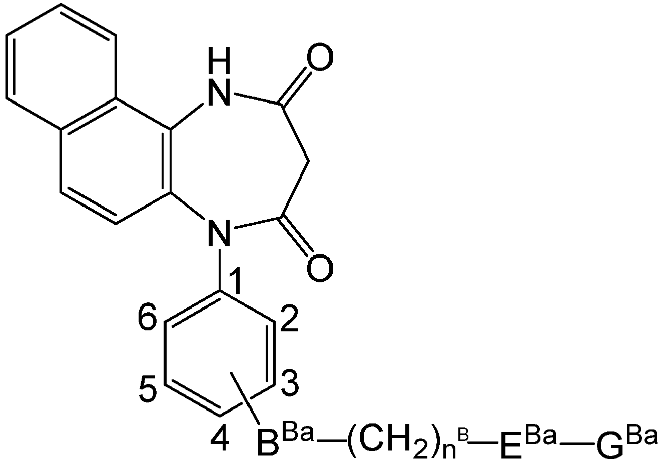

(B^{Ba} (substitution position), n^{B}, E^{Ba}, and G^{Ba} in the formula are indicated in Tables 8 to 17)

**[Table 8]**

| B^{Ba} (substitution position) | n^{B} | E^{Ba} | G^{Ba} |
|---|---|---|---|
| NHCO(4) | 0 | Atomic bond | Phenyl |
| NHCO(4) | 0 | Atomic bond | (2-CF₃)Phenyl |
| NHCO(4) | 0 | Atomic bond | (3-Br)Phenyl |
| NHCO(4) | 0 | Atomic bond | (4-CF₃)Phenyl |
| NHCO(4) | 0 | Atomic bond | (2-Me)Phenyl |
| NHCO(4) | 0 | Atomic bond | (2,6-Me)Phenyl |
| NHCO(4) | 0 | Atomic bond | (2,6-Cl)Phenyl |
| NHCO(4) | 0 | Atomic bond | (3-Cl)Phenyl |
| NHCO(4) | 1 | Atomic bond | Phenyl |
| NHC(=S)NH(4) | 0 | Atomic bond | Phenyl |
| NHCO(4) | 0 | Atomic bond | (2,3-OMe)Phenyl |
| NHCO(4) | 0 | Atomic bond | (2-OMe)Phenyl |
| NHCO(4) | 1 | Atomic bond | (2-Cl)Phenyl |
| NHCO(4) | 0 | Atomic bond | (2,3-Me)Phenyl |
| NHCO(4) | 0 | Atomic bond | (2,5-Me)Phenyl |
| NHCO(4) | 0 | Atomic bond | (2-Cl, 5-Br)Phenyl |

**[Table 9]**

| B^{Ba} (substitution position) | n^{B} | E^{Ba} | G^{Ba} |
|---|---|---|---|
| NHCO(4) | 0 | Atomic bond | (2,4-Cl)Phenyl |
| NHCO(4) | 0 | Atomic bond | (2-OH)Phenyl |
| NHCO(4) | 0 | Atomic bond | (2,3-OH)Phenyl |
| NHC(=O)NH(4) | 0 | Atomic bond | Phenyl |
| NHCO(4) | 1 | Atomic bond | (2,6-Cl)Phenyl |
| NHCO(4) | 1 | Atomic bond | (2-OMe)Phenyl |
| NHCO(4) | 1 | Atomic bond | (2-OH)Phenyl |
| NHC(=S)NH(4) | 0 | Atomic bond | (2-Cl)Phenyl |
| NHCO(4) | 0 | Atomic bond | (3-CF₃)Phenyl |
| NHCO(4) | 1 | Atomic bond | (2-CF₃)Phenyl |
| NHC(=O)NH(4) | 0 | Atomic bond | (2-Cl)Phenyl |
| NHCO(4) | 0 | Atomic bond | (2-Cl,3-OMe)Phenyl |
| NHCO(4) | 2 | Atomic bond | Phenyl |
| NHCO(4) | 0 | Atomic bond | 3-indolyl |
| NHCO(4) | 0 | Atomic bond | (2-Cl,3-OH)Phenyl |
| NHCO(4) | 1 | O | Phenyl |

**[Table 10]**

| B^{Ba} (substitution position) | n^{B} | E^{Ba} | G^{Ba} |
|---|---|---|---|
| NHCO(4) | 1 | Atomic bond | (2-Cl,4-OMe)Phenyl |
| NHCO(4) | 0 | Atomic bond | (1-Me)imidazol 2-yl |
| NHCO(4) | 1 | Atomic bond | (2,4-Cl)Phenyl |
| NHCO(4) | 1 | Atomic bond | (2-Cl,4-OH)Phenyl |
| NHCO(4) | 1 | Atomic bond | pyridin 3-yl |
| NHCO(4) | 0 | Atomic bond | Benzimidazol 2-yl |
| NHCO(4) | 0 | Atomic bond | (2-Cl) Phenyl |
| NHCO(4) | 0 | Atomic bond | (2-Br)Phenyl |
| NHCO(4) | 0 | Atomic bond | (2-I)Phenyl |
| NHCO(4) | 1 | Atomic bond | (2-Me) Phenyl |
| NHCO(4) | 0 | Atomic bond | quinoxalin 2-yl |
| NHCO(4) | 0 | Atomic bond | (5-Me)thiophen 2-yl |
| NHCO(3) | 1 | Atomic bond | (2-Cl)Phenyl |
| NHCO(4) | 0 | Atomic bond | (2,4,6-Me)Phenyl |
| NHCO(4) | 0 | Atomic bond | (2-Et)Phenyl |
| NHC(=S)NH(4) | 0 | Atomic bond | (2-Me)Phenyl |

**[Table 11]**

| B^{Ba} (substitution position) | n^{B} | E^{Ba} | G^{Ba} |
|---|---|---|---|
| NHCO(4) | 0 | Atomic bond | (4-NMe₂)Phenyl |
| NHCO(4) | 1 | O | (2,4-Cl)Phenyl |
| NHCO(4) | 1 | O | (2-Me)Phenyl |
| NHCO(4) | 0 | Atomic bond | (2-Ac)Phenyl |
| NHCO(4) | 0 | Atomic bond | (2-tBu)Phenyl |
| NHCO(3) | 0 | Atomic bond | (2-I)Phenyl |
| NHCO(4) | 0 | Atomic bond | (1-Me) piperidin 4-yl |
| NHCO(4) | 0 | Atomic bond | benzofuran 2-yl |
| NHCO(4) | 0 | Atomic bond | (1-Me) indol 3-yl |
| NHCO(4) | 0 | Atomic bond | (2-allyl)Phenyl |
| NHCO(4) | 0 | Atomic bond | (2-nPr)Phenyl |
| NHCO(4) | 0 | Atomic bond | (2-iPrO)Phenyl |
| NHCO(4) | 0 | Atomic bond | 3-Me thiophen 2-yl |
| NHCO(4) | 1 | O | (2-Me,3-Cl)Phenyl |
| NHCO(4) | 0 | Atomic bond | (2-CF₃,4-F)Phenyl |
| NHCO(4) | 0 | Atomic bond | (2-OMe,4-F)Phenyl |

**[Table 12]**

| B^{Ba} (substitution position) | n^{B} | E^{Ba} | G^{Ba} |
|---|---|---|---|
| NHCO(4) | 0 | Atomic bond | (2-OH, 4-F)Phenyl |
| NHCO(3) | 1 | Atomic bond | (2-I)Phenyl |
| NHCO(4) | 0 | Atomic | (3-NMe₂)Phenyl |
| | | bond | |
| NHCO(4) | 0 | Atomic bond | (2-OMe,4-I)Phenyl |
| NHCO(4) | 0 | Atomic bond | (2-OMe,6-F)Phenyl |
| NHCO(4) | 0 | Atomic bond | (2-OH,4-I)Phenyl |
| NHCO(4) | 0 | Atomic bond | (2-OH, 6-F)Phenyl |
| NHCO(4) | 0 | Atomic bond | (2-F)Phenyl |
| NHCO(4) | 0 | Atomic bond | (2-NMe₂)Phenyl |
| NHCO(4) | 0 | Atomic bond | (2-OMe,6-Me)Phenyl |
| NHCO(4) | 0 | Atomic bond | (2-OH,6-Me)Phenyl |
| NHCO(4) | 2 | Atomic bond | (2-Me) Phenyl |
| CONH(4) | 0 | Atomic bond | Phenyl |
| CONH(4) | 1 | Atomic bond | Phenyl |
| NHCO(4) | 2 | Atomic bond | (2-Cl) Phenyl |
| CONH(4) | 1 | Atomic bond | (2-Cl)Phenyl |

**[Table 13]**

| B^{Ba} (substitution position) | n^{B} | E^{Ba} | G^{Ba} |
|---|---|---|---|
| CONH(4) | 0 | Atomic bond | (2-Cl)Phenyl |
| NHCO(4) | 0 | Atomic bond | (5-Br,2,3-methylenedioxy) Phenyl |
| NHCO(4) | 0 | Atomic bond | (2-OMe,6-Br)Phenyl |
| NHCO(4) | 0 | Atomic bond | (2-OH,6-Br)Phenyl |
| NHCO(4) | 0 | Atomic bond | (2-OMe,6-Cl)Phenyl |
| NHCO(4) | 0 | Atomic bond | (2-OH, 6-Cl)Phenyl |
| NHCO(4) | 0 | Atomic bond | (2-OH, 6-OMe)Phenyl |
| NHCO(4) | 0 | Atomic bond | (2-OMe, 6-CF₃)Phenyl |
| NHCO(4) | 0 | Atomic bond | (2-OH, 6-CF₃)Phenyl |
| NHCO(4) | 0 | Atomic bond | (2-Cl, 5-SMe)Phenyl |
| NHCO(4) | 0 | Atomic bond | (2-SMe)Phenyl |
| NHCO(4) | 0 | Atomic bond | (3-SMe)Phenyl |
| NHCO(4) | 0 | Atomic bond | (2-OMe, 6-Et)Phenyl |
| NHCO(4) | 0 | Atomic bond | (3-SO₂Me)Phenyl |
| NHCO(4) | 0 | Atomic bond | (2-OH, 6-Et)Phenyl |
| NHCO(4) | 0 | Atomic bond | (3 - S (=O) Me) Phenyl |

**[Table 14]**

| B^{Ba} (substitution position) | nB | E^{Ba} | G^{Ba} |
|---|---|---|---|
| NHCO(4) | 0 | Atomic bond | (2-Cl, 5-S(=O)Me)Phenyl |
| NHCO(4) | 0 | Atomic bond | (2-S(=O)Me)Phenyl |
| NHCO(4) | 0 | Atomic bond | (3-Cl) pyridin 2-yl |
| NHCO(4) | 0 | Atomic bond | (2-OMe, 3-Cl)Phenyl |
| NHCO(4) | 0 | Atomic bond | (3-Me) pyridin 2-yl |
| NHCO(4) | 0 | Atomic bond | (2-OH, 3-Cl)Phenyl |
| NHCO(4) | 0 | Atomic bond | (3-OH) pyridin 2-yl |
| NHCO(4) | 0 | Atomic bond | (3-Vinyl) pyridin 2-yl |
| NHCO(4) | 0 | Atomic bond | (2-Et) pyridin 2-yl |
| NHSO₂(4) | 0 | Atomic bond | (2-NO₂)Phenyl |
| NHSO₂(4) | 0 | Atomic bond | Phenyl |
| NHSO₂(4) | 0 | Atomic bond | (3-Br)Phenyl |
| NHSO₂(4) | 0 | Atomic bond | (3-OMe)Phenyl |
| NHSO₂(3) | 0 | Atomic bond | (2-NO₂)Phenyl |
| NMeSO₂(3) | 0 | Atomic bond | (2-NO₂)Phenyl |
| NHSO₂(3) | 0 | Atomic bond | naphthalen 2-yl |

**[Table 15]**

| B^{Ba} (substitution position) | n^{B} | E^{Ba} | G^{Ba} |
|---|---|---|---|
| NHSO₂(3) | 0 | Atomic bond | naphthalen 1-yl |
| NHSO₂(4) | 0 | Atomic bond | Cyclohexyl |
| NHSO₂(4) | 0 | Atomic bond | pyridin 3-yl |
| NHSO₂(4) | 0 | Atomic bond | (4-iPr)Phenyl |
| NHSO₂(4) | 1 | Atomic bond | Phenyl |
| NHSO₂(4) | 0 | Atomic bond | thiophen 2-yl |
| NHSO₂(4) | 0 | Atomic bond | naphthalen 2-yl |
| NBnSO₂(4) | 0 | Atomic bond | (2-NO₂)Phenyl |
| NMeSO₂(4) | 0 | Atomic bond | (3-Br)Phenyl |
| NMeSO₂(4) | 0 | Atomic bond | (2-NO₂)Phenyl |
| N(CH₂CH₂OH)SO₂(4) | 0 | Atomic bond | (2-NO₂)Phenyl |
| NHSO₂(4) | 1 | Atomic bond | (2-Cl)Phenyl |
| NHSO₂(4) | 1 | Atomic bond | (3-Br)Phenyl |
| NHSO₂(4) | 0 | Atomic bond | (2-CF₃)Phenyl |
| NHSO₂(4) | 1 | Atomic bond | (2-Br)Phenyl |
| NHSO₂(4) | 1 | Atomic bond | (2-Me) Phenyl |

**[Table 16]**

| B^{Ba} (substitution position) | n^{B} | E^{Ba} | G^{Ba} |
|---|---|---|---|
| NHSO₂(4) | 1 | Atomic bond | (2-NO₂)Phenyl |
| NHSO₂(4) | 2 | Atomic bond | Phenyl |
| NHSO₂(4) | 1 | Atomic bond | (4-Cl)Phenyl |
| NMeSO₂(4) | 1 | Atomic bond | (2-CF₃) Phenyl |
| NMeSO₂(4) | 1 | Atomic bond | (2-Et)Phenyl |
| NMeSO₂(4) | 1 | Atomic bond | (2,3-Me) Phenyl |
| NMeSO₂(4) | 2 | Atomic bond | (2-Cl)Phenyl |
| NMeSO₂(4) | 1 | Atomic bond | (2-NO₂)Phenyl |
| NMeSO₂(4) | 1 | Atomic bond | (2-NH₂)Phenyl |
| NMeSO₂(4) | 1 | Atomic bond | (2-NMe₂)Phenyl |

**[Table 17]**

| B^{Ba} (substitution position) | n^{B} | E^{Ba} | G^{Ba} |
|---|---|---|---|
| NHCO(4) | 0 | Atomic bond | Pyridin 4-yl |
| NHCO(4) | 1 | O | Pyridin 3-yl |
| NHCO(4) | 0 | Atomic bond | Pyridin 3-yl |
| NHCO(4) | 0 | Atomic bond | (2-Me)Pyridin 3-yl |
| NHCO(4) | 0 | Atomic bond | (2-Cl)Pyridin 3-yl |
| NHCO(4) | 1 | O | Pyridin 2-yl |
| NHCO(4) | 0 | Atomic bond | (4-CF₃)Pyridin 3-yl |
| NHCO(4) | 0 | Atomic bond | (2-iPr)Phenyl |

### <Typical Compound Example B-200>

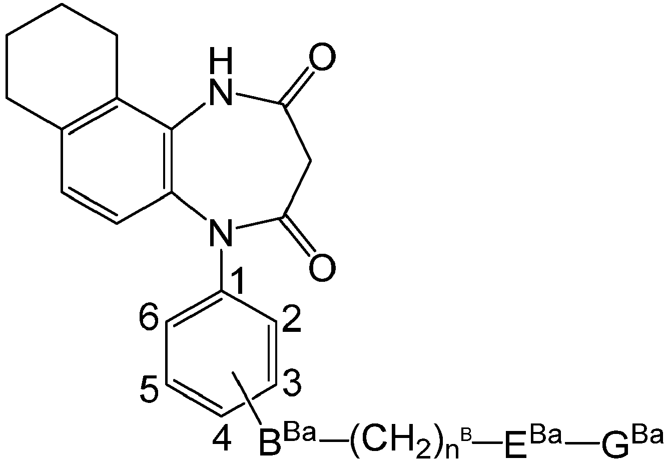

(B^{Ba} (substitution position), n^{B}, E^{Ba}, and G^{Ba} in the formula are indicated in Tables 18 and 19)

**[Table 18]**

| B^{Ba} (substitution position) | n^{B} | E^{Ba} | G^{Ba} |
|---|---|---|---|
| NHCO(4) | 0 | Atomic bond | Cyclohexyl |
| NHCO(4) | 0 | Atomic bond | (6-Me) pyridin-2-yl |
| NHCO(4) | 0 | Atomic bond | (2-Me) pyridin-3-yl |
| NHCO(4) | 0 | Atomic bond | (2-OMe,3-Me)Phenyl |
| NHCO(4) | 0 | Atomic bond | (2,3-Cl)Phenyl |
| NHCO(4) | 0 | Atomic bond | (2-OH,3-Me)Phenyl |
| NHCO(4) | 0 | Atomic bond | (2-I)Phenyl |
| NHCO(4) | 1 | Atomic bond | (1-Me) pyrrol 2-yl |
| NHCO(4) | 1 | Atomic bond | (2-tBu)Phenyl |
| NHCO(4) | 0 | Atomic bond | (2-Isopropenyl) phenyl |
| NHCO(4) | 0 | Atomic bond | (2-iPr)Phenyl |
| NHCO(4) | 1 | Atomic bond | morpholin 2-yl |
| NHCO(4) | 0 | Atomic bond | (2-Cl) pyridin 2-yl |

**[Table 19]**

| B^{Ba}(substitution position) | n^{B} | E^{Ba} | G^{Ba} |
|---|---|---|---|
| NHSO₂(4) | 0 | Atomic bond | (2-NO₂)Phenyl |
| NMeSO₂(4) | 0 | Atomic bond | (2-NO₂)Phenyl |
| SO₂NH(4) | 0 | Atomic bond | Phenyl |
| OSO₂(4) | 0 | Atomic bond | (3-Br) Phenyl |
| NHSO₂(4) | 1 | Atomic bond | (2-Cl) Phenyl |
| NHSO₂(4) | 0 | Atomic bond | (3-Br) Phenyl |
| NHSO₂(4) | 0 | Atomic bond | (3-OMe)Phenyl |
| NHSO₂(4) | 1 | Atomic bond | (2, 3-Cl) Phenyl |
| NHSO₂(4) | 1 | Atomic bond | (2,6-Cl)Phenyl |
| NHSO₂(4) | 1 | Atomic bond | (2-I)Phenyl |
| NMeSO₂(4) | 1 | Atomic bond | (2-Cl)Phenyl |

### <Typical Compound Example B-300>

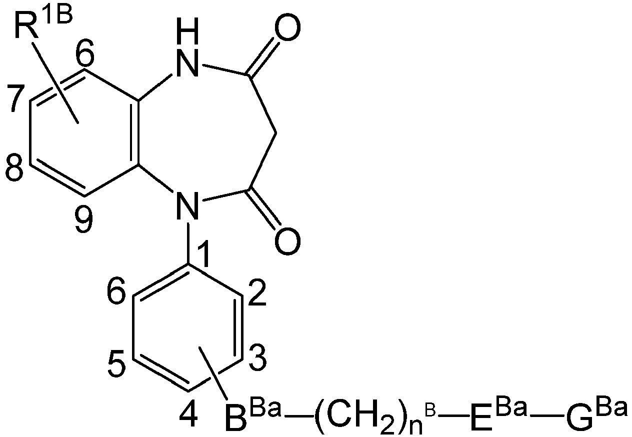

(R^{1B}, B^{Ba} (substitution position), n^{B}, E^{Ba}, and G^{Ba} in the formula are indicated in Table 20)

**[Table 20]**

| R^{1B} | B^{Ba}(substitution position) | n^{B} | E^{Ba} | G^{Ba} |
|---|---|---|---|---|
| 7-OMe | NHCO(4) | 0 | Atomic bond | (2,3-Me)Phenyl |
| 7-OH | NHCO(4) | 0 | Atomic bond | (2,3-Me)Phenyl |
| 6-Me | NHCO(4) | 0 | Atomic bond | (2,3-Me)Phenyl |
| 6,7-Me | NHCO(4) | 0 | Atomic bond | (2-I)Phenyl |
| 6-Et | NHCO(4) | 0 | Atomic bond | (2-I)Phenyl |
| 7-Ph | NHCO(4) | 0 | Atomic bond | (2-Isopropyl))Phenyl |
| 7-(Pyridin-3yl) | NHCO(4) | 0 | Atomic bond | (2-Isopropyl))Phenyl |
| 7-(Pyridin-2yl) | NHCO(4) | 0 | Atomic bond | (2-Isopropyl) Phenyl |
| 7-Cl | NHSO₂(4) | 0 | Atomic bond | (2-Isopropyl)Phenyl |
| 7-Br | NHSO₂(4) | 0 | Atomic bond | (2-Isopropyl)Phenyl |
| 7-CF₃ | NHSO₂(4) | 0 | Atomic bond | (2-Isopropyl)Phenyl |
| H | NHSO₂(4) | 0 | Atomic bond | (2-Isopropyl)Phenyl |
| 6-Me,7-Br | NHSO₂(4) | 0 | Atomic bond | (2-Isopropyl)Phenyl |
| 7-OMe | NHSO₂(4) | 1 | Atomic bond | (2-Cl)Phenyl |
| 7-OH | NHSO₂(4) | 1 | Atomic bond | (2-Cl)Phenyl |
| 6-Me | NHSO₂(4) | 1 | Atomic bond | (2-Cl)Phenyl |

### <Typical Compound Example B-400>

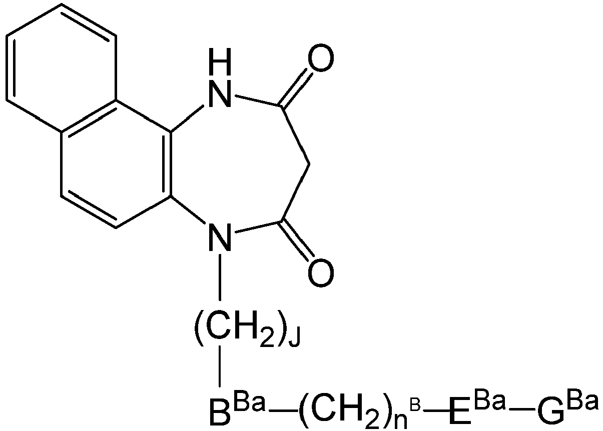

(B^{Ba} (substitution position), n^{B}, E^{Ba}, and G^{Ba} in the formula are indicated in Table 21)

**[Table 21]**

| B^{Ba} (substitution position) | n^{B} | E^{Ba} | G^{Ba} |
|---|---|---|---|
| NHCO | 0 | Atomic bond | (2-Cl,3-OMe)Phenyl |
| NHCO | 0 | Atomic bond | (2-I)Phenyl |
| NHSO₂ | 1 | Atomic bond | (2-Cl)Phenyl |
| NHSO₂ | 1 | Atomic bond | (2-Cl)Phenyl |

### <Typical Compound Example B-500>

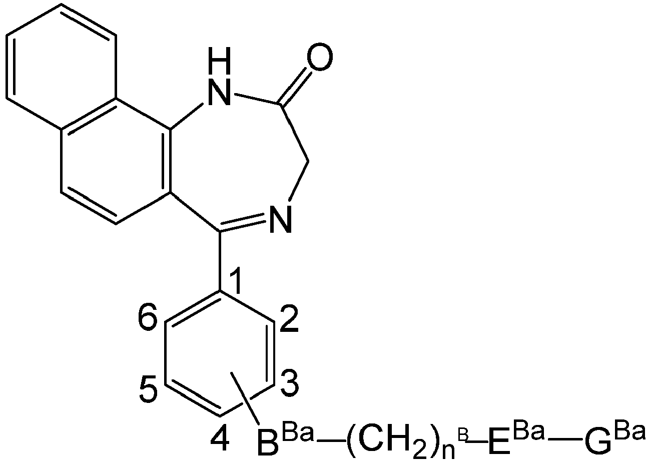

(B^{Ba} (substitution position), n^{B}, E^{Ba}, and G^{Ba} in the formula are indicated in Table 22)

**[Table 22]**

| B^{Ba}(substitution position) | n^{B} | E^{Ba} | G^{Ba} |
|---|---|---|---|
| NHCO(4) | 0 | Atomic | (2-Cl,3-OMe)Phenyl |
| | | bond | |
| NHCO(4) | 0 | Atomic bond | (2-Cl,3-OH)Phenyl |
| NHCO(4) | 0 | Atomic bond | (2-tBu)Phenyl |
| NHCO(4) | 0 | Atomic bond | (2-Cl, 6-OMe)Phenyl |
| NHCO(4) | 0 | Atomic bond | (2-Cl,6-OH)Phenyl |
| NHSO2(3) | 0 | Atomic bond | Phenyl |
| NHSO2(4) | 0 | Atomic bond | (2-Cl)Phenyl |

### <Typical Compound Example B-600>

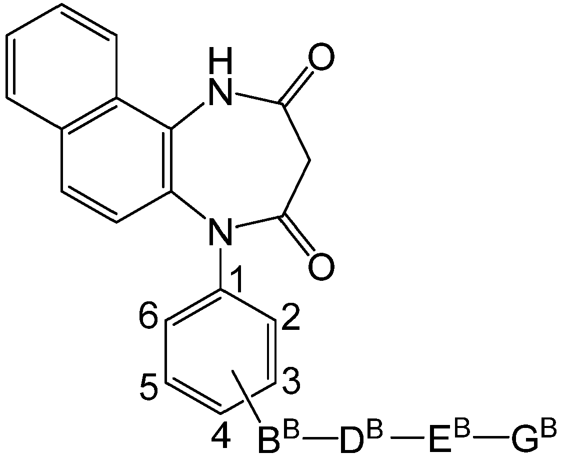

(B^{B} (substitution position), D^{B}, E^{B}, and G^{B} in the formula are indicated in Table 23)

**[Table 23]**

| B^{B}(substitution position) | D^{B} | E^{B} | G^{B} |
|---|---|---|---|
| NHCO(4) | C(Me)H | Atomic bond | Phenyl |
| NHCO(4) | C(Me)₂ | Atomic bond | Phenyl |
| NHCO(4) | CH=CH | Atomic bond | Phenyl |
| NHCO(4) | C(Me)H | O | Phenyl |
| NHCO(4) | C(Me)₂ | O | Phenyl |
| NHCO(4) | CH=CH | Atomic bond | (2-Me)Phenyl |
| NHCO(4) | CH=CH | Atomic bond | (2-Cl)Phenyl |

### <Typical Compound Example B-700>

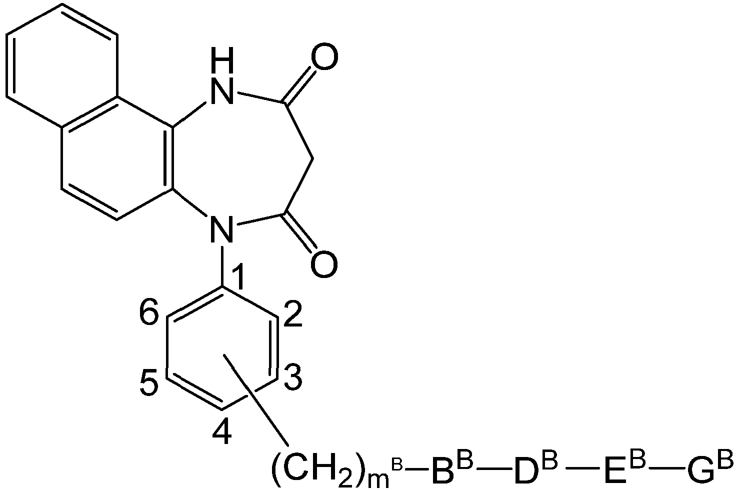

(m^{B} (substitution position), B^{B}, D^{B}, E^{B}, and G^{B} in the formula are indicated in Table 24)

**[Table 24]**

| m^{B}(substitution position) | B^{B} | D^{B} | E^{B} | G^{B} |
|---|---|---|---|---|
| 1(4) | NHCO | Atomic bond | Atomic bond | Phenyl |
| 1(4) | NHCO | Atomic bond | Atomic bond | (2-Cl) Phenyl |
| 1(4) | NHSO₂ | CH₂ | Atomic bond | (2-Cl) Phenyl |

### <Typical Compound Example B-800>

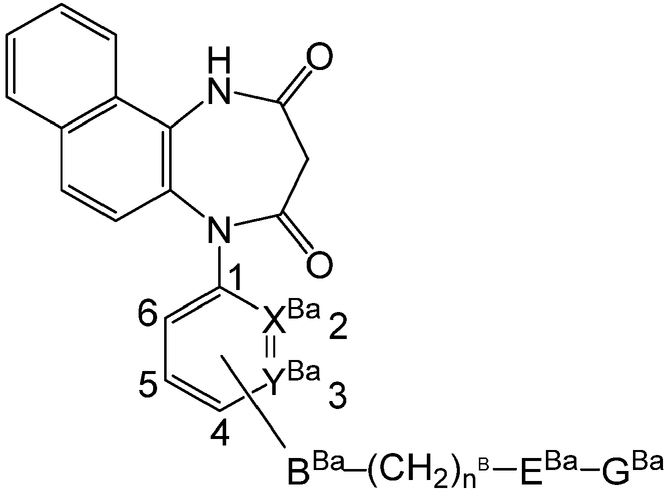

(X^{Ba}, Y^{Ba}, B^{Ba} (substitution position), n^{B}, E^{Ba}, and G^{Ba} in the formula are indicated in Table 25)

**[Table 25]**

| X^{Ba} | Y^{Ba} | B^{Ba}(substitution position) | n^{B} | E^{Ba} | G^{Ba} |
|---|---|---|---|---|---|
| CH | C-F | NHCO(4) | 0 | Atomic bond | (2,3-Me) Phenyl |
| CH | C-OH | NHCO(4) | 0 | Atomic bond | (2,3-Me) Phenyl |
| CH | C-F | NHCO(4) | 0 | Atomic bond | (2-I)Phenyl |
| CH | N | NHCO(4) | 0 | Atomic bond | (2-I)Phenyl |
| CH | N | NHCO(4) | 0 | Atomic bond | Phenyl |
| N | CH | NHCO(4) | 0 | Atomic bond | (2-I)Phenyl |
| CH | N | NHCO(4) | 0 | Atomic bond | (2-Cl) Phenyl |
| CH | N | NHCO(4) | 0 | Atomic bond | (2-OH) Phenyl |
| CH | N | NHC(=O)NH(4) | 0 | Atomic bond | (2-OH) Phenyl |
| CH | N | NHCO(4) | 0 | Atomic bond | (2-OH, 6-Me)Phenyl |
| CH | N | NHCO(4) | 0 | Atomic bond | (2-OH, 6-Cl) Phenyl |
| CH | N | NHCO(3) | 0 | Atomic bond | (2-OH, 6-Cl) Phenyl |
| CH | N | NHCO(4) | 0 | Atomic bond | (2-Cl) pyridin 2-yl |
| CH | N | NHCO(4) | 1 | Atomic bond | (2-Cl) pyridin 2-yl |
| CH | N | NHCO(4) | 0 | Atomic bond | (2-Me) pyridin 2-yl |
| CH | C-OMe | NHSO₂(4) | 1 | Atomic bond | (2-Cl)Phenyl |
| CH | C-OH | NHSO₂(4) | 1 | Atomic bond | (2-Cl)Phenyl |

### <Typical Compound Example B-900>

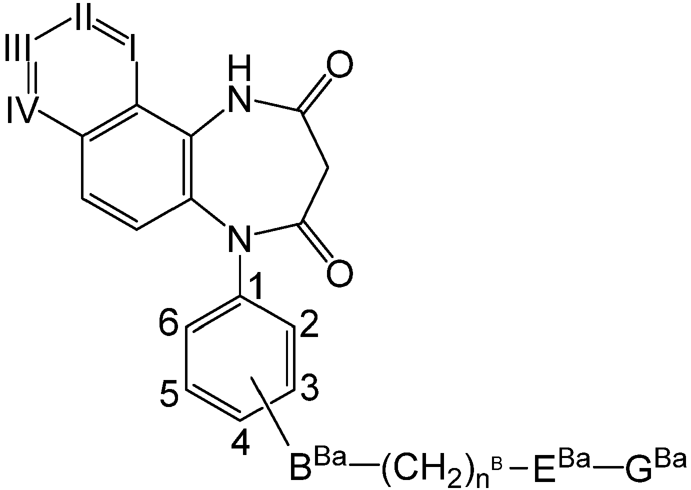

(I=II-III=IV, B^{Ba} (substitution position), n^{B}, E^{Ba}, and G^{Ba} in the formula are indicated in Table 26)

**[Table 26]**

| I=II-III=IV | B^{Ba}(substitution position) | nB | E^{Ba} | G^{Ba} |
|---|---|---|---|---|
| N=CH-CH=CH | NHCO(4) | 0 | Atomic bond | (2-I)Phenyl |
| CH=N-CH=CH | NHCO(4) | 0 | Atomic bond | (2-I)Phenyl |
| CH=CH-N=CH | NHCO(4) | 0 | Atomic bond | (2-I)Phenyl |
| CH=CH-CH=N | NHCO(4) | 0 | Atomic bond | (2-I)Phenyl |
| N=CH-CH=CH | NHCO(4) | 1 | O | Phenyl |
| N=CH-CH=CH | NHCO(3) | 0 | Atomic bond | (2-I)Phenyl |
| N=CH-CH=CH | NHCO(4) | 0 | Atomic bond | (2-Cl) Phenyl |
| N=CH-CH=CH | NHCO(4) | 0 | Atomic bond | (2-OH) Phenyl |
| N=CH-CH=CH | NHC(=O)NH(4) | 0 | Atomic bond | (2-OH) Phenyl |
| N=CH-CH=CH | NHCO(4) | 1 | O | (2-OH, 6-Me)Phenyl |
| N=CH-CH=CH | NHCO(4) | 0 | Atomic bond | (2-OH, 6-Cl)Phenyl |
| N=CH-CH=CH | NHCO(3) | 0 | Atomic bond | (2-OH, 6-Cl)Phenyl |
| N=CH-CH=CH | NHCO(4) | 0 | Atomic bond | (2-Cl) pyridin 2-yl |
| N=CH-CH=CH | NHCO(4) | 1 | Atomic bond | (2-Cl) pyridin 2-yl |
| N=CH-CH=CH | NHCO(4) | 0 | Atomic bond | (2-Me) pyridin 2-yl |
| CH=CH-N=CH | NHCO(4) | 0 | Atomic bond | (2-Cl) pyridin 3-yl |

### <Typical Compound Example B-1000>

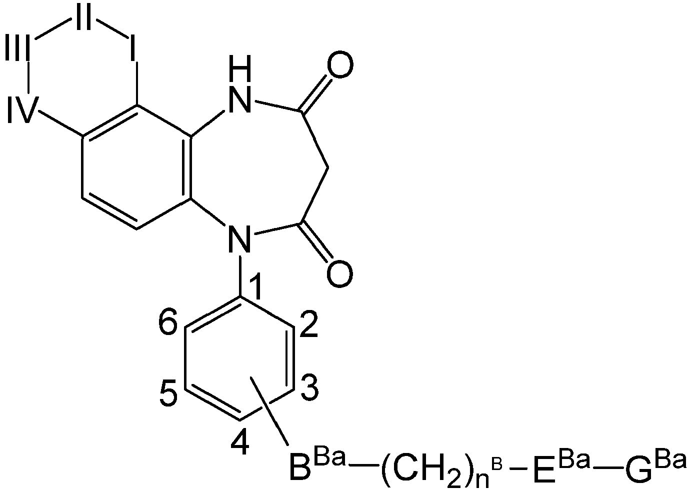

(I-II-III-IV, B^{Ba} (substitution position), n^{B}, E^{Ba}, and G^{Ba} in the formula are indicated in Table 27)

**[Table 27]**

| I-II-III-IV | B^{Ba}(substitution position) | nB | E^{Ba} | G^{Ba} |
|---|---|---|---|---|
| NH-CH₂-CH₂-CH₂ | NHCO(4) | 0 | Atomic bond | (2-I)Phenyl |
| CH₂-NH-CH₂-CH₂ | NHCO(4) | 0 | Atomic bond | (2-I)Phenyl |
| CH₂-CH₂-NH-CH₂ | NHCO(4) | 0 | Atomic bond | (2-I)Phenyl |
| CH₂-CH₂-CH₂-NH | NHCO(4) | 0 | Atomic bond | (2-I)Phenyl |
| CH₂-CH₂-NH-CH₂ | NHCO(4) | 1 | O | Phenyl |
| CH₂-CH₂-NH-CH₂ | NHCO(3) | 0 | Atomic bond | (2-I)Phenyl |
| CH₂-CH₂-NH-CH₂ | NHCO(4) | 0 | Atomic bond | (2-Cl) Phenyl |
| CH₂-CH₂-NH-CH₂ | NHCO(4) | 0 | Atomic bond | (2-Cl) pyridin 3-yl |
| CH₂-CH₂-NH-CH₂ | NHCO(4) | 0 | Atomic bond | (2-OH) Phenyl |
| CH₂-CH₂-NH-CH₂ | NHC(=O)NH(4) | 0 | Atomic bond | (2-OH) Phenyl |
| CH₂-CH₂-NH-CH₂ | NHCO(4) | 1 | O | (2-OH, 6-Me) Phenyl |
| CH₂-CH₂-NH-CH₂ | NHCO(4) | 0 | Atomic bond | (2-OH, 6-Cl) Phenyl |
| CH₂-CH₂-NH-CH₂ | NHCO(3) | 0 | Atomic bond | (2-OH, 6-Cl) Phenyl |
| CH₂-CH₂-NH-CH₂ | NHCO(4) | 0 | Atomic bond | (2-Cl) pyridin 2-yl |
| CH₂-CH₂-NH-CH₂ | NHCO(4) | 1 | Atomic bond | (2-Cl) pyridin 2-yl |
| CH₂-CH₂-NH-CH₂ | NHCO(4) | 0 | Atomic bond | (2-Me) pyridin 2-yl |
| CH₂-CH₂-NH-CH₂ | NHCO(4) | 0 | Atomic bond | (2-Cl) pyridin 3-yl |

### <Typical Compound Example B-1100>

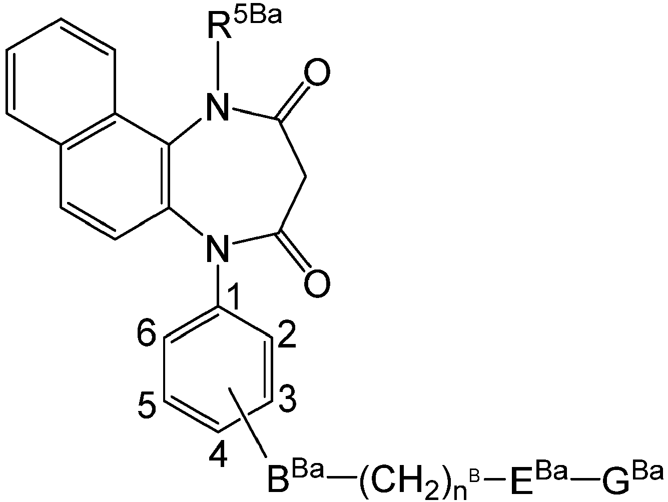

(R^{5Ba}, B^{Ba} (substitution position), n^{B}, E^{Ba}, and G^{Ba} in the formula are indicated in Table 28)

**[Table 28]**

| R^{5Ba} | B^{Ba} (substitution position) | nB | E^{Ba} | G^{Ba} |
|---|---|---|---|---|
| Bn | NBnSO₂(4) | 0 | Atomic bond | (2-NO₂)Phenyl |
| Me | NBnSO₂(4) | 0 | Atomic bond | (2-NO₂)Phenyl |
| Et | NBnSO₂(4) | 0 | Atomic bond | (2-NO₂)Phenyl |

Since the compounds represented by the general formula (A) are disclosed in WO 2010/093061 A, all of the compounds can be easily obtained by referring to this international publication. The disclosure of this international publication is incorporated herein by reference in its entirety.

Since the compounds represented by the general formulas (BI) and (BII) are disclosed in WO 2013/105608 A, all of the compounds can be easily obtained by referring to this international publication. The disclosure of this international publication is incorporated herein by reference in its entirety.

Furthermore, WO 2010/093061 A and WO 2013/105608 A disclose that the compounds represented by the general formulas (A) to (BII) have a P2X4 receptor antagonistic action.

Specific examples of a preferable compound comprised in the compounds represented by the general formulas (A) to (BII) or a pharmaceutically acceptable salt thereof are shown below, however, the compound or a pharmaceutically acceptable salt thereof that can be used as the active ingredient of the pharmaceutical composition of the present invention is not limited thereto.
(Compound A1) 5-[3-(1H-tetrazol-5-yl)phenyl]-1H-naphtho[1,2-b][1,4]diazepine-2,4(3H,5H)-dione;
(Compound A2) 5-[3-(1H-tetrazol-5-yl)phenyl]-1H-naphtho[1,2-b][1,4]diazepine-2,4(3H,5H)-dione sodium salt;
(Compound A3) 5-[3-(1H-tetrazol-5-yl)phenyl]-1H-naphtho[1,2-b][1,4]diazepine-2,4(3H,5H)-dione potassium salt;
(Compound A4) 5-[4-(1H-tetrazol-5-yl)phenyl]-1H-naphtho[1,2-b][1,4]diazepine-2,4(3H,5H)-dione;
(Compound A5) 5-[4-(1H-tetrazol-5-yl)phenyl]-1H-naphtho[1,2-b][1,4]diazepine-2,4(3H,5H)-dione sodium salt;
(Compound A6) 1-methyl-5-[3-(1H-tetrazol-5-yl)phenyl]-1H-naphtho[1,2-b][1,4]diazepine-2,4(3H,5H)-dione;
(Compound A7) 1,3-dimethyl-5-[3-(1H-tetrazol-5-yl)phenyl]-1H-naphtho[1,2-b][1,4]diazepine-2,4(3H,5H)-dione;
(Compound A8) 5-[2-chloro-5-(1H-tetrazol-5-yl)phenyl]-1H-naphtho[1,2-b][1,4]diazepine-2,4(3H,5H)-dione;
(Compound A9) 5-[2-chloro-5-(1H-tetrazol-5-yl)phenyl]-1H-naphtho[1,2-b][1,4]diazepine-2,4(3H,5H)-dione sodium salt;
(Compound A10) 5-[2-methyl-5-(1H-tetrazol-5-yl)phenyl]-1H-naphtho[1,2-b][1,4]diazepine-2,4(3H,5H)-dione;

(Compound A11) 5-[2-methyl-5-(1H-tetrazol-5-yl)phenyl]-1H-naphtho[1,2-b][1,4]diazepine-2,4(3H,5H)-dione sodium salt;
(Compound A12) 5-[2-bromo-5-(1H-tetrazol-5-yl)phenyl]-1H-naphtho[1,2-b][1,4]diazepine-2,4(3H,5H)-dione;
(Compound A13) 5-[3-(2-methyl-2H-tetrazol-5-yl)phenyl]-1H-naphtho[1,2-b][1,4]diazepine-2,4(3H,5H)-dione;
(Compound A14) 5-[3-(1-methyl-1H-tetrazol-5-yl)phenyl]-1H-naphtho[1,2-b][1,4]diazepine-2,4(3H,5H)-dione;
(Compound A15) 5-[3-(5-oxo-4H-[1,2,4]oxadiazol-3-yl)phenyl]-1H-naphtho[1,2-b][1,4]diazepine-2,4(3H,5H)-dione;
(Compound A16) 5-[3-(5-thioxo-4H-[1,2,4]oxadiazol-3-yl)phenyl]-1H-naphtho[1,2-b][1,4]diazepine-2,4(3H,5H)-dione;
(Compound A17) 5-[3-(5-thioxo-4H-[1,2,4]oxadiazol-3-yl)phenyl]-1H-naphtho[1,2-b][1,4]diazepine-2,4(3H,5H)-dione sodium salt;
(Compound A18) 5-[3-(oxazol-2-yl)phenyl]-1H-naphtho[1,2-b][1,4]diazepine-2,4(3H,5H)-dione;
(Compound A19) 5-[3-(1H-pyrazol-4-yl)phenyl]-1H-naphtho[1,2-b][1,4]diazepine-2,4(3H,5H)-dione;
(Compound A20) 5-[4-fluoro-3-(1H-tetrazol-5-yl)phenyl]-1H-naphtho[1,2-b][1,4]diazepine-2,4(3H,5H)-dione;
(Compound A21) 5-[4-fluoro-3-(1H-tetrazol-5-yl)phenyl]-1H-naphtho[1,2-b][1,4]diazepine-2,4(3H,5H)-dione sodium salt;

(Compound B1) 5-(4-benzoylaminophenyl)-1H-naphtho[1,2-b][1,4]diazepine-2,4(3H,5H)-dione;
(Compound B2) 5-[4-[2-(trifluoromethyl)benzoyl]aminophenyl]-1H-naphtho[1,2-b][1,4]diazepine-2,4(3H,5H)-dione;
(Compound B3) 5-[4-(3-bromobenzoyl)aminophenyl]-1H-naphtho[1,2-b][1,4]diazepine-2,4(3H,5H)-dione;
(Compound B4) 5-[4-[4-(trifluoromethyl)benzoyl]aminophenyl]-1H-naphtho[1,2-b][1,4]diazepine-2,4(3H,5H)-dione;
(Compound B5) 5-[4-(2-methylbenzoyl)aminophenyl]-1H-naphtho[1,2-b][1,4]diazepine-2,4(3H,5H)-dione;
(Compound B6) 5-[4-(2,6-dimethylbenzoylamino)phenyl]-1H-naphtho[1,2-b][1,4]diazepine-2,4(3H,5H)-dione;
(Compound B7) 5-[4-(2,6-dichlorobenzoyl)aminophenyl]-1H-naphtho[1,2-b][1,4]diazepine-2,4(3H,5H)-dione;
(Compound B8) 5-[4-(3-chlorobenzoyl)aminophenyl]-1H-naphtho[1,2-b] [1,4]diazepine-2,4(3H,5H)-dione;
(Compound B9) 5[4-(2-phenylacetylamino)phenyl]-1H-naphtho[1,2-b] [1,4]diazepine-2,4(3H,5H)-dione;
(Compound B10) 1-[4-(2,4-dioxo-1,2,3,4-tetrahydronaphtho[1,2-b] [1,4]diazepin-5-yl)phenyl]-3-phenylthiourea;

(Compound B11) 5-[4-(2,3-dimethoxybenzoylamino)phenyl]-1H-naphtho[1,2-b][1,4]diazepine-2,4(3H,5H)-dione;
(Compound B12) 5-[4-(2-methoxybenzoylamino)phenyl]-1H-naphtho[1,2-b] [1,4]diazepine-2,4(3H,5H)-dione;
(Compound B13) 5-[4-[(2-chlorophenylacetyl)amino]phenyl]-1H-naphtho[1,2-b][1,4]diazepine-2,4(3H,5H)-dione;
(Compound B14) 5-[4-(2,3-dimethylbenzoylamino)phenyl]-1H-naphtho[1,2-b][1,4]diazepine-2,4(3H,5H)-dione;
(Compound B15) 5-[4-(2,5-dimethylbenzoylamino)phenyl]-1H-naphtho[1,2-b][1,4]diazepine-2,4(3H,5H)-dione;
(Compound B16) 5-[4-(5-bromo-2-chlorobenzoylamino)phenyl]-1H-naphtho[1,2-b] [1,4]diazepine-2,4(3H,5H)-dione;
(Compound B17) 5-[4-(2,4-dichlorobenzoylamino)phenyl]-1H-naphtho[1,2-b][1,4]diazepine-2,4(3H,5H)-dione;
(Compound B18) 5-[4-(2-hydroxybenzoylamino)phenyl]-1H-naphtho[1,2-b][1,4]diazepine-2,4(3H,5H)-dione;
(Compound B19) 5-[4-(2,3-dihydroxybenzoylamino)phenyl]-1H-naphtho[1,2-b][1,4]diazepine-2,4(3H,5H)-dione;
(Compound B20) 1-[4-(2,4-dioxo-1,2,3,4-tetrahydronaphtho[1,2-b] [1,4]diazepin-5-yl)phenyl]-3-phenylurea;

(Compound B21) 5-[4-[(2,6-dichlorophenylacetyl)amino]phenyl]-1H-naphtho[1,2-b][1,4]diazepine-2,4(3H,5H)-dione;
(Compound B22) 5-[4-[(2-methoxyphenylacetyl)amino]phenyl]-1H-naphtho[1,2-b][1,4]diazepine-2,4(3H,5H)-dione;
(Compound B23) 5-[4-[(2-hydroxyphenylacetyl)amino]phenyl]-1H-naphtho[1,2-b][1,4]diazepine-2,4(3H,5H)-dione;
(Compound B24) 1-(2-chlorophenyl)-3-[4-(2,4-dioxo-1,2,3,4-tetrahydronaphtho[1,2-b][1,4]diazepin-5-yl)phenyl]thiourea;
(Compound B25) 5-[4-[3-(trifluoromethyl)benzoylamino]phenyl]-1H-naphtho[1,2-b][1,4]diazepine-2,4(3H,5H)-dione;
(Compound B26) 5-[4-[2-[2-(trifluoromethyl)phenyl]acetylamino]phenyl]-1H-naphtho[1,2-b][1,4]diazepine-2,4(3H,5H)-dione;
(Compound B27) 1-(2-chlorophenyl)-3-[4-(2,4-dioxo-1,2,3,4-tetrahydronaphtho[1,2-b][1,4]diazepin-5-yl)phenyl]urea;
(Compound B28) 5-[4-[(2-phenylpropionyl)amino]phenyl]-1H-naphtho[1,2-b][1,4]diazepine-2,4(3H,5H)-dione;
(Compound B29) 5-[4-(2-chloro-3-methoxybenzoylamino)phenyl]-1H-naphtho[1,2-b][1,4]diazepine-2,4(3H,5H)-dione;
(Compound B30) 5-[4-(3-phenylpropionylamino)phenyl]-1H-naphtho[1,2-b][1,4]diazepine-2,4(3H,5H)-dione;

(Compound B31) 5-[4-[(1H-indole-3-carbonyl)amino]phenyl]-1H-naphtho[1,2-b][1,4]diazepine-2,4(3H,5H)-dione;
(Compound B32) 5-[4-(2-chloro-3-hydroxybenzoylamino)phenyl]-1H-naphtho[1,2-b][1,4]diazepine-2,4(3H,5H)-dione;
(Compound B33) 5-[4-[(2-methyl-2-phenylpropionyl)amino]phenyl]-1H-naphtho[1,2-b][1,4]diazepine-2,4(3H,5H)-dione;
(Compound B34) 5-[4-(2-phenoxyacetylamino)phenyl]-1H-naphtho[1,2-b][1,4]diazepine-2,4(3H,5H)-dione;
(Compound B35) 5-[4-[2-(2-chloro-4-methoxyphenyl)acetylamino]phenyl]-1H-naphtho[1,2-b][1,4]diazepine-2,4(3H,5H)-dione;
(Compound B36) 5-[4-[(1-methyl-1H-imidazole-2-carbonyl)amino]phenyl]-1H-naphtho[1,2-b][1,4]diazepine-2,4(3H,5H)-dione;
(Compound B37) 5-[4-[2-(2,4-dichlorophenyl)acetylamino]phenyl]-1H-naphtho[1,2-b][1,4]diazepine-2,4(3H,5H)-dione;
(Compound B38) 5-[4-[2-(2-chloro-4-hydroxyphenyl)acetylamino]phenyl]-1H-naphtho[1,2-b][1,4]diazepine-2,4(3H,5H)-dione;
(Compound B39) 5-[4-(3-phenylpropenylamino)phenyl]-1H-naphtho[1,2-b][1,4]diazepine-2,4(3H,5H)-dione;
(Compound B40) 5-[4-[(3-pyridylacetyl)amino]phenyl]-1H-naphtho[1,2-b][1,4]diazepine-2,4(3H,5H)-dione hydrochloride;

(Compound B41) 5-[4-(1H-benzimidazole-2-carbonylamino)phenyl]-1H-naphtho[1,2-b][1,4]diazepine-2,4(3H,5H)-dione;
(Compound B42) 1-[4-(2,3-dimethylbenzoylamino)phenyl]-7-methoxy-1H-1,5-benzodiazepine-2,4(3H,5H)-dione;
(Compound B43) 5-[4-[(benzoylamino)methyl]phenyl]-1H-naphtho[1,2-b][1,4]diazepine-2,4(3H,5H)-dione;
(Compound B44) 5-[4-[(2-chlorobenzoylamino)methyl]phenyl]-1H-naphtho[1,2-b][1,4]diazepine-2,4(3H,5H)-dione;
(Compound B45) 1-[4-(2,3-dimethylbenzoylamino)phenyl]-7-hydroxy-1H-1,5-benzodiazepine-2,4(3H,5H)-dione;
(Compound B46) 5-[4-(2-chlorobenzoylamino)phenyl]-1H-naphtho[1,2-b][1,4]diazepine-2,4(3H,5H)-dione;
(Compound B47) 5-[4-(2-bromobenzoylamino)phenyl]-1H-naphtho[1,2-b][1,4]diazepine-2,4(3H,5H)-dione;
(Compound B48) 5-[4-(2-iodobenzoylamino)phenyl]-1H-naphtho[1,2-b][1,4]diazepine-2,4(3H,5H)-dione;
(Compound B49) 5-[4-(2,3-dimethylbenzoylamino)-3-fluorophenyl]-1H-naphtho[1,2-b][1,4]diazepine-2,4(3H,5H)-dione;
(Compound B50) 5-[4-[2-(2-methylphenyl)acetylamino]phenyl]-1H-naphtho[1,2-b][1,4]diazepine-2,4(3H,5H)-dione;

(Compound B51) 5-[4-[(quinoxalin-2-yl)carbonylamino]phenyl]-1H-naphtho[1,2-b] [1,4]diazepine-2,4(3H,5H)-dione;
(Compound B52) 5-[4-[(5-methylthiophen-2-yl)carbonylamino]phenyl]-1H-naphtho[1,2-b] [1,4]diazepine-2,4(3H,5H)-dione;
(Compound B53) 5-[3-[(2-chlorophenylacetyl)amino]phenyl]-1H-naphtho[1,2-b] [1,4]diazepine-2,4(3H,5H)-dione;
(Compound B54) 5-[4-[(2,4,6-trimethylbenzoyl)amino]phenyl]-1H-naphtho[1,2-b] [1,4]diazepine-2,4(3H,5H)-dione;
(Compound B55) 5-[4-(cyclohexylcarbonylamino)phenyl]-1,2,3,4,8,9,10,11-octahydronaphtho[1,2-b][1,4]diazepine-2,4(3H,5H)-dione;
(Compound B56) 1-[4-(2,3-dimethylbenzoyl)aminophenyl]-6-methyl-1H-1,5-benzodiazepine-2,4(3H,5H)-dione;
(Compound B57) 5-[4-[(2-ethylbenzoyl)amino]phenyl]-1H-naphtho[1,2-b][1,4]diazepine-2,4(3H,5H)-dione;
(Compound B58) 5-[4-[(6-methylpyridin-2-yl)carbonylamino]phenyl]-1,2,3,4,8,9,10,11-octahydronaphtho[1,2-b][1,4]diazepine-2,4(3H,5H)-dione;
(Compound B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
(Compound B60) 1-[4-(2,4-dioxo-1,2,3,4-tetrahydronaphtho[1,2-b][1,4]diazepin-5-yl)phenyl]-3-(2-methylphenyl)thiourea;

(Compound B61) 5-[4-(2-methoxy-3-methylbenzoyl)aminophenyl]-1,2,3,4,8,9,10,11-octahydronaphtho[1,2-b][1,4]diazepine-2,4(3H,5H)-dione;
(Compound B62) 5-[4-(2,3-dichlorobenzoyl)aminophenyl]-1,2,3,4,8,9,10,11-octahydronaphtho[1,2-b][1,4]diazepine-2,4(3H,5H)-dione;
(Compound B63) 5-[4-(2,3-dimethylbenzoylamino)-3-hydroxyphenyl]-1H-naphtho[1,2-b][1,4]diazepine-2,4(3H,5H)-dione;
(Compound B64) 5-[4-(2-chloro-3-methoxybenzoylamino)phenyl]-1,3-dihydronaphtho[1,2-e]-1,4-diazepin-2-one;
(Compound B65) 5-[4-[(4-dimethylaminobenzoyl)amino]phenyl]-1H-naphtho[1,2-b][1,4]diazepine-2,4(3H,5H)-dione;
(Compound B66) 5-[4-[2-(2,4-dichlorophenoxy)acetylamino]phenyl]-1H-naphtho[1,2-b][1,4]diazepine-2,4(3H,5H)-dione;
(Compound B67) 5-[4-[2-(2-methylphenoxy)acetylamino]phenyl]-1H-naphtho[1,2-b][1,4]diazepine-2,4(3H,5H)-dione;
(Compound B68) N-[4-(2,4-dioxo-1,2,3,4-tetrahydronaphtho[1,2-b][1,4]diazepin-5-yl)butyl]-2-chloro-3-methoxybenzamide;
(Compound B69) 5-[4-(2-chloro-3-hydroxybenzoylamino)phenyl]-1,3-dihydronaphtho[1,2-e]-1,4-diazepin-2-one;
(Compound B70) 5-[4-(2-acetylbenzoylamino)phenyl]-1H-naphtho[1,2-b][1,4]diazepine-2,4(3H,5H)-dione;

(Compound B71) 5-[4-(2-tert-butylbenzoylamino)phenyl]-1H-naphtho[1,2-b][1,4]diazepine-2,4(3H,5H)-dione;
(Compound B72) 5-[2-(2-iodobenzoyl)aminoethyl]-1H-naphtho[1,2-b][1,4]diazepine-2,4(3H,5H)-dione;
(Compound B73) 5-[3-[(2-iodobenzoyl)amino]phenyl]-1H-naphtho[1,2-b][1,4]diazepine-2,4(3H,5H)-dione;
(Compound B74) 6,7-dimethyl-1-[4-(2-iodobenzoyl)aminophenyl]-1H-1,5-benzodiazepine-2,4(3H,5H)-dione;
(Compound B75) 5-[4-[(1-methylpiperidin-4-yl)carbonylamino]phenyl]-1H-naphtho[1,2-b][1,4]diazepine-2,4(3H,5H)-dione hydrochloride;
(Compound B76) 5[4-[(benzofuran-2-yl)carbonylamino]phenyl]-1H-naphtho[1,2-b][1,4]diazepine-2,4(3H,5H)-dione;
(Compound B77) 5-[4-[(1-methyl-1H-indol-3-yl)carbonylamino]phenyl]-1H-naphtho[1,2-b][1,4]diazepine-2,4(3H,5H)-dione;
(Compound B78) 5-[4-(2-propenylbenzoylamino)phenyl]-1H-naphtho[1,2-b][1,4]diazepine-2,4(3H,5H)-dione;
(Compound B79) 5-[4-(2-propylbenzoylamino)phenyl]-1H-naphtho[1,2-b][1,4]diazepine-2,4(3H,5H)-dione;
(Compound B80) 5-[3-fluoro-4-(2-iodobenzoylamino)phenyl]-1H-naphtho[1,2-b][1,4]diazepine-2,4(3H,5H)-dione;

(Compound B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
(Compound B82) 5-[4-[(2-isopropoxybenzoyl)amino]phenyl]-1H-naphtho[1,2-b][1,4]diazepine-2,4(3H,5H)-dione;
(Compound B83) 5-[4-[(3-methylthiophen-2-yl)carbonylamino]phenyl]-1H-naphtho[1,2-b][1,4]diazepine-2,4(3H,5H)-dione;
(Compound B84) 5-[4-(2-phenoxypropionylamino)phenyl]-1H-naphtho[1,2-b][1,4]diazepine-2,4(3H,5H)-dione;
(Compound B85) 5-[4-[2-(4-chloro-2-methylphenoxy)acetylamino]phenyl]-1H-naphtho[1,2-b][1,4]diazepine-2,4(3H,5H)-dione;
(Compound B86) 5-[4-[(4-fluoro-2-trifluoromethyl)benzoyl]aminophenyl]-1H-naphtho[1,2-b][1,4]diazepine-2,4(3H,5H)-dione;
(Compound B87) 5-[4-(4-fluoro-2-methoxybenzoyl)aminophenyl]-1H-naphtho[1,2-b][1,4]diazepine-2,4(3H,5H)-dione;
(Compound B88) 5-[4-(4-fluoro-2-hydroxybenzoyl)aminophenyl]-1H-naphtho[1,2-b][1,4]diazepine-2,4(3H,5H)-dione;
(Compound B89) 5-[3-[(2-iodophenylacetyl)amino]phenyl]-1H-naphtho[1,2-b][1,4]diazepine-2,4(3H,5H)-dione;
(Compound B90) 5-[4-(2-methyl-2-phenoxypropionylamino)phenyl]-1H-naphtho[1,2-b][1,4]diazepine-2,4(3H,5H)-dione;

(Compound B91) 5-[4-(2-tert-butylbenzoylamino)phenyl]-1,3-dihydronaphtho[1,2-e]-1,4-diazepin-2-one;
(Compound B92) 5-[4-[(3-dimethylaminobenzoyl)amino]phenyl]-1H-naphtho[1,2-b][1,4]diazepine-2,4(3H,5H)-dione;
(Compound B93) 5-[4-(4-iodo-2-methoxybenzoylamino)phenyl]-1H-naphtho[1,2-b][1,4]diazepine-2,4(3H,5H)-dione;
(Compound B94) 5-[4-(6-fluoro-2-methoxybenzoylamino)phenyl]-1H-naphtho[1,2-b][1,4]diazepine-2,4(3H,5H)-dione;
(Compound B95) 5-[4-(2-hydroxy-4-iodobenzoylamino)phenyl]-1H-naphtho[1,2-b][1,4]diazepine-2,4(3H,5H)-dione;
(Compound B96) 5-[4-(6-fluoro-2-hydroxybenzoylamino)phenyl]-1H-naphtho[1,2-b][1,4]diazepine-2,4(3H,5H)-dione;
(Compound B97) 5-[4-(2-fluorobenzoyl)aminophenyl]-1H-naphtho[1,2-b][1,4]diazepine-2,4(3H,5H)-dione;
(Compound B98) 5-[4-[(2-dimethylaminobenzoyl)amino]phenyl]-1H-naphtho[1,2-b][1,4]diazepine-2,4(3H,5H)-dione;
(Compound B99) 5-[4-(2-methoxy-6-methylbenzoylamino)phenyl]-1H-naphtho[1,2-b][1,4]diazepine-2,4(3H,5H)-dione;
(Compound B100) 5-[4-(2-hydroxy-6-methylbenzoylamino)phenyl]-1H-naphtho[1,2-b][1,4]diazepine-2,4(3H,5H)-dione;

(Compound B101) 5-[4-[3-(2-methylphenyl)propionylamino]phenyl]-1H-naphtho[1,2-b][1,4]diazepine-2,4(3H,5H)-dione;
(Compound B102) 5-(4-phenylcarbamoylphenyl)-1H-naphtho[1,2-b][1,4]diazepine-2,4(3H,5H)-dione;
(Compound B103) 5-(4-benzylcarbamoylphenyl)-1H-naphtho[1,2-b][1,4]diazepine-2,4(3H,5H)-dione;
(Compound B104) 5-[4-[3-(2-methylphenyl)propenoylamino]phenyl]-1H-naphtho[1,2-b][1,4]diazepine-2,4(3H,5H)-dione;
(Compound B105) 5-[4-[3-(2-chlorophenyl)propionylamino]phenyl]-1H-naphtho[1,2-b][1,4]diazepine-2,4(3H,5H)-dione;
(Compound B106) 5-[4-(2-iodobenzoyl)aminophenyl]-1,2,3,4,8,9,10,11-octahydronaphtho[1,2-b][1,4]diazepine-2,4(3H,5H)-dione;
(Compound B107) 5-[4-[(1-methyl-1H-pyrrol-2-ylacetyl)amino]phenyl]-1,2,3,4,8,9,10,11-octahydronaphtho[1,2-b][1,4]diazepine-2,4(3H,5H)-dione;
(Compound B108) 5-[4-(2-chlorobenzyl)carbamoylphenyl]-1H-naphtho[1,2-b][1,4]diazepine-2,4(3H,5H)-dione;
(Compound B109) 5-[4-[3-(2-chlorophenyl)propenoylamino]phenyl]-1H-naphtho[1,2-b][1,4]diazepine-2,4(3H,5H)-dione;
(Compound B110) 5-[4-(2-chlorophenyl)carbamoylphenyl]-1H-naphtho[1,2-b][1,4]diazepine-2,4(3H,5H)-dione;

(Compound B111) 5-[4-(6-bromo-2,3-methylenedioxybenzoylamino)phenyl]-1H-naphtho[1,2-b][1,4]diazepine-2,4(3H,5H)-dione;
(Compound B112) 5-[4-(6-bromo-2-methoxybenzoylamino)phenyl]-1H-naphtho[1,2-b][1,4]diazepine-2,4(3H,5H)-dione;
(Compound B113) 5-[4-[(2-tert-butylbenzoyl)amino]phenyl]-1,2,3,4,8,9,10,11-octahydronaphtho[1,2-b][1,4]diazepine-2,4(3H,5H)-dione;
(Compound B114) 5-[2-(2-iodobenzoyl)aminopyridin-5-yl]-1H-naphtho[1,2-b][1,4]diazepine-2,4(3H,5H)-dione;
(Compound B115) 5-[4-(6-bromo-2-hydroxybenzoylamino)phenyl]-1H-naphtho[1,2-b][1,4]diazepine-2,4(3H,5H)-dione;
(Compound B116) 5-[4-(6-chloro-2-methoxybenzoylamino)phenyl]-1H-naphtho[1,2-b][1,4]diazepine-2,4(3H,5H)-dione;
(Compound B117) 5-[4-(2-iodobenzoylamino)phenyl]-1H-[1,4]diazepino[2,3-h]quinoline-2,4(3H,5H)-dione;
(Compound B118) 5-[4-(6-chloro-2-hydroxybenzoylamino)phenyl]-1H-naphtho[1,2-b][1,4]diazepine-2,4(3H,5H)-dione;
(Compound B119) 5-[4-(2-hydroxy-6-methoxybenzoylamino)phenyl]-1H-naphtho[1,2-b][1,4]diazepine-2,4(3H,5H)-dione;
(Compound B120) 5-[4-[2-methoxy-6-(trifluoromethyl)benzoylamino]phenyl]-1H-naphtho[1,2-b][1,4]diazepine-2,4(3H,5H)-dione;

(Compound B121) 5-[4-[2-hydroxy-6-(trifluoromethyl)benzoylamino]phenyl]-1H-naphtho[1,2-b][1,4]diazepine-2,4(3H,5H)-dione;
(Compound B122) 5-[4-[(2-isopropenylbenzoyl)amino]phenyl]-1,2,3,4,8,9,10,11-octahydronaphtho[1,2-b][1,4]diazepine-2,4(3H,5H)-dione;
(Compound B123) 5-[4-[(2-isopropylbenzoyl)amino]phenyl]-1,2,3,4,8,9,10,11-octahydronaphtho[1,2-b][1,4]diazepine-2,4(3H,5H)-dione;
(Compound B124) 5-[4-[2-chloro-5-(methylthio)benzoylamino]phenyl]-1H-naphtho[1,2-b][1,4]diazepine-2,4(3H,5H)-dione;
(Compound B125) 5-[4-[2-(methylthio)benzoylamino]phenyl]-1H-naphtho[1,2-b][1,4]diazepine-2,4(3H,5H)-dione;
(Compound B126) 5-[4-[3-(methylthio)benzoylamino]phenyl]-1H-naphtho[1,2-b][1,4]diazepine-2,4(3H,5H)-dione;
(Compound B127) 5-[4-[2-ethyl-6-methoxybenzoylamino]phenyl]-1H-naphtho[1,2-b] [1,4]diazepine-2,4(3H,5H)-dione;
(Compound B128) 5-[4-(3-methanesulfonylbenzoylamino)phenyl]-1H-naphtho[1,2-b] [1,4]diazepine-2,4(3H,5H)-dione;
(Compound B129) 6-ethyl-1-[4-(2-iodobenzoyl)aminophenyl]-1H-1,5-benzodiazepine-2,4(3H,5H)-dione;
(Compound B130) 5-[4-[2-ethyl-6-hydroxybenzoylamino]phenyl]-1H-naphtho[1,2-b] [1,4]diazepine-2,4(3H,5H)-dione;

(Compound B131) 5-[4-(3-methanesulfinylbenzoylamino)phenyl]-1H-naphtho[1,2-b] [1,4]diazepine-2,4(3H,5H)-dione;
(Compound B132) 5-[4-(2-chloro-5-methanesulfinylbenzoylamino)phenyl]-1H-naphtho[1,2-b] [1,4]diazepine-2,4(3H,5H)-dione;
(Compound B133) 5-[4-(2-methanesulfinylbenzoylamino)phenyl]-1H-naphtho[1,2-b] [1,4]diazepine-2,4(3H,5H)-dione;
(Compound B134) 5-[4-[[2-(4-morpholinyl)acetyl]amino]phenyl]-1,2,3,4,8,9,10,11-octahydronaphtho[1,2-b] [1,4]diazepine-2,4(3H,5H)-dione hydrochloride;
(Compound B135) 5-[4-(2-chloro-6-methoxybenzoylamino)phenyl]-1,3-dihydronaphtho[1,2-e]-1,4-diazepin-2-one;
(Compound B136) 5-[4-[[(3-chloropyridin-2-yl)carbonyl]amino]phenyl]-1H-naphtho[1,2-b][1,4]diazepine-2,4(3H,5H)-dione;
(Compound B137) 5-[4-(2-chloro-6-hydroxybenzoylamino)phenyl]-1,3-dihydronaphtho[1,2-e]-1,4-diazepin-2-one;
(Compound B138) 5-[4-(3-chloro-2-methoxybenzoylamino)phenyl]-1H-naphtho[1,2-b][1,4]diazepine-2,4(3H,5H)-dione;
(Compound B139) 5-[4-[(3-methylpyridin-2-yl)carbonylamino]phenyl]-1H-naphtho[1,2-b][1,4]diazepine-2,4(3H,5H)-dione;
(Compound B140) 5-[4-[[(3-chloropyridin-2-yl)carbonyl]amino]phenyl]-1,2,3,4,8,9,10,11-octahydronaphtho[1,2-b][1,4]diazepine-2,4(3H,5H)-dione;

(Compound B141) 5-[4-(3-chloro-2-hydroxybenzoylamino)phenyl]-1H-naphtho[1,2-b][1,4]diazepine-2,4(3H,5H)-dione;
(Compound B142) 5-[4-[[(3-hydroxypyridin-2-yl)carbonyl]amino]phenyl]-1H-naphtho[1,2-b][1,4]diazepine-2,4(3H,5H)-dione;
(Compound B143) 5-[4-[(3-vinylpyridin-2-yl)carbonylamino]phenyl]-1H-naphtho[1,2-b][1,4]diazepine-2,4(3H,5H)-dione;
(Compound B144) 5-[4-[(3-ethylpyridin-2-yl)carbonylamino]phenyl]-1H-naphtho[1,2-b][1,4]diazepine-2,4(3H,5H)-dione;
(Compound B145) N-[4-(2,4-dioxo-1,2,3,4-tetrahydronaphtho-[1,2-b][1,4]-diazepin-5-yl)phenyl]-2-nitrobenzenesulfonamide;
(Compound B146) N-[4-(2,4-dioxo-1,2,3,4-tetrahydronaphtho[1,2-b][1,4]diazepin-5-yl)phenyl]benzenesulfonamide;
(Compound B147) 3-bromo-N-[4-(2,4-dioxo-1,2,3,4-tetrahydronaphtho[1,2-b][1,4]diazepin-5-yl)phenyl]benzenesulfonamide;
(Compound B148) N-[4-(2,4-dioxo-1,2,3,4-tetrahydronaphtho[1,2-b][1,4]diazepin-5-yl)phenyl]-3-methoxybenzenesulfonamide;
(Compound B149) N-[3-(2-oxo-2,3-dihydro-1H-naphtho[1,2-e][1,4]diazepin-5-yl)phenyl]benzenesulfonamide;
(Compound B150) N-[3-(2,4-dioxo-1,2,3,4-tetrahydronaphtho-[1,2-b][1,4]-diazepin-5-yl)phenyl]-2-nitrobenzenesulfonamide;

(Compound B151) N-[4-(2,4-dioxo-1,2,3,4,8,9,10,11-octahydro-naphtho[1,2-b][1,4]-diazepin-5-yl)phenyl]-2-nitro-benzenesulfonamide;
(Compound B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
(Compound B153) N-[3-(2,4-dioxo-1,2,3,4-tetrahydronaphtho-[1,2-b][1,4]-diazepin-5-yl)phenyl]-N-methyl-2-nitrobenzenesulfonamide;
(Compound B154) 4-(2,4-dioxo-1,2,3,4,8,9,10,11-octahydronaphtho[1,2-b][1,4]diazepin-5-yl)-N-phenylbenzenesulfonamide;
(Compound B155) N-[3-(2,4-dioxo-1,2,3,4-tetrahydronaphtho[1,2-b]-[1,4]diazepin-5-yl)phenyl]-2-naphthalenesulfonamide;
(Compound B156) N-[3-(2,4-dioxo-1,2,3,4-tetrahydronaphtho[1,2-b]-[1,4]diazepin-5-yl)phenyl]-1-naphthalenesulfonamide;
(Compound B157) N-[4-(2,4-dioxo-1,2,3,4-tetrahydronaphtho[1,2-b][1,4]diazepin-5-yl)phenyl]cyclohexanesulfonamide;
(Compound B158) N-[4-(2,4-dioxo-1,2,3,4-tetrahydronaphtho[1,2-b][1,4]diazepin-5-yl)phenyl]-3-pyridinesulfonamide hydrochloride;
(Compound B159) N-[4-(2,4-dioxo-1,2,3,4-tetrahydronaphtho[1,2-b][1,4]diazepin-5-yl)phenyl]-4-isopropylbenzenesulfonamide;
(Compound B160) N-[4-(2,4-dioxo-1,2,3,4-tetrahydronaphtho[1,2-b][1,4]diazepin-5-yl)phenyl]phenylmethanesulfonamide;

(Compound B161) N-[4-(2,4-dioxo-1,2,3,4-tetrahydronaphtho[1,2-b][1,4]diazepin-5-yl)phenyl]-2-thiophene-sulfonamide;
(Compound B162) N-[4-(2,4-dioxo-1,2,3,4-tetrahydronaphtho[1,2-b][1,4]diazepin-5-yl)phenyl]-2-naphthalenesulfonamide;
(Compound B163) 4-(2,4-dioxo-1,2,3,4,8,9,10,11-octahydronaphtho-[1,2-b][1,4]diazepin-5-yl)phenyl 3-bromobenzene-sulfonate;
(Compound B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
(Compound B165) N-benzyl-N-[4-(2,4-dioxo-1,2,3,4-tetrahydronaphtho[1,2-b][1,4]diazepin-5-yl)phenyl]-2-nitrobenzenesulfonamide;
(Compound B166) 3-bromo-N-[4-(2,4-dioxo-1,2,3,4-tetrahydronaphtho[1,2-b][1,4]diazepin-5-yl)phenyl]-N-methylbenzenesulfonamide;
(Compound B167) N-[4-(2,4-dioxo-1,2,3,4-tetrahydronaphtho-[1,2-b][1,4]-diazepin-5-yl)phenyl]-N-methyl-2-nitrobenzenesulfonamide;
(Compound B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(Compound B169) N-[4-(7-chloro-2,4-dioxo-2,3,4,5-tetrahydro-1H-benzo[b][1,4]diazepin-1-yl)phenyl]benzenesulfonamide;
(Compound B170) N-[4-(7-bromo-2,4-dioxo-2,3,4,5-tetrahydro-1H-benzo[b][1,4]diazepin-1-yl)phenyl]benzenesulfonamide;

(Compound B171) N-[4-[(2,4-dioxo-7-(trifluoromethyl)-2,3,4,5-tetrahydro-1H-benzo[b][1,4]diazepin-1-yl)]phenyl]benzenesulfonamide;
(Compound B172) N-[4-(2,4-dioxo-2,3,4,5-tetrahydro-1H-benzo[b][1,4]diazepin-1-yl)phenyl]benzenesulfonamide;
(Compound B173) 1-(2-chlorophenyl)-N-[4-(2,4-dioxo-1,2,3,4-tetrahydronaphtho[1,2-b][1,4]diazepin-5-yl)phenyl]methanesulfonamide;
(Compound B174) 1-(3-bromophenyl)-N-[4-(2,4-dioxo-1,2,3,4-tetrahydronaphtho[1,2-b][1,4]diazepin-5-yl)phenyl]methanesulfonamide;
(Compound B175) N-[4-(2,4-dioxo-1,2,3,4-tetrahydronaphtho-[1,2-b][1,4]-diazepin-5-yl)phenyl]-2-trifluoromethylbenzenesulfonamide;
(Compound B176) N-[4-(7-bromo-6-methyl-2,4-dioxo-2,3,4,5-tetrahydro-1H-benzo[b][1,4]diazepin-1-yl)phenyl]benzenesulfonamide;
(Compound B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
(Compound B178) 3-bromo-N-[4-(2,4-dioxo-1,2,3,4,8,9,10,11-octahydronaphtho[1,2-b][1,4]diazepin-5-yl)phenyl]benzenesulfonamide;
(Compound B179) N-[4-(2,4-dioxo-1,2,3,4,8,9,10,11-octahydronaphtho[1,2-b][1,4]diazepin-5-yl)phenyl]-3-methoxybenzenesulfonamide;
(Compound B180) 1-(2-bromophenyl)-N-[4-(2,4-dioxo-1,2,3,4-tetrahydronaphtho[1,2-b][1,4]diazepin-5-yl)phenyl]methanesulfonamide;

(Compound B181) N-[4-(2,4-dioxo-1,2,3,4-tetrahydronaphtho[1,2-b][1,4]diazepin-5-yl)phenyl]-1-(2-methylphenyl)methanesulfonamide;
(Compound B182) N-[4-(2,4-dioxo-1,2,3,4-tetrahydronaphtho[1,2-b][1,4]diazepin-5-yl)phenyl]-1-(2-nitrophenyl)methanesulfonamide;
(Compound B183) N-[4-(2,4-dioxo-1,2,3,4-tetrahydronaphtho[1,2-b][1,4]diazepin-5-yl)phenyl]-2-phenylethanesulfonamide;
(Compound B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B185) 1-(2-chlorophenyl)-N-[4-(2,4-dioxo-7-methoxy-1H-benzo[1,2-b][1,4]diazepin-1-yl)phenyl]methanesulfonamide;
(Compound B186) 1-(2-chlorophenyl)-N-[4-(2,4-dioxo-7-hydroxy-1H-benzo[1,2-b][1,4]diazepin-1-yl)phenyl]methanesulfonamide;
(Compound B187) 1-(4-chlorophenyl)-N-[4-(2,4-dioxo-1,2,3,4-tetrahydronaphtho[1,2-b][1,4]diazepin-5-yl)phenyl]methanesulfonamide;
(Compound B188) 1-(2-chlorophenyl)-N-[4-(2,4-dioxo-1,2,3,4-tetrahydronaphtho[1,2-b][1,4]diazepin-5-yl)benzyl]methanesulfonamide;
(Compound B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
(Compound B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;

(Compound B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B192) 1-(2-chlorophenyl)-N-[4-(2,4-dioxo-6-methyl-1H-benzo[1,2-b][1,4]diazepin-1-yl)phenyl]methanesulfonamide;
(Compound B193) 1-(2-chlorophenyl)-N-[3-(2,4-dioxy-1,2,3,4-tetrahydronaphtho[1,2-b][1,4]diazepin-5-yl)propyl]methanesulfonamide;
(Compound B194) 1-(2-chlorophenyl)-N-[2-(2,4-dioxo-1,2,3,4-tetrahydronaphtho[1,2-b][1,4]diazepin-5-yl)ethyl]methanesulfonamide;
(Compound B195) N-[4-(2,4-dioxo-1,2,3,4,8,9,10,11-octahydronaphtho[1,2-b][1,4]diazepin-5-yl)phenyl]-1-(2-iodophenyl)methanesulfonamide;
(Compound B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
(Compound B197) 1-(2-chlorophenyl)-N-[4-(2-oxo-2,3-dihydro-1H-naphtho[1,2-e][1,4]diazepin-5-yl)phenyl]methanesulfonamide;
(Compound B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
(Compound B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
(Compound B200) 1-(2,3-dimethylphenyl)-N-[4-(2,4-dioxo-1,2,3,4-tetrahydronaphtho[1,2-b] [1,4]diazepin-5-yl)phenyl]phenyl-N-methylmethanesulfonamide;

(Compound B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
(Compound B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B204) 1-(2-dimethylaminophenyl)-N-[4-(2,4-dioxo-1,2,3,4-tetrahydronaphtho[1,2-b] [1,4]diazepin-5-yl)phenyl]phenyl-N-methylmethanesulfonamide;
(Compound B205) 5-[4-[(pyridin-4-yl)carbonylamino]phenyl]-1H-naphtho[1,2-b] [1,4]diazepine-2,4(3H,5H)-dione hydrochloride;
(Compound B206) 5-[4-[2-[(pyridin-3-yl)oxy]acetylamino]phenyl]-1H-naphtho[1,2-b] [1,4]diazepine-2,4(3H,5H)-dione hydrochloride;
(Compound B207) 5-[4-[(pyridin-3-yl)carbonylamino]phenyl]-1H-naphtho[1,2-b] [1,4]diazepine-2,4(3H,5H)-dione hydrochloride;
(Compound B208) 5-[4-[(2-methylpyridin-3-yl)carbonylamino]phenyl]-1H-naphtho[1,2-b] [1,4]diazepine-2,4(3H,5H)-dione hydrochloride;
(Compound B209) 5-[4-[(2-chloropyridin-3-yl)carbonylamino]phenyl]-1H-naphtho[1,2-b] [1,4]diazepine-2,4(3H,5H)-dione;
(Compound B210) 5-[4-[2-[(pyridin-2-yl)oxy]acetylamino]phenyl]-1H-naphtho[1,2-b] [1,4]diazepine-2,4(3H,5H)-dione;

(Compound B211) 5-[4-[[4-(trifluoromethyl)pyridin-3-yl]carbonylamino]phenyl]-1H-naphtho[1,2-b][1,4]diazepine-2,4(3H,5H)-dione;
(Compound B212) 5-[4-[(2-chloropyridin-3-yl)carbonylamino]phenyl]-1H-[1,4]diazepino[2,3-f]isoquinoline-2,4(3H,5H)-dione;
(Compound B213) 5-[4-[(2-chloropyridin-3-yl)carbonylamino]phenyl]-8,9,10,11-tetrahydro-1H-[1,4]diazepino[2,3-f]isoquinoline-2,4(3H,5H)-dione; and
(Compound B214) 5-[4-[(2-isopropylbenzoyl)amino]phenyl]-1H-naphtho[1,2-b] [1,4]diazepine-2,4(3H,5H)-dione

A more preferable compound or a pharmaceutically acceptable salt thereof as the active ingredient of the pharmaceutical composition of the present invention is comprised in the compounds represented by the general formulas (A) to (BII) or a pharmaceutically acceptable salt thereof. Specific examples of the more preferable compound or a pharmaceutically acceptable salt thereof can comprise 5-[3-(1H-tetrazol-5-yl)phenyl]-1H-naphtho[1,2-b][1,4]diazepine-2,4(3H,5H)-dione; a 5-[3-(1H-tetrazol-5-yl)phenyl]-1H-naphtho[1,2-b][1,4]diazepine-2,4(3H,5H)-dione sodium salt; a 5-[3-(1H-tetrazol-5-yl)phenyl]-1H-naphtho[1,2-b][1,4]diazepine-2,4(3H,5H)-dione potassium salt; 5-[3-(5-thioxo-4H-[1,2,4]oxadiazol-3-yl)phenyl]-1H-naphtho[1,2-b][1,4]diazepine-2,4(3H,5H)-dione; a 5-[3-(5-thioxo-4H-[1,2,4]oxadiazol-3-yl)phenyl]-1H-naphtho[1,2-b][1,4]diazepine-2,4(3H,5H)-dione sodium salt; 5-[4-fluoro-3-(1H-tetrazol-5-yl)phenyl]-1H-naphtho[1,2-b][1,4]diazepine-2,4(3H,5H)-dione; a 5-[4-fluoro-3-(1H-tetrazol-5-yl)phenyl]-1H-naphtho[1,2-b][1,4]diazepine-2,4(3H,5H)-dione sodium salt; 5-[4-[2-(trifluoromethyl)benzoyl]aminophenyl]-1H-naphtho[1,2-b][1,4]diazepine-2,4(3H,5H)-dione; 5-[4-[(2-chlorophenylacetyl)amino]phenyl]-1H-naphtho[1,2-b][1,4]diazepine-2,4(3H,5H)-dione; 1-[4-(2,4-dioxo-1,2,3,4-tetrahydronaphtho[1,2-b][1,4]diazepin-5-yl)phenyl]-3-phenylurea; 5-[4-(2-iodobenzoylamino)phenyl]-1H-naphtho[1,2-b][1,4]diazepine-2,4(3H,5H)-dione; 5-[4-[(2-ethylbenzoyl)amino]phenyl]-1H-naphtho[1,2-b][1,4]diazepine-2,4(3H,5H)-dione; 5-[4-(2-tert-butylbenzoylamino)phenyl]-1H-naphtho[1,2-b][1,4]diazepine-2,4(3H,5H)-dione; 5-[4-(2-iodobenzoyl)aminophenyl]-1,2,3,4,8,9,10,11-octahydronaphtho[1,2-b][1,4]diazepine-2,4(3H,5H)-dione; 5-[4-(6-chloro-2-hydroxybenzoylamino)phenyl]-1H-naphtho[1,2-b][1,4]diazepine-2,4(3H,5H)-dione; N-[4-(2,4-dioxo-1,2,3,4-tetrahydronaphtho[1,2-b][1,4]diazepin-5-yl)phenyl]benzenesulfonamide; 1-(2-chlorophenyl)-N-[4-(2,4-dioxo-1,2,3,4-tetrahydronaphtho[1,2-b][1,4]diazepin-5-yl)phenyl]methanesulfonamide; 1-(2-chlorophenyl)-N-[4-(2,4-dioxo-1,2,3,4,8,9,10,11-octahydronaphtho[1,2-b][1,4]diazepin-5-yl)phenyl]methanesulfonamide; 1-(2-bromophenyl)-N-[4-(2,4-dioxo-1,2,3,4-tetrahydronaphtho[1,2-b][1,4]diazepin-5-yl)phenyl]methanesulfonamide; N-[4-(2,4-dioxo-1,2,3,4-tetrahydronaphtho[1,2-b][1,4]diazepin-5-yl)phenyl]-1-(2-methylphenyl)methanesulfonamide; N-[4-(2,4-dioxo-1,2,3,4-tetrahydronaphtho[1,2-b][1,4]diazepin-5-yl)phenyl]-2-phenylethanesulfonamide; 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide; 1-(2-chlorophenyl)-N-[4-(2-oxo-2,3-dihydro-1H-naphtho[1,2-e][1,4]diazepin-5-yl)phenyl]methanesulfonamide; 5-[4-[(2-methylpyridin-3-yl)carbonylamino]phenyl]-1H-naphtho[1,2-b][1,4]diazepine-2,4(3H,5H)-dione hydrochloride; 5-[4-[(2-chloropyridin-3-yl)carbonylamino]phenyl]-1H-naphtho[1,2-b][1,4]diazepine-2,4(3H,5H)-dione; 5-[4-[2-[(pyridin-2-yl)oxy]acetylamino]phenyl]-1H-naphtho[1,2-b][1,4]diazepine-2,4(3H,5H)-dione; and 5-[4-[(2-isopropylbenzoyl)amino]phenyl]-1H-naphtho[1,2-b] [1,4]diazepine-2,4(3H,5H)-dione. Note that the active ingredient of the pharmaceutical composition of the present invention is not limited to the above specific compounds or a pharmaceutically acceptable salt thereof.

An even more preferable compound or a pharmaceutically acceptable salt thereof as the active ingredient of the pharmaceutical composition of the present invention is comprised in the compounds represented by the general formulas (A) to (BII) or a pharmaceutically acceptable salt thereof. Specific examples of the even more preferable compound or a pharmaceutically acceptable salt thereof can comprise 5-[3-(1H-tetrazol-5-yl)phenyl]-1H-naphtho[1,2-b][1,4]diazepine-2,4(3H,5H)-dione; a 5-[3-(1H-tetrazol-5-yl)phenyl]-1H-naphtho[1,2-b][1,4]diazepine-2,4(3H,5H)-dione sodium salt; 5-[3-(5-thioxo-4H-[1,2,4]oxadiazol-3-yl)phenyl]-1H-naphtho[1,2-b] [1,4]diazepine-2,4(3H,5H)-dione; a 5-[3-(5-thioxo-4H-[1,2,4]oxadiazol-3-yl)phenyl]-1H-naphtho[1,2-b] [1,4]diazepine-2,4(3H,5H)-dione sodium salt; 5-[4-[2-(trifluoromethyl)benzoyl]aminophenyl]-1H-naphtho[1,2-b][1,4]diazepine-2,4(3H,5H)-dione; 5-[4-(2-iodobenzoylamino)phenyl]-1H-naphtho[1,2-b][1,4]diazepine-2,4(3H,5H)-dione; and 5-[4-(6-chloro-2-hydroxybenzoylamino)phenyl]-1H-naphtho[1,2-b] [1,4]diazepine-2,4(3H,5H)-dione. Note that the active ingredient of the pharmaceutical composition of the present invention is not limited to the above specific compounds or a pharmaceutically acceptable salt thereof.

Stereoisomers of the compounds represented by the general formulas (A) to (BII), such as a cis-trans isomer, an optically active form, or a racemic form thereof, may exist as well, all of which are comprised in the present invention.

Tautomers of the compounds represented by the general formulas (A) to (BII) may exist as well, and the tautomers show the same activities as those of the compounds and are comprised in the present invention.

Furthermore, the compounds represented by the general formulas (A) to (BII) may have one or two or more asymmetric carbons depending on the type of substituent, and any optical isomer or any mixture or a racemic form of optical isomers, which is based on the asymmetric carbon, or a diastereoisomer or any mixture of diastereoisomers, which is based on two or more asymmetric carbons, may also be used as the active ingredient of the pharmaceutical composition of the present invention. In a case where the compounds represented by the general formulas (A) to (BII) have a double bond or a cyclic structure, a geometric isomer may exist, and, in addition to a pure form of the geometric isomer, a mixture of geometric isomers, which are contained in the mixture at any ratio, may also be used as the active ingredient of the pharmaceutical composition of the present invention.

In addition to the compounds represented by the general formulas (A) to (BII), any solvate of a free form of the compound or a salt form of the compound may also be used as active ingredient of the pharmaceutical composition of the present invention. The solvate also comprises a hydrate.

In the present specification, unless otherwise specified, the compounds represented by general formulas (A) to (BII) can comprise the stereoisomer of the compound, such as the cis-trans isomer, the optically active form, or the racemic form thereof; the tautomer of the compound; any optical isomer or racemic form of the compound, which is based on an asymmetric carbon; and the diastereoisomer of the compound and a mixture thereof, which is based on two or more asymmetric carbons.

In addition to the compounds represented by the general formulas (A) to (BII), acid addition salts or base addition salts of these compounds may also be used as the active ingredient of the medicament of the present invention. As the acid addition salt, for example, a mineral acid salt such as hydrochloride, sulfate, and nitrate; an organic acid salt such as methanesulfonate, p-toluenesulfonate, oxalate, and malate; and the like can be used, however, the acid addition salt is not limited thereto. Examples of the base addition salt can comprise a metal salt such as a lithium salt, a sodium salt, a potassium salt, a magnesium salt, and a calcium salt; an ammonium salt; an organic amine salt such as a triethylamine salt and an ethanolamine salt; and the like, however, the base addition salt is not limited thereto. Among these salts, a pharmaceutically acceptable salt is preferably used as the active ingredient of the pharmaceutical composition of the present invention.

The pharmaceutical composition provided by the present invention can be used for preventing or treating respiratory disease, particularly, inflammatory respiratory disease.

In addition, the pharmaceutical composition provided by the present invention can be used for preventing or treating, for example, bronchial obstruction, allergic pulmonary disease, or interstitial lung disease.

The pharmaceutical composition provided by the present invention can be used for preventing or treating, for example, COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia.

In one aspect, the pharmaceutical composition provided by the present invention can be used for preventing or treating the respiratory disease which is accompanied by depression of respiratory function.

In one aspect, the pharmaceutical composition provided by the present invention can be used for preventing or treating the respiratory disease which is accompanied by airflow obstruction.

In one aspect, the pharmaceutical composition provided by the present invention can be used for preventing or treating the respiratory disease which is accompanied by respiratory inflammation.

In one aspect, the pharmaceutical composition provided by the present invention can be used for preventing or treating the respiratory disease which is accompanied by cough reflex.

In an experiment in which the respiratory function in a cigarette smoke-exposed model obtained using guinea pigs was measured, the pharmaceutical composition provided by the present invention showed the tendency of increasing the tidal volume and the minute volume in a dose-dependent manner compared to the control (placebo) group, and the tendency of decreasing the respiratory rate in a dose-dependent manner compared to the control (placebo) group.

Therefore, in one aspect, it is suggested that the pharmaceutical composition provided by the present invention has a therapeutic effect against the respiratory disease accompanied by depression of respiratory function.

In an experiment in which the respiratory function in the cigarette smoke-exposed model obtained using guinea pigs was measured, the pharmaceutical composition provided by the present invention showed the tendency of decreasing specific airway resistance (sRaw) in a dose-dependent manner compared to the control (placebo) group, and especially in the results on Day 13, the specific airway resistance was significantly decreased in a high-dose group. Furthermore, peak expiratory flow (PEF) which indicates the force of exhalation showed the tendency of being increased in a dose-dependent manner compared to the control (placebo) group.

Therefore, in one aspect, it is suggested that the pharmaceutical composition provided by the present invention has a therapeutic effect against the respiratory disease accompanied by airflow obstruction.

As for the number of cells in bronchoalveolar lavage fluid (BALF) in the cigarette smoke-exposed model obtained using guinea pigs, the pharmaceutical composition provided by the present invention showed the tendency of decreasing the number of inflammatory cells (WBCs, macrophages, and neutrophils) in a dose-dependent manner compared to the control (placebo) group, and especially in the results for WBCs and macrophages, significantly decrease was observed.

Therefore, in one aspect, it is suggested that the pharmaceutical composition provided by the present invention has a therapeutic effect against the respiratory disease accompanied by respiratory inflammation.

In an experiment in which cough reflex in the cigarette smoke-exposed model obtained using guinea pigs was measured, the pharmaceutical composition provided by the present invention showed the tendency of decreasing the number of cough reflexes in a dose-dependent manner compared to the control (placebo) group.

Therefore, in one aspect, it is suggested that the pharmaceutical composition provided by the present invention has a therapeutic effect against the respiratory disease accompanied by cough reflex.

The pharmaceutical composition of the present invention can be administered orally or parenterally. The pharmaceutical composition of the present invention can be produced as a pharmaceutical composition in an appropriate dosage form such as tablets, granules, a powder, capsules, a suspending agent, an inhalant, a dry powder inhaler, an inhalation liquid, an inhalation aerosol, an injection, and suppositories, according to the conventional method in the technical field of formulation.

These pharmaceutical preparations can be manufactured using a general technique. For example, in a case of the tablet, a general pharmaceutically acceptable additive such as a diluent, a disintegrant, a binder, a lubricant, and a coloring agent can be used. Examples of the diluent can comprise lactose, D-mannitol, microcrystalline cellulose, glucose, and the like. Examples of the disintegrant can comprise starch, carboxymethylcellulose calcium (CMC-Ca), and the like. Examples of the lubricant can comprise magnesium stearate, talc, and the like. Examples of the binder can comprise hydroxypropyl cellulose (HPC), gelatin, polyvinyl pyrrolidone (PVP), and the like.

A pharmaceutically acceptable additive such as a solvent, a stabilizer, a solubilizing agent, a suspension, an emulsifier, an analgesic agent, a buffer, and a preservative is used in preparation of the injection. The pharmaceutically acceptable additive and a method of preparing a pharmaceutical preparation can be suitably selected by those skilled in the art.

Types of the inhalation for the parenteral administration comprise an aerosol, a dry powder for inhalation, a liquid for inhalation (for example, a solution for inhalation, a suspension for inhalation, and the like), and a capsule inhalation, and the liquid for inhalation may be in a form that is used by being dissolved or suspended in water or another suitable medium at the time of use. The inhalation can be applied using a suitable inhalation container. For example, when administering the liquid for inhalation, a sprayer (an atomizer or a nebulizer) or the like can be used, and when administering the dry powder for inhalation, an inhaler for a dry powder drug or the like can be used.

The inhalation can be manufactured according to a known method. For example, the inhalation is manufactured by obtaining a dry powder or a liquid of the compounds represented by the general formulas (A) to (BII), blending the dry powder or the liquid of the compounds into an inhalation propellant or a carrier, and filling a suitable inhalation container with the blended product. In a case of powderizing the compounds represented by the general formulas (A) to (BII), the powderization is performed according to a general method. For example, a dry powder is prepared by obtaining a fine powder of the compounds and lactose, starch, magnesium stearate, or the like, thereby obtaining a homogeneous mixture, or by granulating the compounds. In addition, in a case where the compounds represented by the general formulas (A) to (BII) are liquefied, for example, the compounds may be dissolved in a liquid carrier such as water, physiological saline, and an organic solvent. As the propellant, a conventionally known propellant, for example, alternative chlorofluorocarbon, a liquefied gas propellant (for example, fluorohydrocarbon, liquefied petroleum, diethyl ether, dimethyl ether, and the like), a compressed gas (for example, a soluble gas (for example, carbon dioxide gas, nitrous oxide gas, and the like), an insoluble gas (for example, nitrogen gas and the like), and the like are used.

A pharmaceutically acceptable additive may be suitably blended into the inhalation as necessary. The additive may be any additive that is generally used. For example, a solid diluent (for example, sucrose, lactose, glucose, mannitol, sorbitol, maltose, cellulose, and the like), a liquid diluent (for example, propylene glycol and the like), a binder (starch, dextrin, methylcellulose, hydroxypropylcellulose, polyvinylpyrrolidone, polyethylene glycol, sucrose, and the like), a lubricant (for example, magnesium stearate, light anhydrous silicic acid, talc, sodium lauryl sulfate, and the like), a corrigent (for example, citric acid, menthol, glycyrrhizin ammonium salt, glycine, an orange powder, and the like), a preservative (for example, sodium benzoate, sodium bisulfite, methylparaben, propylparaben, and the like), a stabilizer (for example, citric acid, sodium citrate, and the like), a suspending agent or an emulsifier (for example, methyl cellulose, polyvinyl pyrrolidone, polyvinyl alcohol, lecithin, sorbitan trioleate, and the like), a dispersing agent (for example, a surfactant and the like), a solvent (for example, water and the like), a tonicity agent (for example, sodium chloride, concentrated glycerin, and the like), a pH regulator (for example, hydrochloric acid, sulfuric acid, and the like), a solubilizer (for example, ethanol and the like), an antiseptic agent (benzalkonium chloride, paraben, and the like), a colorant, a buffer agent (sodium phosphate, sodium acetate, and the like), a thickening agent (cariboxyvinyl polymer and the like), an absorption promoter, and the like are used. The liquid for inhalation is prepared by, for example, suitably selecting the antiseptic agent, the colorant, the buffer agent, the tonicity agent, the thickening agent, the absorption promoter, or the like as necessary. Furthermore, the dry powder for inhalation is prepared by, for example, suitably selecting the lubricant, the binder, the diluent, the colorant, the antiseptic agent, the absorption promoter (bile salt, chitosan, and the like), or the like as necessary.

Furthermore, in order to prepare the compounds represented by the general formulas (A) to (BII) in a sustained release form, the inhalation may contain a biodegradable polymer. Examples of the biodegradable polymer can comprise a fatty acid ester polymer or a copolymer thereof, polyacrylic acid esters, polyhydroxybutyric acids, polyalkylene oxalates, polyorthoester, polycarbonate, and polyamino acids, and one polymer can be used alone, or two or more thereof can be used in combination. Furthermore, a phospholipid such as egg yolk lecithin, chitosan, or the like may also be used. Examples of the fatty acid ester polymer or a copolymer thereof can comprise polylactic acid, polyglycolic acid, polycitric acid, polymalic acid, and a lactic acid-glycolic acid copolymer, and one polymer or copolymer can be used alone, or two or more thereof can be used in combination. In addition, one of poly-α-cyanoacrylic acid ester, poly-β-hydroxybutyric acid, polytrimethylene oxide, polyorthoester, polyorthocarbonate, polyethylenecarbonate, poly-γ-benzyl-L-glutamic acid, and poly-L-alanine can be used alone, or two or more thereof can be used in combination. The fatty acid ester polymer or a copolymer thereof is preferably polylactic acid, polyglycolic acid, or a lactic acid-glycolic acid copolymer, and is more preferably a lactic acid-glycolic acid copolymer. Furthermore, a microsphere or a nanosphere may be prepared by encapsulating a drug using the biodegradable polymer such as a lactic acid-glycolic acid copolymer.

In other words, the pharmaceutical composition of the present invention can be provided as a pharmaceutical composition comprising the active ingredient and the pharmaceutically acceptable additive. Furthermore, the pharmaceutical composition of the present invention can be provided as a medicine including the pharmaceutical composition of the present invention.

A dose of the pharmaceutical composition of the present invention is not particularly limited, and generally, to an adult, approximately 0.01 µg to 100 mg, and preferably 0.3 µg to 10 mg can be administered per day as an active ingredient amount, in a case of administration by an inhalant, a dry powder inhaler, an inhalation liquid, an inhalation aerosol, approximately 0.01 mg to 100 mg can be administered per day as an active ingredient amount, in a case of administration by an injection, and approximately 0.01 mg to 2,000 mg can be administered per day, in a case of oral administration. Note that the dose is not limited to the above doses, and the dose can be increased or decreased depending on the age or symptom.

The pharmaceutical composition of the present invention can be used in combination with another drug that is useful in treating or preventing various respiratory diseases, particularly, inflammatory respiratory diseases. Individual components in such combination can be administered in divided preparations or in a single preparation separately at different times or at the same time, during the period of the treatment or prevention. Therefore, it should be understood that the present invention includes both administration at the same time or administrations at different times, and the administration in the present invention should be understood in such a way. The scope of the combination of the pharmaceutical composition of the present invention and another drug that is useful in treating or preventing the various respiratory diseases, particularly, inflammatory respiratory diseases includes, in principle, a combination with a certain medicinal preparation that is useful in treating or preventing the various respiratory diseases, particularly, inflammatory respiratory diseases. Another drug that is useful in treating or preventing the various respiratory diseases, particularly, inflammatory respiratory diseases can also include a pharmaceutical composition useful in treating or preventing inflammation per se which causes the shortness of breath or the like.

Various forms can be selected for each preparation in the combined preparation according to the present invention, and each preparation can be produced in the same manner as the above-described preparations. Furthermore, a drug combination which includes the pharmaceutical composition of the present invention and another drug for treating or preventing various respiratory diseases, particularly, inflammatory respiratory diseases can also be easily produced by those skilled in the art according to a conventional method or a conventional art.

The combination includes not only a combination of the pharmaceutical composition of the present invention with one other active substance, but also a combination of the pharmaceutical composition of the present invention with two or more other active substances. There are many examples of the combination of the pharmaceutical composition of the present invention and one, two, or more activators selected from the drug for treating or preventing the various respiratory diseases, particularly, inflammatory respiratory diseases.

The present invention has the following aspects.
<1a> A method of preventing or treating respiratory disease, the method including administering a therapeutically effective dose of a compound having a P2X4 receptor antagonistic action (for example, the compounds represented by General Formulas (A) to (BII)) or a pharmaceutically acceptable salt thereof to a subject in need of the method (for example, mammals including a human) ;
<2a> the method according to <1a>, in which the respiratory disease is inflammatory respiratory disease;
<3a> the method according to <2a>, in which the inflammatory respiratory disease is bronchial obstruction, allergic pulmonary disease, or interstitial lung disease;
<4a> the method according to <2a> or <3a>, in which the inflammatory respiratory disease is COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia;
<5a> the method according to any one of <1a> to <4a>, in which the respiratory disease is respiratory disease accompanied by depression of respiratory function;
<6a> the method according to any one of <1a> to <5a>, in which the respiratory disease is respiratory disease accompanied by airflow obstruction;
<7a> the method according to any one of <1a> to <6a>, in which the respiratory disease is respiratory disease accompanied by respiratory inflammation; and
<8a> the method according to any one of <1a> to <7a>, in which the respiratory disease is respiratory disease accompanied by cough reflex.

The present invention has the following other aspects.
<1b> A compound having a P2X4 receptor antagonistic action (for example, the compounds represented by General Formulas (A) to (BII)) or a pharmaceutically acceptable salt thereof, which is for use in prevention or treatment of respiratory disease;
<2b> the compound or a pharmaceutically acceptable salt thereof for the use according to <1b>, in which the respiratory disease is inflammatory respiratory disease;
<3b> the compound or a pharmaceutically acceptable salt thereof for the use according to <2b>, in which the inflammatory respiratory disease is bronchial obstruction, allergic pulmonary disease, or interstitial lung disease;
<4b> the compound or a pharmaceutically acceptable salt thereof for the use according to <2b> or <3a>, in which the inflammatory respiratory disease is COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia;
<5b> the compound or a pharmaceutically acceptable salt thereof for the use according to any one of <1b> to <4b>, in which the respiratory disease is respiratory disease accompanied by depression of respiratory function;
<6b> the compound or a pharmaceutically acceptable salt thereof for the use according to any one of <1b> to <5b>, in which the respiratory disease is respiratory disease accompanied by airflow obstruction;
<7b> the compound or a pharmaceutically acceptable salt thereof for the use according to any one of <1b> to <6b>, in which the respiratory disease is respiratory disease accompanied by respiratory inflammation; and
<8b> the compound or a pharmaceutically acceptable salt thereof for the use according to any one of <1b> to <7b>, in which the respiratory disease is respiratory disease accompanied by cough reflex.

The present invention further has the following other aspects.
<1c> Use of a compound having a P2X4 receptor antagonistic action (for example, the compounds represented by the general formulas (A) to (BII)) or a pharmaceutically acceptable salt thereof for producing a pharmaceutical composition for preventing or treating respiratory disease;
<2c> the use according to <1c>, in which the respiratory disease is inflammatory respiratory disease;
<3c> the use according to <2c>, in which the inflammatory respiratory disease is bronchial obstruction, allergic pulmonary disease, or interstitial lung disease;
<4c> the use according to <2c> or <3c>, in which the inflammatory respiratory disease is COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia;
<5a> the use according to any one of <1a> to <4a>, in which the respiratory disease is respiratory disease accompanied by depression of respiratory function.
<6a> the use according to any one of <1a> to <5a>, in which the respiratory disease is respiratory disease accompanied by airflow obstruction;
<7a> the use according to any one of <1a> to <6a>, in which the respiratory disease is respiratory disease accompanied by respiratory inflammation; and
<8a> the use according to any one of <1a> to <7a>, in which the respiratory disease is respiratory disease accompanied by cough reflex.

### Examples

Hereinafter, the present invention is more specifically described with Examples, however, the scope of the present invention is not limited to the following Examples.

In the following Examples, as a P2X4 receptor antagonist, 5-[4-(2-iodobenzoylamino)phenyl]-1H-naphtho[1,2-b] [1,4]diazepine-2,4(3H,5H)-dione (a compound in Example 48 of WO 2013/105608 A: hereinafter, referred to as "Compound A"), and other compounds shown below were used:
5-[4-[2-(trifluoromethyl)benzoyl]aminophenyl]-1H-naphtho[1,2-b] [1,4]diazepine-2,4(3H,5H)-dione; 5-[4-[(2-chlorophenylacetyl)amino]phenyl]-1H-naphtho[1,2-b] [1,4]diazepine-2,4(3H,5H)-dione; 1-[4-(2,4-dioxo-1,2,3,4-tetrahydronaphtho[1,2-b] [1,4]diazepin-5-yl)phenyl]-3-phenylurea; 5-[4-[(2-ethylbenzoyl)amino]phenyl]-1H-naphtho[1,2-b] [1,4]diazepine-2,4(3H,5H)-dione; 5-[4-(2-tert-butylbenzoylamino)phenyl]-1H-naphtho[1,2-b][1,4]diazepine-2,4(3H,5H)-dione; 5-[4-(2-iodobenzoyl)aminophenyl]-1,2,3,4,8,9,10,11-octahydronaphtho[1,2-b][1,4]diazepine-2,4(3H,5H)-dione; 5-[4-(6-chloro-2-hydroxybenzoylamino)phenyl]-1H-naphtho[1,2-b][1,4]diazepine-2,4(3H,5H)-dione; N-[4-(2,4-dioxo-1,2,3,4-tetrahydronaphtho[1,2-b][1,4]diazepin-5-yl)phenyl]benzenesulfonamide; 1-(2-chlorophenyl)-N-[4-(2,4-dioxo-1,2,3,4-tetrahydronaphtho[1,2-b][1,4]diazepin-5-yl)phenyl]methanesulfonamide; 1-(2-chlorophenyl)-N-[4-(2,4-dioxo-1,2,3,4,8,9,10,11-octahydronaphtho[1,2-b][1,4]diazepin-5-yl)phenyl]methanesulfonamide; 1-(2-bromophenyl)-N-[4-(2,4-dioxo-1,2,3,4-tetrahydronaphtho[1,2-b][1,4]diazepin-5-yl)phenyl]methanesulfonamide; N-[4-(2,4-dioxo-1,2,3,4-tetrahydronaphtho[1,2-b][1,4]diazepin-5-yl)phenyl]-1-(2-methylphenyl)methanesulfonamide; N-[4-(2,4-dioxo-1,2,3,4-tetrahydronaphtho[1,2-b][1,4]diazepin-5-yl)phenyl]-2-phenylethanesulfonamide; 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide; 1-(2-chlorophenyl)-N-[4-(2-oxo-2,3-dihydro-1H-naphtho[1,2-e][1,4]diazepin-5-yl)phenyl]methanesulfonamide; 5-[4-[(2-methylpyridin-3-yl)carbonylamino]phenyl]-1H-naphtho[1,2-b][1,4]diazepine-2,4(3H,5H)-dione hydrochloride; 5-[4-[(2-chloropyridin-3-yl)carbonylamino]phenyl]-1H-naphtho[1,2-b] [1,4]diazepine-2,4(3H,5H)-dione; 5-[4-[2-[(pyridin-2-yl)oxy]acetylamino]phenyl]-1H-naphtho[1,2-b] [1,4]diazepine-2,4(3H,5H)-dione; and 5-[4-[(2-isopropylbenzoyl)amino]phenyl]-1H-naphtho[1,2-b][1,4]diazepine-2,4(3H,5H)-dione.

Note that, in Examples of the present application, significant differences are indicated with the symbol "*" in the drawings, which indicates that P < 0.05. In addition, "*" represents a significant difference compared to the control (placebo) group.

Example 1: Measurement of P2X4 Receptor Antagonistic Action of Compound Which Is Active Ingredient of Present Invention)

### (Test Method)

A P2X4 receptor antagonistic action of a compound which is the active ingredient of the present invention was measured. 1321N1 cells into which ATP receptors (human P2X4) were introduced were used as a P2X4 receptor stable expression system. The P2X4-receptor-expressing cells were seeded into a 96-well plate and cultured for 24 hours under a condition of 37°C and 5% CO₂ to be used for calcium measurement. Fura-2AM, which is a calcium fluorescent indicator, was dissolved in an extracellular fluid for calcium imaging, and the seeded cells were treated therewith and left to stand at room temperature for 45 minutes, thereby incorporating Fura-2AM into the cells. EnVision (PerkinElmer) which is a microplate reader was used for the measurement. 510 nm fluorescences F340 and F380 that are emitted when the cells are irradiated with light radiating from a xenon lamp and transmitted through 340 nm and 380 nm filters, respectively, were observed, and change in a ratio value F340/F380 was used as an index of intracellular calcium change. Measurement was performed by adding ATP to each well such that the final concentration of ATP becomes 1 µM and observing an ATP-induced intracellular calcium response over time. Inhibitory activity of a test substance was measured by performing pretreatment on the test substance for 15 minutes by adding ATP thereto, and the inhibitory activity was calculated by comparison with a case of absence of the test substance. The results are shown in the following Table 29.

### (Test Results)

**[Table 29]**

| Test substance | IC₅₀ (µM) |
|---|---|
| Compound A2 | 0.53 |
| Compound A17 | 0.27 |
| Compound B2 | 0.75 |
| Compound B13 | 0.54 |
| Compound B20 | 1.2 |
| Compound B48 (Compound A) | 0.30 |
| Compound B57 | 0.72 |
| Compound B71 | 0.4 |
| Compound B106 | 1.8 |
| Compound B118 | 1.10 |
| Compound B146 | 0.67 |
| Compound B173 | 0.064 |
| Compound B177 | 0.056 |
| Compound B180 | 0.05 |
| Compound B181 | 0.068 |
| Compound B183 | 0.42 |
| Compound B196 | 0.97 |
| Compound B197 | 0.44 |
| Compound B208 | 1.3 |
| Compound B209 | 0.94 |
| Compound B210 | 1.4 |
| Compound B214 | 0.62 |

### <Material and Method>

### (Animals)

Male Hartley guinea pigs (manufactured by Japan SLC, Inc., Slc: Hartley, SPF) were used in the test. The guinea pigs were purchased at 5 weeks old. The weights at the time of the purchase were about 300 to 350 g. After arrival, the guinea pigs were provided with an acclimation period of 9 days, including the day of receipt. During the above period, the clinical symptoms of the animals were observed once a day. In addition, on the last day of the acclimation period, the animals were weighed and the health status was measured. It was confirmed that there were no abnormal clinical signs or weight gain in any of the animals. Body weights on the last day of the acclimation (that is, at the beginning of exposure to cigarette smoke) were about 390 to 450 g.

### (Preparation of Experimental Animals (Exposure to Smoking))

Experimental animals were prepared with reference to Non Patent Literature 2. Specifically, guinea pigs exposed to cigarette smoke using a smoking exposure system (INH06-CIG01A, INH06-CIG02, M.I.P.S. Inc.) were used in the experiment. Each guinea pig was placed in an exposure holder (one per cage), and the holder was secured in the exposure chamber. The animals were exposed to the smoke of 30 cigarette per day, 5 days a week, for 4 weeks, the smoke being generated by a mainstream smoke generator. In the fourth week, the exposure was for 4 days (smoking period was from Day 1 to Day 25). As for the cigarettes, Peace (no filter, tar: 28 mg, nicotine: 2.3 mg, Japan Tobacco Inc., Peace is a registered trademark of Japan Tobacco Inc.) was used.

### (Time of Measurement)

Unless otherwise specified, various evaluations were performed on the day after the last cigarette smoke-exposure in the second week (Day 13) and the day after the last cigarette smoke-exposure (Day 26).

### (Grouping and Drugs Used)

The guinea pigs were stratified and randomly assigned to 5 groups as shown in Table 30, and each guinea pig was assigned an animal number within the group to conduct the experiment.

As for the drug, Compound A and Gefapixant, which has been reported to inhibit P2X3 receptors, were used.

The solvent of Compound A was used as the placebo of the control group.

Note that, in the present specification, the low-dose group, medium-dose group, and high-dose group of Compound A may be collectively referred to as the "Compound A groups".

**[Table 30]**

| Group | Dose (mg/kg) | Number of Animals |
|---|---|---|
| 1 Control (placebo) | 0 | 8 |
| 2 Compound A-low-dose group | 3 | 8 |
| 3 Compound A-medium-dose group | 10 | 8 |
| 4 Compound A-high-dose group | 30 | 8 |
| 5 Gefapixant | 10 | 8 |

### (Drug Administration)

The placebo and each drug were administered by forced oral administration into the stomach using a disposable syringe equipped with a gastric catheter. As for the administration frequency, repetitive administration was performed once a day for 25 days from Day 1 to Day 25. The administration time was 1 hour (± 5 minutes) before the cigarette smoke-exposure. The dose was 5 mL/kg. The actual amount of each drug administered to each guinea pig was calculated based on the most recent weight.

Example 2 (Measurement of Respiratory Function)

### (Measurement Method)

Respiratory function in conscious guinea pigs was measured by double flow plethysmography using a respiratory function measurement system (Pulmos-I, M.I.P.S Inc.). The measurement parameters were specific airway resistance (sRaw), tidal volume (TV), respiratory rate (RR), minute volume (MV), and peak expiratory flow (PEF). The average value of the measurement values obtained with 100 breaths was considered as the measurement value for each measurement day.

### (Specific Airway Resistance (sRaw))

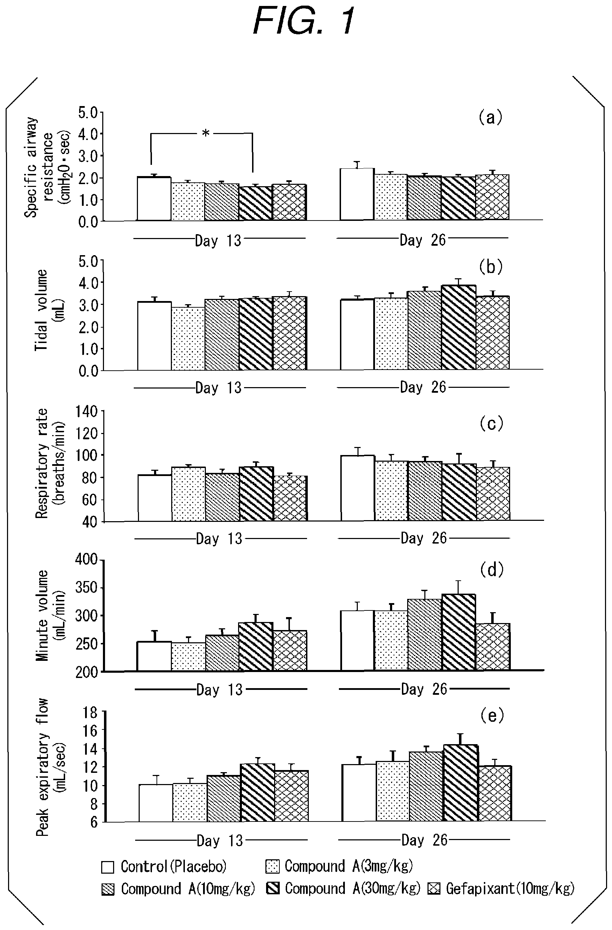

(a) of Fig. 1 shows the results of measuring the specific airway resistance. In the control (placebo) group, sRaws on Day 13 and Day 26 were 2.021 and 2.393 cmH₂O·sec, respectively. In the low-dose group, the medium-dose group, and the high-dose group of Compound A, sRaws on Day 13 were 1.755, 1.711, and 1.556 cmH₂O·sec, respectively, and sRaws on Day 26 were 2.105, 2.017, and 1.994 cmH₂O·sec, respectively, which decreased in a dose-dependent manner. Furthermore, a significant decrease was observed in the high-dose group on Day 13 compared to the control (placebo) group. In the Gefapixant group, sRaws on the Day 13 and Day 26 were 1.675 and 2.066 cmH₂O·sec, respectively, and there was no significant difference compared to the control (placebo) group.

### (Tidal Volume (TV))

(b) of Fig. 1 shows the results of measuring the specific tidal volume. In the control (placebo) group, TVs on Day 13 and Day 26 were 3.097 mL and 3.172 mL, respectively. In the Compound A groups, TVs showed a tendency of increasing in a dose-dependent manner.

### (Respiratory Rate (RR))

(c) of Fig. 1 shows the results of measuring the respiratory rate per 1 minute. In the control (placebo) group, RRs on Day 13 and Day 26 were 82.0 and 98.9 breaths/min, respectively. In the Compound A groups, RRs on Day 26 showed a tendency of decreasing in a dose-dependent manner.

### (Minute Volume (MV))

(d) of Fig. 1 shows the results for the minute volume ("tidal volume" × "respiratory rate per 1 minute"). In the control (placebo) group, MVs on Day 13 and Day 26 were 252.7 and 307.5 mL/min, respectively. In the Compound A groups, MVs showed a tendency of increasing in a dose-dependent manner, and the values of MV in the medium-dose group and the high-dose group were shown to be higher than those in the control (placebo) group.

### (Peak Expiratory Flow (PEF))

(e) of Fig. 1 shows the results of measuring the peak expiratory flow. In the control (placebo) group, PEFs on Day 13 and Day 26 were 10.08 and 12.16 mL/sec, respectively. In the Compound A groups, PEFs showed the tendency of increasing in a dose-dependent manner, and the values of PEF in all Compound A groups were shown to be higher than those in the control (placebo) group.

As a result of Example 2 above, in the Compound A groups, sRaws and RRs decreased in a dose-dependent manner, and TVs, MVs, and PEFs increased in a dose-dependent manner. Therefore, the oral administration of Compound A for 25 days suppressed the dose-dependent increase in sRaws and RRs and the dose-dependent decrease in TVs, MVs, and PEFs in the cigarette smoke-exposed guinea pigs, thus improving the respiratory function.

On the other hand, Gefapixant at a dose of 10 mg/kg did not have a significant effect on the respiratory function. Gefapixant has been developed for the treatment of chronic cough, since it has been reported to inhibit P2X3 receptors (Non Patent Literature 3). However, in the cigarette smoke-exposed model, Gefapixant had no significant effect on the respiratory function.

Example 3 (Measurement of Cough Reflex)

### (Measurement Day)

Measurements were performed 1 day or 2 days after the last cigarette smoke exposure during Week 3 (Day 20 or Day 21).

### (Time of Measurement)

The measurements were performed 2 hours after the administration of the placebo or various drugs.

### (Measurement Method)

The guinea pigs were placed in the inhalation chamber of the respiratory function measurement system (Pulmos-I, M.I.P.S. Co.). Cough reflex was induced by inhaling citric acid (lot no. M8K7523, manufactured by NACALAI TESQUE, INC.) for 3 minutes from the front side of the chamber using an ultrasonic nebulizer. The concentration of the citric acid was 0.1 mol/L. The response was observed for a total of 15 minutes, during the inhalation and for 12 minutes after the inhalation. The presence or absence of a cough reflex was determined from the sound during the cough reflex, the movement of the animal, and the respiratory function. Before the measurement on the day of the administration, the animals were placed in the chamber for 15 minutes or longer to acclimatize to the experimental environment. Note that the respiratory function (respiratory rate (RR), tidal volume (TV), peak expiratory flow (PEF), and minute volume (MV)) was measured immediately before the citric acid inhalation. The respiratory function was measured by the body side sensor of the respiratory function measurement system.

### (Results)

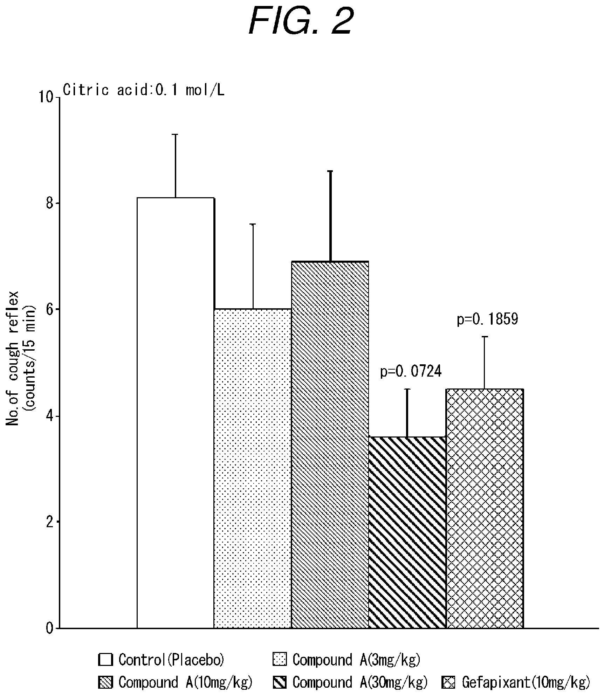

The results are shown in Fig. 2. In the control (placebo) group, the mean number of cough reflexes was 8.1. In the Compound A-low-dose group and medium-dose group, the mean numbers of cough reflexes were 6.0 and 6.9, respectively, and there was no significant difference from the control (placebo) group. Meanwhile, the mean number of cough reflexes in the high-dose group was 3.6, and a tendency of suppressing the cough reflex was observed, compared to the control (placebo) group. On the other hand, in the Gefapixant group, the mean number of cough reflexes was 4.5, and there was no significant difference between the Gefapixant group and the control (placebo) group.

As a result of Example 3 above, when Compound A was orally administered to the cigarette smoke-exposed guinea pigs for 25 days, a tendency of suppressing the number of cough reflexes in a dose-dependent manner was observed.

On the other hand, Gefapixant at a dose of 10 mg/kg did not have an effect on the cough reflex.

Example 4 (Bronchoalveolar Lavage Fluid (BALF) Collection and Number of Cells)

### (Time of Measurement)

The measurement was performed after the day after the last cigarette smoke exposure (Day 26).

### (Measurement Target)

### Right lung

### (Measurement Method)

After sacrificing the guinea pigs by bleeding under anesthesia, bronchoalveolar lavage fluid (BALF) was collected according to the following operating procedure.
(1) After ligating the left main bronchus with a thread, a cannula was inserted into the trachea and fixed.
(2) 5 mL of saline was injected into the right lung through the cannula, and the lavage fluid was collected by gentle suctioning. BALF was collected by repeating this procedure twice (a total of 10 mL).

(Measurement of Number of White Blood Cells (WBCs)) The pellet was suspended in 1 mL of saline, and the number of WBCs were counted using an automated hematology analyzer with a BALF analysis area (XT-2000iV, Sysmex Corporation). The counting was performed by adjusting the smear preparation. The smear preparation was performed by centrifuging the cell suspension again and suspending the obtained pellet in 75% FBS (fetal bovine serum and saline). After drying, the smear preparation was Wright-Giemsa stained.

### (Observation Target of Specimen)

The percentages of neutrophils, macrophages, eosinophil, and lymphocytes with respect to total WBCs were obtained, and the numbers of the cells per 1 µL were calculated.

### (WBCs)

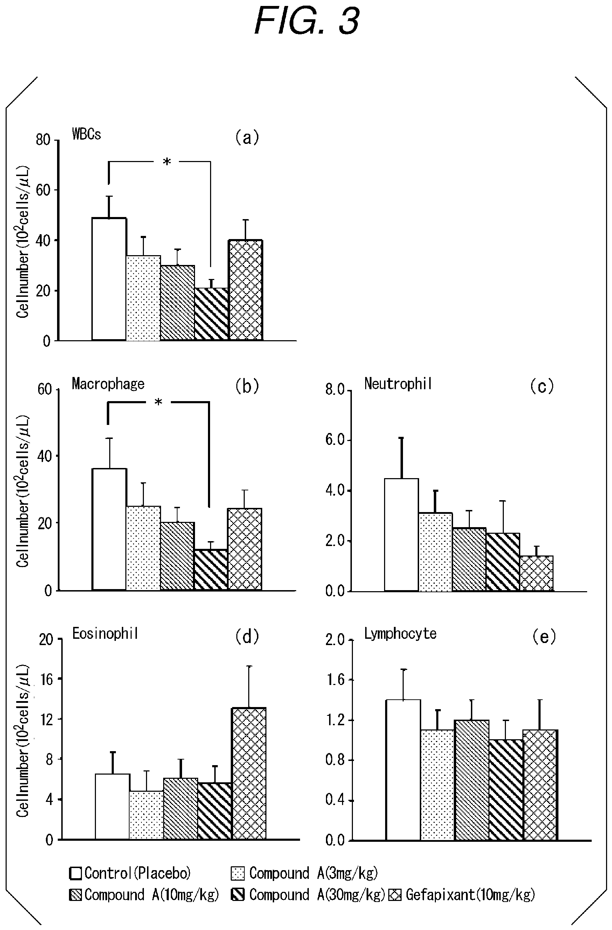

(a) of Fig. 3 shows the results of measuring WBCs. Control (placebo) group: the mean number of total WBCs was 48.7 × 10² cells/µL. The mean numbers of total WBCs in the Compound A-low-dose group, medium-dose group, and high-dose group were 33.9, 30.1, and 20.9 × 10² cells/µL, respectively, which were smaller than that in the control (placebo) group in a dose-dependent manner. The total number of WBCs in the high-dose group was significantly suppressed compared to the control (placebo) group. On the other hand, the mean number of total WBCs in the Gefapixant group was 40.0 × 10² cells/µL, and there was no significant different between the Gefapixant group and the control (placebo) group.

### (Macrophages)

(b) of Fig. 3 shows the results of measuring WBCs. In the control (placebo) group, the mean number of macrophages was 36.3 × 10² cells/µL. The mean numbers of macrophages in the Compound A groups were 25.0, 20.2, and 12.0 × 10² cells/µL, respectively, which were smaller than that in the control (placebo) group in a dose-dependent manner. The number of macrophages in the high-dose group was significantly suppressed compared to the control (placebo) group. On the other hand, the mean number of macrophages in the Gefapixant group was 24.4 × 10² cells/µL, and there was no significant different between the Gefapixant group and the control (placebo) group.

### (Neutrophils)

(c) of Fig. 3 shows the results of measuring neutrophils. In the control (placebo) group, the mean number of neutrophils was 4.5 × 10² cells/µL. The mean numbers of neutrophils in the Compound A groups were 3.1, 2.5, and 2.3 × 10² cells/µL, respectively, thus showing a tendency of decreasing in a dose-dependent manner.

### (Eosinophils)

(d) of Fig. 3 shows the results of measuring eosinophils. In the control (placebo) group, the mean number of eosinophils was 6.5 × 10² cells/µL. The mean numbers of eosinophils in the Compound A were 4.8, 6.1, and 5.6 × 10² cells/µL, respectively, and there was no significant difference from the control (placebo) group.

### (Lymphocytes)

(e) of Fig. 3 shows the results of measuring lymphocytes. In the control (placebo) group, the mean number of lymphocytes was 1.4 × 10² cells/µL. The mean numbers of lymphocytes in the Compound A groups were 1.1, 1.2, and 1.0 × 10² cells/µL, respectively, and there was no significant difference from the control (placebo) group.

From the results of Example 4 above, the numbers of WBCs, macrophages, and neutrophils in the Compound A groups decreased in a dose-dependent manner. In the Compound A-high-dose group, the numbers of WBCs and macrophages were significantly suppressed.

Based on these results, the oral administration of Compound A for 25 days suppressed the number of inflammatory cells in a dose-dependent manner in cigarette smoke-exposed guinea pigs.

On the other hand, Gefapixant at a dose of 10 mg/kg did not have an effect on the changes in the number of cells.

### Example 5 (Measurement of Weights)

### (Time and Method of Measurement)

The weights were measured with an electronic balance (MSA4202S, Sartorius Japan K.K.) before the research operation on Days 1, 8, 15, 22, and 26.

### (Results)

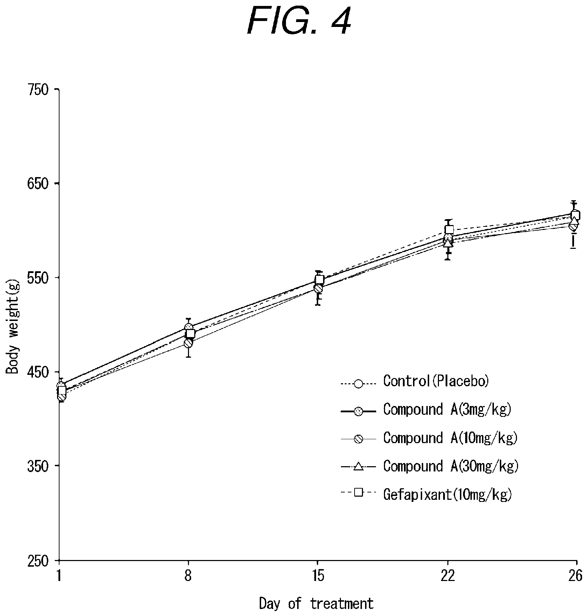

The results are shown in Fig. 4.

In the control (placebo) group, the weight increased every day from 429 g on Day 1 to 605 g on Day 26. The weight changes in the Compound A groups and the gefapixant group were comparable to that in the control (placebo) group.

In Examples of the present specification, the effects of Compound A on respiratory function, cough reflex, and the changes in the number of cells in the cigarette smoke-exposed model obtained using guinea pigs was studied by comparing with those of gefapixant.

In the control (placebo) group, sRaw, which is an indicator of airway contraction and airway obstruction, increased, and TV and PEF, which are indicators of gas exchange capacity, decreased compared to those in normal animals. As for the number of cells in BALF, the numbers of WBCs, neutrophils, and macrophages increased compared to those in the normal animals. Furthermore, the mean number of cough reflexes induced by 0.1 mol/L citric acid increased compared to that in normal animals. Such a result suggests that hypersensitivity of cough reflex was induced by the exposure to the cigarette smoke.

When Compound A was orally administered for 25 days in the Compound A groups, sRaws, the numbers of inflammatory cells in BALF (WBCs, macrophages, and neutrophils), and the numbers of cough reflexes decreased in a dose-dependent manner, and TV, MV, and PEF increased in a dose-dependent manner. Particularly, in the high-dose group, sRaw and the numbers of WBCs and macrophages on Day 13 were significantly suppressed, and a tendency of suppressing the number of cough reflexes was observed, compared to the control (placebo) group. From these results, it was confirmed that, due to the administration of Compound A, depression of respiratory function, airflow obstruction, and inflammation in the lung were improved, and it was suggested that alveoli expanded due to increase in FRC and RV.

On the other hand, in the Gefapixant group, there was no significant effect on the respiratory function, cough reflex, and the changes in the number of cells. Gefapixant group has been reported to inhibit P2X3 receptors and developed for the treatment of chronic cough. However, in the cigarette smoke-exposed model, Gefapixant did not have an effect on cough reflex or other changes.

### Industrial Applicability

The pharmaceutical composition of the present invention can be used for preventing or treating respiratory disease, particularly, inflammatory respiratory disease.

## Claims

1. A pharmaceutical composition for preventing or treating respiratory disease, the pharmaceutical composition comprising: a compound having a P2X4 receptor antagonistic action or a pharmaceutically acceptable salt thereof as an active ingredient.

2. The pharmaceutical composition according to claim 1, wherein the compound is a compound represented by the following general formula (A): 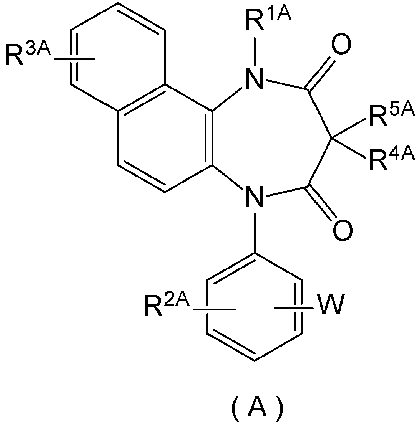
(in the formula, R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group,
R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group,
R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms substituted with a phenyl group, and
W^{A} represents a five or six membered heterocyclic ring including 1 to 4 nitrogen atoms as the members of the ring, which may have a substituent).

3. The pharmaceutical composition according to claim 1, wherein the compound is a compound represented by the following general formula (BI): 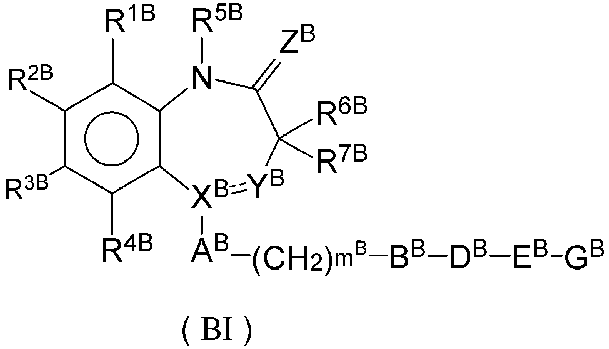
(in the formula, R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with a benzene ring to which they bind, and the ring constituted by R^{1B} and R^{2B} bound to each other, together with carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
X^{B} represents C, CH, or N,
Y^{B} represents N, NH, or C(=O),
provided that when X^{B} is N, Y^{B} is not N or NH, and when X^{B} is C or CH, Y^{B} is not C(=O),
the double line consisting of the solid line and the broken line represents a single bond or a double bond,
Z^{B} represents an oxygen atom or a sulfur atom,
A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
B^{B} represents N(R^{8B})C(=O), NHCONH, CON(R^{9B}), NHC(=S)NH, N(R^{10B})SO₂, SO₂N(R^{11B}), or OSO₂, wherein
R^{8B}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
E^{B} represents O, S, NR^{12B}, or an atomic bond, wherein
R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
m^{B} represents an integer of 0 to 5,
provided that when R^{1B} and R^{2B} do not bind together to form a ring, those compounds are excluded wherein, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).

4. The pharmaceutical composition according to claim 1, wherein the compound or a pharmaceutically acceptable salt thereof is a compound or a pharmaceutically acceptable salt thereof selected from the group consisting of the following (A1) to (A21) and (B1) to (B214):
(A1) 5-[3-(1H-tetrazol-5-yl)phenyl]-1H-naphtho[1,2-b][1,4]diazepine-2,4(3H,5H)-dione;
(A2) 5-[3-(1H-tetrazol-5-yl)phenyl]-1H-naphtho[1,2-b][1,4]diazepine-2,4(3H,5H)-dione sodium salt;
(A3) 5-[3-(1H-tetrazol-5-yl)phenyl]-1H-naphtho[1,2-b][1,4]diazepine-2,4(3H,5H)-dione potassium salt;
(A4) 5-[4-(1H-tetrazol-5-yl)phenyl]-1H-naphtho[1,2-b][1,4]diazepine-2,4(3H,5H)-dione;
(A5) 5-[4-(1H-tetrazol-5-yl)phenyl]-1H-naphtho[1,2-b][1,4]diazepine-2,4(3H,5H)-dione sodium salt;
(A6) 1-methyl-5-[3-(1H-tetrazol-5-yl)phenyl]-1H-naphtho[1,2-b][1,4]diazepine-2,4(3H,5H)-dione;
(A7) 1,3-dimethyl-5-[3-(1H-tetrazol-5-yl)phenyl]-1H-naphtho[1,2-b][1,4]diazepine-2,4(3H,5H)-dione;
(A8) 5-[2-chloro-5-(1H-tetrazol-5-yl)phenyl]-1H-naphtho[1,2-b][1,4]diazepine-2,4(3H,5H)-dione;
(A9) 5-[2-chloro-5-(1H-tetrazol-5-yl)phenyl]-1H-naphtho[1,2-b][1,4]diazepine-2,4(3H,5H)-dione sodium salt;
(A10) 5-[2-methyl-5-(1H-tetrazol-5-yl)phenyl]-1H-naphtho[1,2-b][1,4]diazepine-2,4(3H,5H)-dione;
(A11) 5-[2-methyl-5-(1H-tetrazol-5-yl)phenyl]-1H-naphtho[1,2-b][1,4]diazepine-2,4(3H,5H)-dione sodium salt;
(A12) 5-[2-bromo-5-(1H-tetrazol-5-yl)phenyl]-1H-naphtho[1,2-b][1,4]diazepine-2,4(3H,5H)-dione;
(A13) 5-[3-(2-methyl-2H-tetrazol-5-yl)phenyl]-1H-naphtho[1,2-b][1,4]diazepine-2,4(3H,5H)-dione;
(A14) 5-[3-(1-methyl-1H-tetrazol-5-yl)phenyl]-1H-naphtho[1,2-b][1,4]diazepine-2,4(3H,5H)-dione;
(A15) 5-[3-(5-oxo-4H-[1,2,4]oxadiazol-3-yl)phenyl]-1H-naphtho[1,2-b][1,4]diazepine-2,4(3H,5H)-dione;
(A16) 5-[3-(5-thioxo-4H-[1,2,4]oxadiazol-3-yl)phenyl]-1H-naphtho[1,2-b][1,4]diazepine-2,4(3H,5H)-dione;
(A17) 5-[3-(5-thioxo-4H-[1,2,4]oxadiazol-3-yl)phenyl]-1H-naphtho[1,2-b][1,4]diazepine-2,4(3H,5H)-dione sodium salt;
(A18) 5-[3-(oxazol-2-yl)phenyl]-1H-naphtho[1,2-b][1,4]diazepine-2,4(3H,5H)-dione; and
(A19) 5-[3-(1H-pyrazol-4-yl)phenyl]-1H-naphtho[1,2-b][1,4]diazepine-2,4(3H,5H)-dione;
(A20) 5-[4-fluoro-3-(1H-tetrazol-5-yl)phenyl]-1H-naphtho[1,2-b][1,4]diazepine-2,4(3H,SH)-dione;
(A21) 5-[4-fluoro-3-(1H-tetrazol-5-yl)phenyl]-1H-naphtho[1,2-b][1,4]diazepine-2,4(3H,5H)-dione sodium salt;
(B1) 5-(4-benzoylaminophenyl)-1H-naphtho[1,2-b][1,4]diazepine-2,4(3H,5H)-dione;
(B2) 5-[4-[2-(trifluoromethyl)benzoyl]aminophenyl]-1H-naphtho[1,2-b][1,4]diazepine-2,4(3H,5H)-dione;
(B3) 5-[4-(3-bromobenzoyl)aminophenyl]-1H-naphtho[1,2-b][1,4]diazepine-2,4(3H,5H)-dione;
(B4) 5-[4-[4-(trifluoromethyl)benzoyl]aminophenyl]-1H-naphtho[1,2-b][1,4]diazepine-2,4(3H,5H)-dione;
(B5) 5-[4-(2-methylbenzoyl)aminophenyl]-1H-naphtho[1,2-b][1,4]diazepine-2,4(3H,5H)-dione;
(B6) 5-[4-(2,6-dimethylbenzoylamino)phenyl]-1H-naphtho[1,2-b][1,4]diazepine-2,4(3H,5H)-dione;
(B7) 5-[4-(2,6-dichlorobenzoyl)aminophenyl]-1H-naphtho[1,2-b][1,4]diazepine-2,4(3H,5H)-dione;
(B8) 5-[4-(3-chlorobenzoyl)aminophenyl]-1H-naphtho[1,2-b][1,4]diazepine-2,4(3H,5H)-dione;
(B9) 5[4-(2-phenylacetylamino)phenyl]-1H-naphtho[1,2-b][1,4]diazepine-2,4(3H,5H)-dione;
(B10) 1-[4-(2,4-dioxo-1,2,3,4-tetrahydronaphtho[1,2-b][1,4]diazepin-5-yl)phenyl]-3-phenylthiourea;
(B11) 5-[4-(2,3-dimethoxybenzoylamino)phenyl]-1H-naphtho[1,2-b][1,4]diazepine-2,4(3H,5H)-dione;
(B12) 5-[4-(2-methoxybenzoylamino)phenyl]-1H-naphtho[1,2-b][1,4]diazepine-2,4(3H,5H)-dione;
(B13) 5-[4-[(2-chlorophenylacetyl)amino]phenyl]-1H-naphtho[1,2-b][1,4]diazepine-2,4(3H,5H)-dione;
(B14) 5-[4-(2,3-dimethylbenzoylamino)phenyl]-1H-naphtho[1,2-b][1,4]diazepine-2,4(3H,5H)-dione;
(B15) 5-[4-(2,5-dimethylbenzoylamino)phenyl]-1H-naphtho[1,2-b][1,4]diazepine-2,4(3H,5H)-dione;
(B16) 5-[4-(5-bromo-2-chlorobenzoylamino)phenyl]-1H-naphtho[1,2-b][1,4]diazepine-2,4(3H,5H)-dione;
(B17) 5-[4-(2,4-dichlorobenzoylamino)phenyl]-1H-naphtho[1,2-b][1,4]diazepine-2,4(3H,5H)-dione;
(B18) 5-[4-(2-hydroxybenzoylamino)phenyl]-1H-naphtho[1,2-b][1,4]diazepine-2,4(3H,5H)-dione;
(B19) 5-[4-(2,3-dihydroxybenzoylamino)phenyl]-1H-naphtho[1,2-b][1,4]diazepine-2,4(3H,5H)-dione;
(B20) 1-[4-(2,4-dioxo-1,2,3,4-tetrahydronaphtho[1,2-b][1,4]diazepin-5-yl)phenyl]-3-phenylurea;
(B21) 5-[4-[(2,6-dichlorophenylacetyl)amino]phenyl]-1H-naphtho[1,2-b][1,4]diazepine-2,4(3H,5H)-dione;
(B22) 5-[4-[(2-methoxyphenylacetyl)amino]phenyl]-1H-naphtho[1,2-b][1,4]diazepine-2,4(3H,5H)-dione;
(B23) 5-[4-[(2-hydroxyphenylacetyl)amino]phenyl]-1H-naphtho[1,2-b][1,4]diazepine-2,4(3H,5H)-dione;
(B24) 1-(2-chlorophenyl)-3-[4-(2,4-dioxo-1,2,3,4-tetrahydronaphtho[1,2-b][1,4]diazepin-5-yl)phenyl]thiourea;
(B25) 5-[4-[3-(trifluoromethyl)benzoylamino]phenyl]-1H-naphtho[1,2-b][1,4]diazepine-2,4(3H,5H)-dione;
(B26) 5-[4-[2-[2-(trifluoromethyl)phenyl]acetylamino]phenyl]-1H-naphtho[1,2-b][1,4]diazepine-2,4(3H,5H)-dione;
(B27) 1-(2-chlorophenyl)-3-[4-(2,4-dioxo-1,2,3,4-tetrahydronaphtho[1,2-b][1,4]diazepin-5-yl)phenyl]urea;
(B28) 5-[4-[(2-phenylpropionyl)amino]phenyl]-1H-naphtho[1,2-b][1,4]diazepine-2,4(3H,5H)-dione;
(B29) 5-[4-(2-chloro-3-methoxybenzoylamino)phenyl]-1H-naphtho[1,2-b] [1,4]diazepine-2,4(3H,5H)-dione;
(B30) 5-[4-(3-phenylpropionylamino)phenyl]-1H-naphtho[1,2-b] [1,4]diazepine-2,4(3H,5H)-dione;
(B31) 5-[4-[(1H-indole-3-carbonyl)amino]phenyl]-1H-naphtho[1,2-b] [1,4]diazepine-2,4(3H,5H)-dione;
(B32) 5-[4-(2-chloro-3-hydroxybenzoylamino)phenyl]-1H-naphtho[1,2-b] [1,4]diazepine-2,4(3H,5H)-dione;
(B33) 5-[4-[(2-methyl-2-phenylpropionyl)amino]phenyl]-1H-naphtho[1,2-b][1,4]diazepine-2,4(3H,5H)-dione;
(B34) 5-[4-(2-phenoxyacetylamino)phenyl]-1H-naphtho[1,2-b] [1,4]diazepine-2,4(3H,5H)-dione;
(B35) 5-[4-[2-(2-chloro-4-methoxyphenyl)acetylamino]phenyl]-1H-naphtho[1,2-b][1,4]diazepine-2,4(3H,5H)-dione;
(B36) 5-[4-[(1-methyl-1H-imidazole-2-carbonyl)amino]phenyl]-1H-naphtho[1,2-b] [1,4]diazepine-2,4(3H,5H)-dione;
(B37) 5-[4-[2-(2,4-dichlorophenyl)acetylamino]phenyl]-1H-naphtho[1,2-b][1,4]diazepine-2,4(3H,5H)-dione;
(B38) 5-[4-[2-(2-chloro-4-hydroxyphenyl)acetylamino]phenyl]-1H-naphtho[1,2-b][1,4]diazepine-2,4(3H,5H)-dione;
(B39) 5-[4-(3-phenylpropenylamino)phenyl]-1H-naphtho[1,2-b][1,4]diazepine-2,4(3H,5H)-dione;
(B40) 5-[4-[(3-pyridylacetyl)amino]phenyl]-1H-naphtho[1,2-b][1,4]diazepine-2,4(3H,5H)-dione hydrochloride;
(B41) 5-[4-(1H-benzimidazole-2-carbonylamino)phenyl]-1H-naphtho[1,2-b][1,4]diazepine-2,4(3H,5H)-dione;
(B42) 1-[4-(2,3-dimethylbenzoylamino)phenyl]-7-methoxy-1H-1,5-benzodiazepine-2,4(3H,5H)-dione;
(B43) 5-[4-[(benzoylamino)methyl]phenyl]-1H-naphtho[1,2-b][1,4]diazepine-2,4(3H,5H)-dione;
(B44) 5-[4-[(2-chlorobenzoylamino)methyl]phenyl]-1H-naphtho[1,2-b][1,4]diazepine-2,4(3H,5H)-dione;
(B45) 1-[4-(2,3-dimethylbenzoylamino)phenyl]-7-hydroxy-1H-1,5-benzodiazepine-2,4(3H,5H)-dione;
(B46) 5-[4-(2-chlorobenzoylamino)phenyl]-1H-naphtho[1,2-b][1,4]diazepine-2,4(3H,5H)-dione;
(B47) 5-[4-(2-bromobenzoylamino)phenyl]-1H-naphtho[1,2-b][1,4]diazepine-2,4(3H,5H)-dione;
(B48) 5-[4-(2-iodobenzoylamino)phenyl]-1H-naphtho[1,2-b][1,4]diazepine-2,4(3H,5H)-dione;
(B49) 5-[4-(2,3-dimethylbenzoylamino)-3-fluorophenyl]-1H-naphtho[1,2-b][1,4]diazepine-2,4(3H,5H)-dione;
(B50) 5-[4-[2-(2-methylphenyl)acetylamino]phenyl]-1H-naphtho[1,2-b][1,4]diazepine-2,4(3H,5H)-dione;
(B51) 5-[4-[(quinoxalin-2-yl)carbonylamino]phenyl]-1H-naphtho[1,2-b][1,4]diazepine-2,4(3H,5H)-dione;
(B52) 5-[4-[(5-methylthiophen-2-yl)carbonylamino]phenyl]-1H-naphtho[1,2-b][1,4]diazepine-2,4(3H,5H)-dione;
(B53) 5-[3-[(2-chlorophenylacetyl)amino]phenyl]-1H-naphtho[1,2-b][1,4]diazepine-2,4(3H,5H)-dione;
(B54) 5-[4-[(2,4,6-trimethylbenzoyl)amino]phenyl]-1H-naphtho[1,2-b][1,4]diazepine-2,4(3H,5H)-dione;
(B55) 5-[4-(cyclohexylcarbonylamino)phenyl]-1,2,3,4,8,9,10,11-octahydronaphtho[1,2-b][1,4]diazepine-2,4(3H,5H)-dione;
(B56) 1-[4-(2,3-dimethylbenzoyl)aminophenyl]-6-methyl-1H-1,5-benzodiazepine-2,4(3H,5H)-dione;
(B57) 5-[4-[(2-ethylbenzoyl)amino]phenyl]-1H-naphtho[1,2-b][1,4]diazepine-2,4(3H,5H)-dione;
(B58) 5-[4-[(6-methylpyridin-2-yl)carbonylamino]phenyl]-1,2,3,4,8,9,10,11-octahydronaphtho[1,2-b][1,4]diazepine-2,4(3H,5H)-dione;
(B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
(B60) 1-[4-(2,4-dioxo-1,2,3,4-tetrahydronaphtho[1,2-b][1,4]diazepin-5-yl)phenyl]-3-(2-methylphenyl)thiourea;
(B61) 5-[4-(2-methoxy-3-methylbenzoyl)aminophenyl]-1,2,3,4,8,9,10,11-octahydronaphtho[1,2-b][1,4]diazepine-2,4(3H,5H)-dione;
(B62) 5-[4-(2,3-dichlorobenzoyl)aminophenyl]-1,2,3,4,8,9,10,11-octahydronaphtho[1,2-b][1,4]diazepine-2,4(3H,5H)-dione;
(B63) 5-[4-(2,3-dimethylbenzoylamino)-3-hydroxyphenyl]-1H-naphtho[1,2-b][1,4]diazepine-2,4(3H,5H)-dione;
(B64) 5-[4-(2-chloro-3-methoxybenzoylamino)phenyl]-1,3-dihydronaphtho[1,2-e]-1,4-diazepin-2-one;
(B65) 5-[4-[(4-dimethylaminobenzoyl)amino]phenyl]-1H-naphtho[1,2-b][1,4]diazepine-2,4(3H,5H)-dione;
(B66) 5-[4-[2-(2,4-dichlorophenoxy)acetylamino]phenyl]-1H-naphtho[1,2-b][1,4]diazepine-2,4(3H,5H)-dione;
(B67) 5-[4-[2-(2-methylphenoxy)acetylamino]phenyl]-1H-naphtho[1,2-b][1,4]diazepine-2,4(3H,5H)-dione;
(B68) N-[4-(2,4-dioxo-1,2,3,4-tetrahydronaphtho[1,2-b][1,4]diazepin-5-yl)butyl]-2-chloro-3-methoxybenzamide;
(B69) 5-[4-(2-chloro-3-hydroxybenzoylamino)phenyl]-1,3-dihydronaphtho[1,2-e]-1,4-diazepin-2-one;
(B70) 5-[4-(2-acetylbenzoylamino)phenyl]-1H-naphtho[1,2-b][1,4]diazepine-2,4(3H,5H)-dione;
(B71) 5-[4-(2-tert-butylbenzoylamino)phenyl]-1H-naphtho[1,2-b][1,4]diazepine-2,4(3H,5H)-dione;
(B72) 5-[2-(2-iodobenzoyl)aminoethyl]-1H-naphtho[1,2-b][1,4]diazepine-2,4(3H,5H)-dione;
(B73) 5-[3-[(2-iodobenzoyl)amino]phenyl]-1H-naphtho[1,2-b][1,4]diazepine-2,4(3H,5H)-dione;
(B74) 6,7-dimethyl-1-[4-(2-iodobenzoyl)aminophenyl]-1H-1,5-benzodiazepine-2,4(3H,5H)-dione;
(B75) 5-[4-[(1-methylpiperidin-4-yl)carbonylamino]phenyl]-1H-naphtho[1,2-b][1,4]diazepine-2,4(3H,5H)-dione hydrochloride;
(B76) 5[4-[(benzofuran-2-yl)carbonylamino]phenyl]-1H-naphtho[1,2-b][1,4]diazepine-2,4(3H,5H)-dione;
(B77) 5-[4-[(1-methyl-1H-indol-3-yl)carbonylamino]phenyl]-1H-naphtho[1,2-b][1,4]diazepine-2,4(3H,5H)-dione;
(B78) 5-[4-(2-propenylbenzoylamino)phenyl]-1H-naphtho[1,2-b][1,4]diazepine-2,4(3H,5H)-dione;
(B79) 5-[4-(2-propylbenzoylamino)phenyl]-1H-naphtho[1,2-b][1,4]diazepine-2,4(3H,5H)-dione;
(B80) 5-[3-fluoro-4-(2-iodobenzoylamino)phenyl]-1H-naphtho[1,2-b][1,4]diazepine-2,4(3H,5H)-dione;
(B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
(B82) 5-[4-[(2-isopropoxybenzoyl)amino]phenyl]-1H-naphtho[1,2-b][1,4]diazepine-2,4(3H,5H)-dione;
(B83) 5-[4-[(3-methylthiophen-2-yl)carbonylamino]phenyl]-1H-naphtho[1,2-b][1,4]diazepine-2,4(3H,5H)-dione;
(B84) 5-[4-(2-phenoxypropionylamino)phenyl]-1H-naphtho[1,2-b][1,4]diazepine-2,4(3H,5H)-dione;
(B85) 5-[4-[2-(4-chloro-2-methylphenoxy)acetylamino]phenyl]-1H-naphtho[1,2-b][1,4]diazepine-2,4(3H,5H)-dione;
(B86) 5-[4-[(4-fluoro-2-trifluoromethyl)benzoyl]aminophenyl]-1H-naphtho[1,2-b][1,4]diazepine-2,4(3H,5H)-dione;
(B87) 5-[4-(4-fluoro-2-methoxybenzoyl)aminophenyl]-1H-naphtho[1,2-b][1,4]diazepine-2,4(3H,5H)-dione;
(B88) 5-[4-(4-fluoro-2-hydroxybenzoyl)aminophenyl]-1H-naphtho[1,2-b][1,4]diazepine-2,4(3H,5H)-dione;
(B89) 5-[3-[(2-iodophenylacetyl)amino]phenyl]-1H-naphtho[1,2-b][1,4]diazepine-2,4(3H,5H)-dione;
(B90) 5-[4-(2-methyl-2-phenoxypropionylamino)phenyl]-1H-naphtho[1,2-b][1,4]diazepine-2,4(3H,5H)-dione;
(B91) 5-[4-(2-tert-butylbenzoylamino)phenyl]-1,3-dihydronaphtho[1,2-e]-1,4-diazepin-2-one;
(B92) 5-[4-[(3-dimethylaminobenzoyl)amino]phenyl]-1H-naphtho[1,2-b][1,4]diazepine-2,4(3H,5H)-dione;
(B93) 5-[4-(4-iodo-2-methoxybenzoylamino)phenyl]-1H-naphtho[1,2-b][1,4]diazepine-2,4(3H,5H)-dione;
(B94) 5-[4-(6-fluoro-2-methoxybenzoylamino)phenyl]-1H-naphtho[1,2-b][1,4]diazepine-2,4(3H,5H)-dione;
(B95) 5-[4-(2-hydroxy-4-iodobenzoylamino)phenyl]-1H-naphtho[1,2-b][1,4]diazepine-2,4(3H,5H)-dione;
(B96) 5-[4-(6-fluoro-2-hydroxybenzoylamino)phenyl]-1H-naphtho[1,2-b][1,4]diazepine-2,4(3H,5H)-dione;
(B97) 5-[4-(2-fluorobenzoyl)aminophenyl]-1H-naphtho[1,2-b][1,4]diazepine-2,4(3H,5H)-dione;
(B98) 5-[4-[(2-dimethylaminobenzoyl)amino]phenyl]-1H-naphtho[1,2-b][1,4]diazepine-2,4(3H,5H)-dione;
(B99) 5-[4-(2-methoxy-6-methylbenzoylamino)phenyl]-1H-naphtho[1,2-b][1,4]diazepine-2,4(3H,5H)-dione;
(B100) 5-[4-(2-hydroxy-6-methylbenzoylamino)phenyl]-1H-naphtho[1,2-b][1,4]diazepine-2,4(3H,5H)-dione;
(B101) 5-[4-[3-(2-methylphenyl)propionylamino]phenyl]-1H-naphtho[1,2-b][1,4]diazepine-2,4(3H,5H)-dione;
(B102) 5-(4-phenylcarbamoylphenyl)-1H-naphtho[1,2-b][1,4]diazepine-2,4(3H,5H)-dione;
(B103) 5-(4-benzylcarbamoylphenyl)-1H-naphtho[1,2-b][1,4]diazepine-2,4(3H,5H)-dione;
(B104) 5-[4-[3-(2-methylphenyl)propenoylamino]phenyl]-1H-naphtho[1,2-b][1,4]diazepine-2,4(3H,5H)-dione;
(B105) 5-[4-[3-(2-chlorophenyl)propionylamino]phenyl]-1H-naphtho[1,2-b][1,4]diazepine-2,4(3H,5H)-dione;
(B106) 5-[4-(2-iodobenzoyl)aminophenyl]-1,2,3,4,8,9,10,11-octahydronaphtho[1,2-b][1,4]diazepine-2,4(3H,5H)-dione;
(B107) 5-[4-[(1-methyl-1H-pyrrol-2-ylacetyl)amino]phenyl]-1,2,3,4,8,9,10,11-octahydronaphtho[1,2-b][1,4]diazepine-2,4(3H,5H)-dione;
(B108) 5-[4-(2-chlorobenzyl)carbamoylphenyl]-1H-naphtho[1,2-b][1,4]diazepine-2,4(3H,5H)-dione;
(B109) 5-[4-[3-(2-chlorophenyl)propenoylamino]phenyl]-1H-naphtho[1,2-b][1,4]diazepine-2,4(3H,5H)-dione;
(B110) 5-[4-(2-chlorophenyl)carbamoylphenyl]-1H-naphtho[1,2-b][1,4]diazepine-2,4(3H,5H)-dione;
(B111) 5-[4-(6-bromo-2,3-methylenedioxybenzoylamino)phenyl]-1H-naphtho[1,2-b][1,4]diazepine-2,4(3H,5H)-dione;
(B112) 5-[4-(6-bromo-2-methoxybenzoylamino)phenyl]-1H-naphtho[1,2-b][1,4]diazepine-2,4(3H,5H)-dione;
(B113) 5-[4-[(2-tert-butylbenzoyl)amino]phenyl]-1,2,3,4,8,9,10,11-octahydronaphtho[1,2-b][1,4]diazepine-2,4(3H,5H)-dione;
(B114) 5-[2-(2-iodobenzoyl)aminopyridin-5-yl]-1H-naphtho[1,2-b][1,4]diazepine-2,4(3H,5H)-dione;
(B115) 5-[4-(6-bromo-2-hydroxybenzoylamino)phenyl]-1H-naphtho[1,2-b][1,4]diazepine-2,4(3H,5H)-dione;
(B116) 5-[4-(6-chloro-2-methoxybenzoylamino)phenyl]-1H-naphtho[1,2-b][1,4]diazepine-2,4(3H,5H)-dione;
(B117) 5-[4-(2-iodobenzoylamino)phenyl]-1H-[1,4]diazepino[2,3-h]quinoline-2,4(3H,5H)-dione;
(B118) 5-[4-(6-chloro-2-hydroxybenzoylamino)phenyl]-1H-naphtho[1,2-b][1,4]diazepine-2,4(3H,5H)-dione;
(B119) 5-[4-(2-hydroxy-6-methoxybenzoylamino)phenyl]-1H-naphtho[1,2-b][1,4]diazepine-2,4(3H,5H)-dione;
(B120) 5-[4-[2-methoxy-6-(trifluoromethyl)benzoylamino]phenyl]-1H-naphtho[1,2-b][1,4]diazepine-2,4(3H,5H)-dione;
(B121) 5-[4-[2-hydroxy-6-(trifluoromethyl)benzoylamino]phenyl]-1H-naphtho[1,2-b][1,4]diazepine-2,4(3H,5H)-dione;
(B122) 5-[4-[(2-isopropenylbenzoyl)amino]phenyl]-1,2,3,4,8,9,10,11-octahydronaphtho[1,2-b][1,4]diazepine-2,4(3H,5H)-dione;
(B123) 5-[4-[(2-isopropylbenzoyl)amino]phenyl]-1,2,3,4,8,9,10,11-octahydronaphtho[1,2-b][1,4]diazepine-2,4(3H,5H)-dione;
(B124) 5-[4-[2-chloro-5-(methylthio)benzoylamino]phenyl]-1H-naphtho[1,2-b][1,4]diazepine-2,4(3H,5H)-dione;
(B125) 5-[4-[2-(methylthio)benzoylamino]phenyl]-1H-naphtho[1,2-b][1,4]diazepine-2,4(3H,5H)-dione;
(B126) 5-[4-[3-(methylthio)benzoylamino]phenyl]-1H-naphtho[1,2-b][1,4]diazepine-2,4(3H,5H)-dione;
(B127) 5-[4-[2-ethyl-6-methoxybenzoylamino]phenyl]-1H-naphtho[1,2-b][1,4]diazepine-2,4(3H,5H)-dione;
(B128) 5-[4-(3-methanesulfonylbenzoylamino)phenyl]-1H-naphtho[1,2-b][1,4]diazepine-2,4(3H,5H)-dione;
(B129) 6-ethyl-1-[4-(2-iodobenzoyl)aminophenyl]-1H-1,5-benzodiazepine-2,4(3H,5H)-dione;
(B130) 5-[4-[2-ethyl-6-hydroxybenzoylamino]phenyl]-1H-naphtho[1,2-b][1,4]diazepine-2,4(3H,5H)-dione;
(B131) 5-[4-(3-methanesulfinylbenzoylamino)phenyl]-1H-naphtho[1,2-b][1,4]diazepine-2,4(3H,5H)-dione;
(B132) 5-[4-(2-chloro-5-methanesulfinylbenzoylamino)phenyl]-1H-naphtho[1,2-b][1,4]diazepine-2,4(3H,5H)-dione;
(B133) 5-[4-(2-methanesulfinylbenzoylamino)phenyl]-1H-naphtho[1,2-b][1,4]diazepine-2,4(3H,5H)-dione;
(B134) 5-[4-[[2-(4-morpholinyl)acetyl]amino]phenyl]-1,2,3,4,8,9,10,11-octahydronaphtho[1,2-b][1,4]diazepine-2,4(3H,5H)-dione hydrochloride;
(B135) 5-[4-(2-chloro-6-methoxybenzoylamino)phenyl]-1,3-dihydronaphtho[1,2-e]-1,4-diazepin-2-one;
(B136) 5-[4-[[(3-chloropyridin-2-yl)carbonyl]amino]phenyl]-1H-naphtho[1,2-b][1,4]diazepine-2,4(3H,5H)-dione;
(B137) 5-[4-(2-chloro-6-hydroxybenzoylamino)phenyl]-1,3-dihydronaphtho[1,2-e]-1,4-diazepin-2-one;
(B138) 5-[4-(3-chloro-2-methoxybenzoylamino)phenyl]-1H-naphtho[1,2-b][1,4]diazepine-2,4(3H,5H)-dione;
(B139) 5-[4-[(3-methylpyridin-2-yl)carbonylamino]phenyl]-1H-naphtho[1,2-b][1,4]diazepine-2,4(3H,5H)-dione;
(B140) 5-[4-[[(3-chloropyridin-2-yl)carbonyl]amino]phenyl]-1,2,3,4,8,9,10,11-octahydronaphtho[1,2-b][1,4]diazepine-2,4(3H,5H)-dione;
(B141) 5-[4-(3-chloro-2-hydroxybenzoylamino)phenyl]-1H-naphtho[1,2-b][1,4]diazepine-2,4(3H,5H)-dione;
(B142) 5-[4-[[(3-hydroxypyridin-2-yl)carbonyl]amino]phenyl]-1H-naphtho[1,2-b][1,4]diazepine-2,4(3H,5H)-dione;
(B143) 5-[4-[(3-vinylpyridin-2-yl)carbonylamino]phenyl]-1H-naphtho[1,2-b][1,4]diazepine-2,4(3H,5H)-dione;
(B144) 5-[4-[(3-ethylpyridin-2-yl)carbonylamino]phenyl]-1H-naphtho[1,2-b][1,4]diazepine-2,4(3H,5H)-dione;
(B145) N-[4-(2,4-dioxo-1,2,3,4-tetrahydronaphtho-[1,2-b][1,4]-diazepin-5-yl)phenyl]-2-nitrobenzenesulfonamide;
(B146) N-[4-(2,4-dioxo-1,2,3,4-tetrahydronaphtho[1,2-b][1,4]diazepin-5-yl)phenyl]benzenesulfonamide;
(B147) 3-bromo-N-[4-(2,4-dioxo-1,2,3,4-tetrahydronaphtho[1,2-b][1,4]diazepin-5-yl)phenyl]benzenesulfonamide;
(B148) N-[4-(2,4-dioxo-1,2,3,4-tetrahydronaphtho[1,2-b][1,4]diazepin-5-yl)phenyl]-3-methoxybenzenesulfonamide;
(B149) N-[3-(2-oxo-2,3-dihydro-1H-naphtho[1,2-e][1,4]diazepin-5-yl)phenyl]benzenesulfonamide;
(B150) N-[3-(2,4-dioxo-1,2,3,4-tetrahydronaphtho-[1,2-b][1,4]-diazepin-5-yl)phenyl]-2-nitrobenzenesulfonamide;
(B151) N-[4-(2,4-dioxo-1,2,3,4,8,9,10,11-octahydro-naphtho[1,2-b][1,4]-diazepin-5-yl)phenyl]-2-nitrobenzenesulfonamide;
(B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
(B153) N-[3-(2,4-dioxo-1,2,3,4-tetrahydronaphtho-[1,2-b][1,4]-diazepin-5-yl)phenyl]-N-methyl-2-nitrobenzenesulfonamide;
(B154) 4-(2,4-dioxo-1,2,3,4,8,9,10,11-octahydronaphtho[1,2-b][1,4]diazepin-5-yl)-N-phenylbenzenesulfonamide;
(B155) N-[3-(2,4-dioxo-1,2,3,4-tetrahydronaphtho[1,2-b]-[1,4]diazepin-5-yl)phenyl]-2-naphthalenesulfonamide;
(B156) N-[3-(2,4-dioxo-1,2,3,4-tetrahydronaphtho[1,2-b]-[1,4]diazepin-5-yl)phenyl]-1-naphthalenesulfonamide;
(B157) N-[4-(2,4-dioxo-1,2,3,4-tetrahydronaphtho[1,2-b][1,4]diazepin-5-yl)phenyl]cyclohexanesulfonamide;
(B158) N-[4-(2,4-dioxo-1,2,3,4-tetrahydronaphtho[1,2-b][1,4]diazepin-5-yl)phenyl]-3-pyridinesulfonamide hydrochloride;
(B159) N-[4-(2,4-dioxo-1,2,3,4-tetrahydronaphtho[1,2-b][1,4]diazepin-5-yl)phenyl]-4-isopropylbenzenesulfonamide;
(B160) N-[4-(2,4-dioxo-1,2,3,4-tetrahydronaphtho[1,2-b][1,4]diazepin-5-yl)phenyl]phenylmethanesulfonamide;
(B161) N-[4-(2,4-dioxo-1,2,3,4-tetrahydronaphtho[1,2-b][1,4]diazepin-5-yl)phenyl]-2-thiophene-sulfonamide;
(B162) N-[4-(2,4-dioxo-1,2,3,4-tetrahydronaphtho[1,2-b][1,4]diazepin-5-yl)phenyl]-2-naphthalenesulfonamide;
(B163) 4-(2,4-dioxo-1,2,3,4,8,9,10,11-octahydronaphtho-[1,2-b][1,4]diazepin-5-yl)phenyl 3-bromobenzene-sulfonate;
(B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
(B165) N-benzyl-N-[4-(2,4-dioxo-1,2,3,4-tetrahydronaphtho[1,2-b][1,4]diazepin-5-yl)phenyl]-2-nitrobenzenesulfonamide;
(B166) 3-bromo-N-[4-(2,4-dioxo-1,2,3,4-tetrahydronaphtho[1,2-b][1,4]diazepin-5-yl)phenyl]-N-methylbenzenesulfonamide;
(B167) N-[4-(2,4-dioxo-1,2,3,4-tetrahydronaphtho-[1,2-b][1,4]-diazepin-5-yl)phenyl]-N-methyl-2-nitrobenzenesulfonamide;
(B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(B169) N-[4-(7-chloro-2,4-dioxo-2,3,4,5-tetrahydro-1H-benzo[b][1,4]diazepin-1-yl)phenyl]benzenesulfonamide;
(B170) N-[4-(7-bromo-2,4-dioxo-2,3,4,5-tetrahydro-1H-benzo[b][1,4]diazepin-1-yl)phenyl]benzenesulfonamide;
(B171) N-[4-[(2,4-dioxo-7-(trifluoromethyl)-2,3,4,5-tetrahydro-1H-benzo[b][1,4]diazepin-1-yl)]phenyl]benzenesulfonamide;
(B172) N-[4-(2,4-dioxo-2,3,4,5-tetrahydro-1H-benzo[b][1,4]diazepin-1-yl)phenyl]benzenesulfonamide;
(B173) 1-(2-chlorophenyl)-N-[4-(2,4-dioxo-1,2,3,4-tetrahydronaphtho[1,2-b][1,4]diazepin-5-yl)phenyl]methanesulfonamide;
(B174) 1-(3-bromophenyl)-N-[4-(2,4-dioxo-1,2,3,4-tetrahydronaphtho[1,2-b][1,4]diazepin-5-yl)phenyl]methanesulfonamide;
(B175) N-[4-(2,4-dioxo-1,2,3,4-tetrahydronaphtho-[1,2-b][1,4]-diazepin-5-yl)phenyl]-2-trifluoromethylbenzenesulfonamide;
(B176) N-[4-(7-bromo-6-methyl-2,4-dioxo-2,3,4,5-tetrahydro-1H-benzo[b][1,4]diazepin-1-yl)phenyl]benzenesulfonamide;
(B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
(B178) 3-bromo-N-[4-(2,4-dioxo-1,2,3,4,8,9,10,11-octahydronaphtho[1,2-b][1,4]diazepin-5-yl)phenyl]benzenesulfonamide;
(B179) N-[4-(2,4-dioxo-1,2,3,4,8,9,10,11-octahydronaphtho[1,2-b][1,4]diazepin-5-yl)phenyl]-3-methoxybenzenesulfonamide;
(B180) 1-(2-bromophenyl)-N-[4-(2,4-dioxo-1,2,3,4-tetrahydronaphtho[1,2-b][1,4]diazepin-5-yl)phenyl]methanesulfonamide;
(B181) N-[4-(2,4-dioxo-1,2,3,4-tetrahydronaphtho[1,2-b][1,4]diazepin-5-yl)phenyl]-1-(2-methylphenyl)methanesulfonamide;
(B182) N-[4-(2,4-dioxo-1,2,3,4-tetrahydronaphtho[1,2-b][1,4]diazepin-5-yl)phenyl]-1-(2-nitrophenyl)methanesulfonamide;
(B183) N-[4-(2,4-dioxo-1,2,3,4-tetrahydronaphtho[1,2-b][1,4]diazepin-5-yl)phenyl]-2-phenylethanesulfonamide;
(B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
(B185) 1-(2-chlorophenyl)-N-[4-(2,4-dioxo-7-methoxy-1H-benzo[1,2-b][1,4]diazepin-1-yl)phenyl]methanesulfonamide;
(B186) 1-(2-chlorophenyl)-N-[4-(2,4-dioxo-7-hydroxy-1H-benzo[1,2-b][1,4]diazepin-1-yl)phenyl]methanesulfonamide;
(B187) 1-(4-chlorophenyl)-N-[4-(2,4-dioxo-1,2,3,4-tetrahydronaphtho[1,2-b][1,4]diazepin-5-yl)phenyl]methanesulfonamide;
(B188) 1-(2-chlorophenyl)-N-[4-(2,4-dioxo-1,2,3,4-tetrahydronaphtho[1,2-b][1,4]diazepin-5-yl)benzyl]methanesulfonamide;
(B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
(B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;
(B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
(B192) 1-(2-chlorophenyl)-N-[4-(2,4-dioxo-6-methyl-1H-benzo[1,2-b][1,4]diazepin-1-yl)phenyl]methanesulfonamide;
(B193) 1-(2-chlorophenyl)-N-[3-(2,4-dioxy-1,2,3,4-tetrahydronaphtho[1,2-b][1,4]diazepin-5-yl)propyl]methanesulfonamide;
(B194) 1-(2-chlorophenyl)-N-[2-(2,4-dioxo-1,2,3,4-tetrahydronaphtho[1,2-b][1,4]diazepin-5-yl)ethyl]methanesulfonamide;
(B195) N-[4-(2,4-dioxo-1,2,3,4,8,9,10,11-octahydronaphtho[1,2-b][1,4]diazepin-5-yl)phenyl]-1-(2-iodophenyl)methanesulfonamide;
(B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
(B197) 1-(2-chlorophenyl)-N-[4-(2-oxo-2,3-dihydro-1H-naphtho[1,2-e][1,4]diazepin-5-yl)phenyl]methanesulfonamide;
(B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
(B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
(B200) 1-(2,3-dimethylphenyl)-N-[4-(2,4-dioxo-1,2,3,4-tetrahydronaphtho[1,2-b][1,4]diazepin-5-yl)phenyl]phenyl-N-methylmethanesulfonamide;
(B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
(B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
(B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
(B204) 1-(2-dimethylaminophenyl)-N-[4-(2,4-dioxo-1,2,3,4-tetrahydronaphtho[1,2-b] [1,4]diazepin-5-yl)phenyl]phenyl-N-methylmethanesulfonamide;
(B205) 5-[4-[(pyridin-4-yl)carbonylamino]phenyl]-1H-naphtho[1,2-b][1,4]diazepine-2,4(3H,5H)-dione hydrochloride;
(B206) 5-[4-[2-[(pyridin-3-yl)oxy]acetylamino]phenyl]-1H-naphtho[1,2-b] [1,4]diazepine-2,4(3H,5H)-dione hydrochloride;
(B207) 5-[4-[(pyridin-3-yl)carbonylamino]phenyl]-1H-naphtho[1,2-b][1,4]diazepine-2,4(3H,5H)-dione hydrochloride;
(B208) 5-[4-[(2-methylpyridin-3-yl)carbonylamino]phenyl]-1H-naphtho[1,2-b] [1,4]diazepine-2,4(3H,5H)-dione hydrochloride;
(B209) 5-[4-[(2-chloropyridin-3-yl)carbonylamino]phenyl]-1H-naphtho[1,2-b] [1,4]diazepine-2,4(3H,5H)-dione;
(B210) 5-[4-[2-[(pyridin-2-yl)oxy]acetylamino]phenyl]-1H-naphtho[1,2-b] [1,4]diazepine-2,4(3H,5H)-dione;
(B211) 5-[4-[[4-(trifluoromethyl)pyridin-3-yl]carbonylamino]phenyl]-1H-naphtho[1,2-b][1,4]diazepine-2,4(3H,5H)-dione;
(B212) 5-[4-[(2-chloropyridin-3-yl)carbonylamino]phenyl]-1H-[1,4]diazepino[2,3-f]isoquinoline-2,4(3H,5H)-dione;
(B213) 5-[4-[(2-chloropyridin-3-yl)carbonylamino]phenyl]-8,9,10,11-tetrahydro-1H-[1,4]diazepino[2,3-f]isoquinoline-2,4(3H,5H)-dione; and
(B214) 5-[4-[(2-isopropylbenzoyl)amino]phenyl]-1H-naphtho[1,2-b][1,4]diazepine-2,4(3H,5H)-dione.

5. The pharmaceutical composition according to any one of claims 1 to 4, wherein the respiratory disease is inflammatory respiratory disease.

6. The pharmaceutical composition according to claim 5, wherein the inflammatory respiratory disease is bronchial obstruction, allergic pulmonary disease, or interstitial lung disease.

7. The pharmaceutical composition according to claim 5 or 6, wherein the inflammatory respiratory disease is COPD, bullous lung disease, diffuse panbronchiolitis, bronchiolitis obliterans, bronchiectasis, ciliary dyskinesia, sinobronchial syndrome, bronchial asthma, allergic bronchopulmonary aspergillosis, acute eosinophilic pneumonia, chronic eosinophilic pneumonia, eosinophilic granulomatosis with polyangiitis, chronic cough (cough variant asthma), hypersensitivity pneumonitis, idiopathic interstitial pneumonia, or acute interstitial pneumonia.

8. The pharmaceutical composition according to any one of claims 1 to 7, wherein the respiratory disease is respiratory disease accompanied by depression of respiratory function.

9. The pharmaceutical composition according to any one of claims 1 to 8, wherein the respiratory disease is respiratory disease accompanied by airflow obstruction.

10. The pharmaceutical composition according to any one of claims 1 to 9, wherein the respiratory disease is respiratory disease accompanied by respiratory inflammation.

11. The pharmaceutical composition according to any one of claims 1 to 10, wherein the respiratory disease is respiratory disease accompanied by cough reflex.
